# EUROPEAN PATENT SPECIFICATION

(11) **EP 1 689 737 B1**
(45) Date of publication and mention of the grant of the patent: **16.07.2008**
(21) Application number: 04797809.3
(22) Date of filing: 10.11.2004
(51) Int. Cl.: C07D 401/04, A61P 25/00, A61K 31/4439

(54) **BETA-LACTAMS FOR THE TREATMENT OF CNS DISORDERS**
BETA-LACTAME ZUR BEHANDLUNG VON ERKRANKUNGEN DES ZNS
BETA-LACTAMES POUR LE TRAITEMENT DE TROUBLES DU SYSTEME NERVEUX CENTRAL

(30) Priority: 12.11.2003 GB 0326407
(43) Date of publication of application: 16.08.2006
(73) Proprietor: GLAXO GROUP LIMITED, Greenford, Middlesex UB6 0NN (GB)
(72) Inventor: ALVARO, Giuseppe, GlaxoSmithKline, I-37100 Verona (IT); DI FABIO, Romano, GlaxoSmithKline, I-37100 Verona (IT); GIOVANNINI, Riccardo, GlaxoSmithKline, I-37100 Verona (IT); PAIO, Alfredo, GlaxoSmithKline, I-37100 Verona (IT); TRANQUILLINI, Maria Elvira, GlaxoSmithKline, I-37100 Verona (IT); MATTIOLI, Lucia, GlaxoSmithKline, I-37100 Verona (IT)
(74) Representative: Giddings, Peter John
(86) International application number: PCT/EP2004/012772
(87) International publication number: WO 2005/049600

(56) References cited:
- US-A- 5 677 317
- US-A- 5 780 466

## Description

The present invention relates to lactam derivatives, to processes for their preparation, to pharmaceutical compositions containing them and to their medical use.

Neurokinin receptor antagonists are known from US 5 780 466. The present invention provides a compound of formula (I) 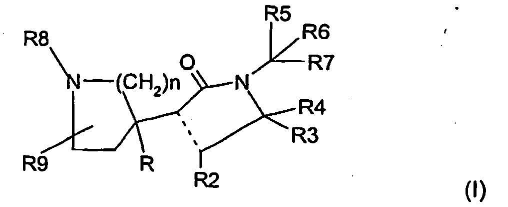 wherein
- ---- represents a single or a double bond;
- R represents a radical selected from: 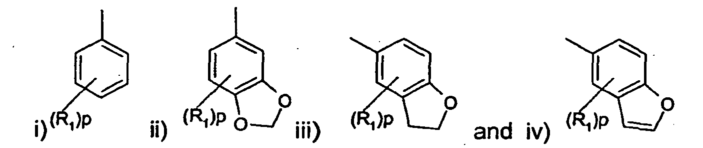 in which R₁ is halogen, cyano, C₁₋₄ alkyl, C₁₋₄ alkoxy, trifluoromethyl or trifluoromethoxy and p is zero or an integer from 1 to 3;
- R₂ represents hydrogen or C₁₋₄ alkyl;
- R₃ represents hydrogen, hydroxy or C₁₋₄ alkyl;
- R₄ represents hydrogen or R₄ together with R₃ represents =O or =CH2;
- R₅ represents phenyl, naphthyl, a 9 to 10 membered fused bicyclic heterocyclic group or a 5 or 6 membered heteroaryl group, wherein said groups are optionally substituted by 1 to 3 groups independently selected from trifluoromethyl, C₁₋₄ alkyl, hydroxy, cyano, C₁₋₄ alkoxy, trifluoromethoxy, halogen or S(O)qC₁₋₄ alkyl;
- R₆ and R₇ independently represent hydrogen, cyano, C₁₋₄ alkyl;
- R₈ is (CH₂)rR₁₀;
- R₉ represents hydrogen, halogen, C₃₋₇ cycloalkyl, hydroxy, nitro, cyano or C₁₋₄ alkyl optionally substituted by one or two groups selected from halogen, cyano, hydroxy or C₁₋₄ alkoxy;
- R₁₀ represents hydrogen or C₃₋₇ cycloalkyl;
- n represents 1 or 2;
- q is 0, 1 or 2;
- r is 0 or an integer from 1 to 4;
   or a pharmaceutically acceptable salt or a solvate thereof.

Another embodiment of the invention provides compounds of formula (I), wherein
- ---- represents a single or a double bond;
- R represents 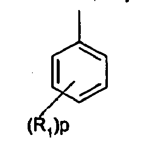 in which R₁ is halogen, C₁₋₄ alkyl, cyano, C₁₋₄ alkoxy, trifluoromethyl or trifluoromethoxy p is zero or an integer from 1 to 3;
- R₂ represents hydrogen or C₁₋₄ alkyl;
- R₃ represents hydrogen, hydroxy or C₁₋₄ alkyl;
- R₄ represents hydrogen or R₄ together with R₃ represents =O;
- R₅ represents phenyl, naphthyl, a 9 to 10 membered fused bicyclic heterocyclic group or a 5 or 6 membered heteroaryl group, wherein said groups are optionally substituted by 1 to 3 groups independently selected from trifluoromethyl, C₁₋₄ alkyl, hydroxy, cyano, C₁₋₄ alkoxy, trifluoromethoxy, halogen or S(O)qC₁₋₄ alkyl;
- R₆ and R₇ independently represent hydrogen, cyano, C₁₋₄ alkyl or R₃ together with R₄ represents C₃₋₇ cycloalkyl;
- R₈ represents (CH₂)rR₁₀;
- R₉ represents hydrogen, halogen, C₃₋₇ cycloalkyl, hydroxy, nitro, cyano or C₁₋₄ alkyl optionally substituted by one or two groups selected from halogen, cyano, hydroxy or C₁₋₄ alkoxy;
- R₁₀ represents hydrogen or C₃₋₇ cycloalkyl;
- n represents 1 or 2;
- q is 0, 1 or 2;
- r is 0 or an integer from 1 to 4;
   or a pharmaceutically acceptable salt and a solvate thereof.

A further embodiment of the invention provides compounds of formula (I), wherein
- ---- represents a single or a double bond;
- R represents 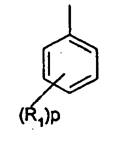 in which R₁ is halogen, C₁₋₄ alkyl, cyano, C₁₋₄ alkoxy, trifluoromethyl or trifluoromethoxy p is zero or an integer from 1 to 3;
- R₂ represents hydrogen or C₁₋₄ alkyl;
- R₃ represents hydrogen, hydroxy or C₁₋₄ alkyl;
- R₄ represents hydrogen or R₄ together with R₃ represents =O;
- R₅ represents phenyl, naphthyl, a 9 to 10 membered fused bicyclic heterocyclic group or a 5 or 6 membered heteroaryl group, wherein said groups are optionally substituted by 1 to 3 groups independently selected from trifluoromethyl, C₁₋₄ alkyl, hydroxy, cyano, C₁₋₄ alkoxy, trifluoromethoxy, halogen or S(O)qC₁₋₄ alkyl;
- R₆ and R₇ independently represent hydrogen, cyano, C₁₋₄ alkyl;
- R₈ represents (CH₂)rR₁₀;
- R₉ represents hydrogen, halogen, C₃₋₇ cycloalkyl, hydroxy, nitro, cyano or C₁₋₄ alkyl optionally substituted by one or two groups selected from halogen, cyano, hydroxy or C₁₋₄ alkoxy;
- R₁₀ represents hydrogen or C₃₋₇ cycloalkyl;
- n represents 1 or 2;
- q is 0, 1 or 2;
- r is 0 or an integer from 1 to 4;
or a pharmaceutically acceptable salt and a solvate thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric, hydroiodic, metaphosphoric, or phosphoric acid; and organic acids e.g. succinic, maleic; acetic, fumaric, citric, tartaric, benzoic, trifluoroacetic, malic, lactic, formic, propionic, glycolic, gluconic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), ethanesulfonic, pantothenic, stearic, sulfinilic, alginic and galacturonic acid; and arylsulfonic, for example benzenesulfonic, p-toluenesulfonic, methanesulfonic or naphthalenesulfonic acid; base addition salts formed with alkali metals and alkaline earth metals and organic bases such as N,N-dibenzylethylenediamine, chloroprocaine, choline, diethanolamine, ethylenediamine, meglumine (N-methylglucamine), lysine and procaine; and internally formed salts. Certain of the compounds of formula (I) may form acid addition salts with less than one or one or more equivalents of the acid, for example to form a dihydrochloride salt. The present invention includes within its scope all possible stoichiometric and non-stoichiometric forms. Salts having a non-physiologically acceptable anion or cation are within the scope of the invention as useful intermediates for the preparation of physiologically acceptable salts and/or for use in non-therapeutic, for example, *in vitro*, situations.

The solvates may, for example, be hydrates.

This invention also includes within its scope stoichiometric hydrates or solvates as well as compounds containing variable amounts of water and/or solvent.

The compounds of formula (I) may be prepared in crystalline or non-crystalline form, and, if crystalline, may optionally be hydrated or solvated. Furthermore, some of the crystalline forms of the compounds of formula(I) may exist in alternative polymorphic forms, which are included in the present invention.

It will be appreciated by those skilled in the art that the compounds of formula (I), wherein n is 1, contain at least one chiral centre (namely the carbon atom shown as * in formula (1a) and (1b) and may be represented by formula (1a) and (1b).

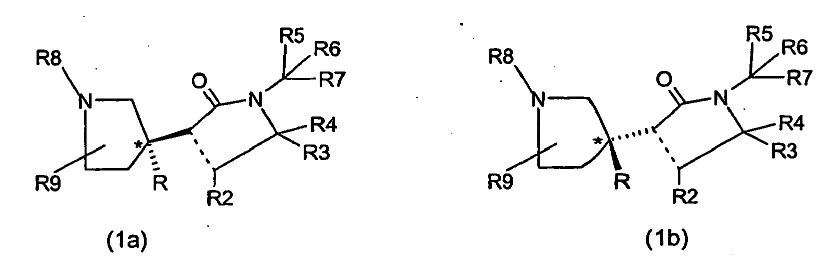

The wedged bond indicates that the bond is above the plane of the paper. The broken bond indicates that the bond is below the plane of the paper.

At least two asymmetric carbon atoms are present in the compounds of formula (I), wherein R₉ is different from hydrogen and may be represented by formula (1 c), (1d), (1e) and (1f).

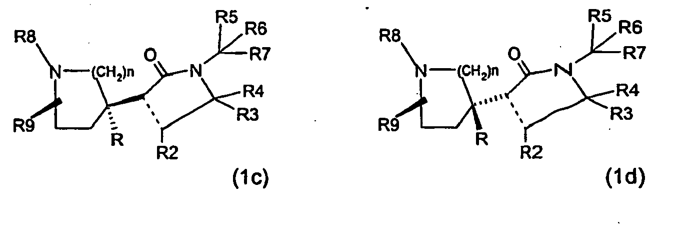

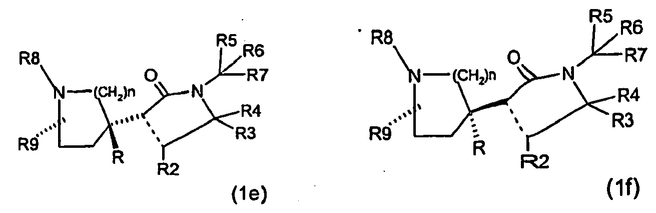

Further asymmetric carbon atoms are possible when R₃ and R₄ are not the same group and/or when R₆ and R₇ are not the same group and/or when ---- is a single bond and/or when ---- is a single bond and R₂ is C1-4 alkyl.

Thus, for example at least two asymmetric carbon atoms are present when R₆ and R₇ are not the same group and ----- is a single bond (namely the carbon atoms shown as ** and as *** in formula (1g)).

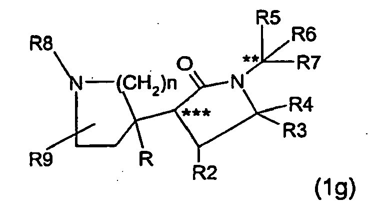

It is to be understood that all stereoisomeric forms, including all enantiomers, diastereoisomers and all mixtures thereof, including racemates, are encompassed within the scope of the present invention and the reference to compounds of formula (I) includes all stereoisomeric forms unless otherwise stated.

The present invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I.

Compounds of the present invention and pharmaceutically acceptable salts of said compounds that contain the aforementioned isotopes and/or other isotopes of other atoms are within the scope of the present invention. Isotopically - labeled compounds of the present invention, for example those into which radioactive isotopes such as ³H, ¹⁴C are incorporated, are useful in drug and/or substrate tissue distribution assays. Tritiated , i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium , i.e., ²H, can afford certain therapeutic advantages resulting from greater metabolic stability, for example increased *in vivo* half-life or reduced dosage requirements and, hence, may be preferred in some circumstances. Isotopically labeled compounds of formulas I and following of this invention can generally be prepared by carrying out the procedures disclosed in the Schemes and/or in the Examples below, by substituting a readily available isotopically labeled reagent for a non-isotopically labeled reagent.

Where used herein the term naphthyl, whether alone or as part of another group, is intended, unless otherwise stated, to denote both 1-naphthyl and 2-naphthyl groups.

The term C₁₋₄ alkyl as used herein as a group or a part of the group refers to a straight or branched alkyl group containing from 1 to 4 carbon atoms; examples of such groups include methyl, ethyl, propyl, iso-propyl, n-butyl, iso-butyl, tert-butyl.

The term halogen refers to fluorine, chlorine, bromine or iodine.

The term C₃₋₇ cycloalkyl group means a non aromatic monocyclic hydrocarbon ring of 3 to 7 carbon atoms such as, for example, cyclopropyl, cyclobutyl, cyclopentyl, cyclohexyl or cycloheptyl.

The term C₁₋₄ alkoxy group may be a straight chain or a branched chain alkoxy group, for example methoxy, ethoxy, prop-1-oxy, prop-2-oxy, but-1-oxy, but-2-oxy or 2-methylprop-2-oxy.

When R₅ is a 5 or 6 membered heteroaryl group according to the invention this includes furanyl, thiophenyl, pyrrolyl, imidazolyl, thiazolyl, oxazolyl, pyrazolyl, isoxazolyl, isothiazolyl, 1,2,3-triazolyl, 1,2,3-oxadiazolyl, 1,2,3-thiadiazolyl, 1,2,4-triazolyl, 1,3,4-oxadiazolyl, 1,3,4-thiadiazolyl, 1,2,5-oxadiazolyl, 1,2,5-thiadiazolyl, pyridyl, pyridazinyl, pyrimidinyl, pyrazinyl, 1,2,4-oxadiazolyl, 1,2,5-triazinyl or 1,3,5-triazinyl and the like.

The term 9 to 10 membered fused bicyclic heterocyclic group refers to a 5, 6 or 6, 6 bicyclic ring system, containing at least one heteroatom selected from oxygen, sulphur or nitrogen, which may be saturated, unsaturated or aromatic. The term 9 to 10 membered fused bicyclic heterocyclic group also refers to a phenyl fused to a 5 or 6 membered heterocyclic group. Example of such groups include benzofuranyl, benzothiophenyl, indolyl, benzoxazolyl, 3H-imidazo[4,5-c]pyridin-yl, dihydrophthazinyl, 1H-imidazo[4,5-c]pyridin-1-yl, imidazo[4,5-b]pyridyl, 1,3-benzo[1,3]dioxolyl, 2H-chromanyl, isochromanyl, 5-oxo-2,3-dihydro-5H-[1,3]thiazolo[3,2-a]pyrimidyl, 1,3-benzothiazolyl, 1,4,5,6-tetrahydropyridazyl, 1,2,3,4,7,8-hexahydropteridinyl, 2-thioxo-2,3,6,9-tetrahydro-1H-purin-8-yl, 3,7-dihydro-1H-purin-8-yl, 3,4-dihydropyrimidin-1-yl, 2,3-dihydro-1,4-benzodioxinyl, benzo[1,3]dioxolyl, 2H-chromenyl, chromanyl, 3,4-dihydrophthalazinyl, 2,3-dihydro-1H-indolyl, 1,3-dihydro-2H-isoindol-2-yl, 2,4,7-trioxo-1,2,3,4,7,8-hexahydropteridinyl, thieno[3,2-d]pyrimidinyl, 4-oxo-4,7-dihydro-3H-pyrrolo[2,3-d]pyrimidinyl, 1,3-dimethyl-6-oxo-2-thioxo-2,3,6,9-tetrahydro-1H-purinyl, 1,2-dihydroisoquinolinyl, 2-oxo-1,3-benzoxazolyl, 2,3-dihydro-5H-1,3-thiazolo[3,2-a]pyrimidinyl, 5,6,7,8-tetrahydro-quinazolinyl, 4-oxochromanyl, 1,3-benzothiazolyl, benzimidazolyl, benzotriazolyl, purinyl, furylpyridyl, thiophenylpyrimidyl, thiophenylpyridyl, pyrrolylpiridyl, oxazolylpyridyl, thiazolylpiridyl, 3,4-dihydropyrimidin-1-yl imidazolylpiridyl, quinoliyl, isoquinolinyl, quinazoliriyl, quinoxalinyl, naphthyridinyl, pyrazolyl[3.4]pyridine, 1,2-dihydroisoquinolinyl, cinnolinyl, 2,3-dihydro-benzo[1,4]dioxin-6-yl, 4,5.6,7-tetrahydro-benzo[b]thiophenyl-2-yl, 1,8-naphthyridinyl, 1,6-naphthyridinyl, 3,4-dihydro-2H-1,4-benzothiazine, 4,8-dihydroxy-quinolinyl, 1-oxo-1,2-dihydro-isoquinolinyl or 4-phenyl-[1,2,3]thiadiazolyl and the like.

In the compounds of formula (I) wherein n is 1 the group R₉ may be in position 2, 4, or 5 as represented in formula(1h)

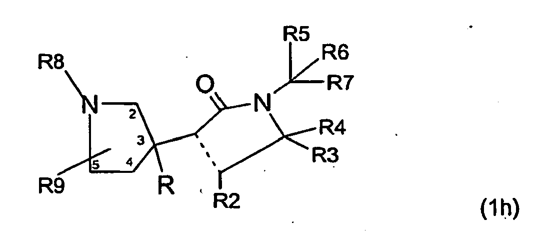

In the compounds of formula (I) wherein n is 2 the group R₉ may be in position 2, 3, 5 or 6 of the piperidine ring as represented in formula (1i)

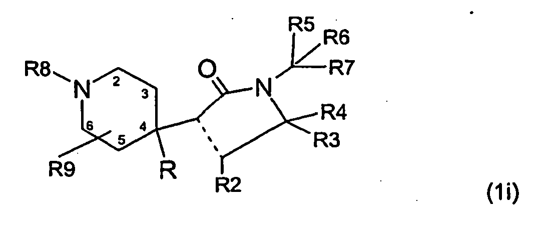

In one embodiment, n is 2.

In one embodiment R represents 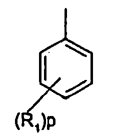 in which R₁ is halogen, C₁₋₄ alkyl, cyano, C₁₋₄ alkoxy, trifluoromethyl or trifluoromethoxy p is zero or an integer from 1 to 3.

In one embodiment, R is phenyl optionally substituted by one or two groups selected from halogen(e.g. fluorine) cyano, C₁₋₄ alkyl(e.g. methyl), C₁₋₄ alkoxy(e.g.methoxy), trifluoromethyl or trifluoromethoxy.

In another embodiment, R is phenyl substituted by a fluorine.

In one embodiment, R₂ is hydrogen or methyl.

In one embodiment, R₃ is hydrogen hydroxy or methyl, or together with R₄ forms =O or =CH2.

In another embodiment, R₃ is hydrogen hydroxy or methyl, or together with R₄ forms =O.

In one embodiment, R₄ is hydrogen.

In one embodiment, R₅ is phenyl or naphthyl optionally substituted by one or two groups selected from trifluoromethyl, cyano, C₁₋₄ alkyl or halogen.

In one embodiment, R₆ is hydrogen or methyl.

In one embodiment, R₇ is hydrogen or methyl.

In one embodiment, R₈ is (CH₂)ᵣR₁₀ in which R₁₀ is hydrogen or C₃₋₇ cycloalkyl (e.g cyclopropyl) and r is 0 or 1.

In one embodiment, R₉ is hydrogen or C₁₋₄ alkyl optionally substituted by one or two groups selected from halogen.

In one embodiment R is phenyl substituted by a fluorine, R₂, R₉ and R₄ are hydrogen, R₃ is hydrogen, hydroxy or methyl, or together with R₄ form =O or =CH₂, R₆ and R₇ are independently hydrogen or methyl, R₅ is phenyl or naphthyl optionally substituted by one or two groups independently selected from cyano, methyl, chlorine, bromine or fluorine atom, R₈ is hydrogen, methyl or cyclopropylmethyl, and n is 2.

In onother embodiment R is phenyl substituted by a fluorine, R₂, R₉ and R₄ are hydrogen, R₃ is hydrogen, hydroxy or methyl, or together with R₄ form =O, R₆ and R₇ are independently hydrogen or methyl, R₅ is phenyl or naphthyl optionally substituted by one or two groups independently selected from cyano, methyl, chlorine, bromine or fluorine atom, R₈ is hydrogen, methyl or cyclopropylmethyl, and n is 2.

Examples of the compounds of the present invention include:
- 1-[(3,5-Dichlorophenyl)methyl]-3-[4-(4-fluorophenyl)-4-piperidinyl]-1,5-dihydro-2*H-*pyrrol-2-one;
- 1-[(3,5-Dichlorophenyl)methyl]-3-[4-(4-fluorophenyl)-1-methyl-4-piperidinyl]-1,5-dihydro-2*H*-pyrrol-2-one;
- 1-[1-(3,5-Dichlorophenyl)ethyl]-3-[4-(4-fluorophenyl)-4-piperidinyl]-1,5-dihydro-2*H-*pyrrol-2-one (Chain Enantiomer 1);
- 1-[(1*S*)-1-(3-Chloro-1-naphthalenyl)ethyl]-3-[4-(4-fluorophenyl)-4-piperidinyl]-1,5-dihydro-2*H*-pyrrol-2-one ;
- 1-[(3-Chloro-1-naphthalenyl)methyl]-3-[4-(4-fluorophenyl)-4-piperidinyl]-1,5-dihydro-2*H*-pyrrol-2-one;
- 4-({3-[4-(4-Fluorophenyl)-4-piperidinyl]-2-oxo-2,5-dihydro-1*H*-pyrrol-1-yl}methyl)-2-naphthalenecarbonitrile;
- 1-[1-(3,5-Dichlorophenyl)ethyl]-3-[4-(4-fluorophenyl)-4-piperidinyl]-1,5-dihydro-2*H-*pyrrol-2-one (Chain Enantiomer 2);
- 1-[(1*R*)-1-(3-Chloro-1-naphthalenyl)ethyl]-3-[4-(4-fluorophenyl)-4-piperidinyl]-1,5-dihydro-2H-pyrrol-2-one;
- 1-[1-(3,5-Dichlorophenyl)ethyl]-3-[4-(4-fluorophenyl)-1-methyl-4-piperidinyl]-1,5-dihydro-2*H*-pyrrol-2-one (Chain Enantiomer 1);
- 1-[(1*S*)-1-(3-Chloro-1-naphthalenyl)ethyl]-3-[4-(4-fluorophenyl)-1-methyl-4-piperidinyl]-1,5-dihydro-2*H*-pyrrol-2-one ;
- 1-[(3-Chloro-1-naphthalenyl)methyl]-3-[4-(4-fluorophenyl)-1-methyl-4-piperidinyl]-1,5-dihydro-2*H*-pyrrol-2-one;
- 1-[1-(3,5-Dichlorophenyl)ethyl]-3-[4-(4-fluorophenyl)-1-methyl-4-piperidinyl]-1,5-dihydro-2*H*-pyrrol-2-one (Chain Enantiomer 2);
- 1-[(1*R*)-(3-Chloro-1-naphthalenyl)ethyl]-3-[4-(4-fluorophenyl)-1-methyl-4-piperidinyl]-1,5-dihydro-2*H*-pyrrol-2-one;
- 4-({3-[4-(4-Fluorophenyl)-1-methyl-4-piperidinyl]-2-oxo-2,5-dihydro-1*H*-pyrrol-1-yl}methyl)-2-naphthalenecarbonitrile;
- 1-[1-(3,5-Dichlorophenyl)ethyl]-3-[4-(4-fluorophenyl)-1-methyl-4-piperidinyl]-1,5-dihydro-2*H*-pyrrol-2-one hydrochloride (Chain Enantiomer 1);
- 1-[1-(3,5-Dichlorophenyl)ethyl]-3-[4-(4-fluorophenyl)-1-methyl-4-piperidinyl]-1,5-dihydro-2*H*-pyrrol-2-one hydrochloride (Chain Enantiomer 1);
- 1-[(3-Chloro-1-naphthalenyl)methyl]-3-[1-(cyclopropylmethyl)-4-(4-fluorophenyl)-4-piperidinyl]-1,5-dihydro-2*H*-pyrrol-2-one;
- 1-[(3,5-Dichlorophenyl)methyl]-3-[4-(4-fluorophenyl)-4-piperidinyl]-2-pyrrolidinone 1-[(1*S*)-1-(3-Chloro-1-naphthalenyl)ethyl]-3-[4-(4-fluorophenyl)-4-piperidinyl]-2-pyrrolidinone (Diastereoisomer 1);
- 1-[(1*S*)-1-(3-Chloro-1-naphthalenyl)ethyl]-3-[4-(4-fluorophenyl)-4-piperidinyl]-2-pyrrolidinone;
- 1-[1-(3,5-Dichlorophenyl)ethyl]-3-[4-(4-fluorophenyl)-4-piperidinyl]-2-pyrrolidinone (Diastereoisomer 1 Chain Enantiomer 1);
- 1-[1-(3,5-Dichlorophenyl)ethyl]-3-[4-(4-fluorophenyl)-4-piperidinyl]-2-pyrrolidinone (Diastereoisomer 2 Chain Enantiomer 1);
- 1-[1-(3,5-Dichlorophenyl)ethyl]-3-[4-(4-fluorophenyl)-4-piperidinyl]-2-pyrrolidinone (Diastereoisomer 1 Chain Enantiomer 2);
- 1-[1-(3,5-Dichlorophenyl)ethyl]-3-[4-(4-fluorophenyl)-4-piperidinyl]-2-pyrrolidinone (Diastereoisomer 2 Chain Enantiomer 2);
- 4-({3-[4-(4-Fluorophenyl)-4-piperidinyl]-2-oxo-1-pyrrolidinyl}methyl)-2-naphthalenecarbonitrile (Enantiomer 1);
- 4-({3-[4-(4-Fluorophenyl)-4-piperidinyl]-2-oxo-1-pyrrolidinyl}methyl)-2-naphthalenecarbonitrile (Enantiomer 2);
- 7-Fluoro-4-({3-[4-(4-fluorophenyl)-4-piperidinyl]-2-oxo-1-pyrrolidinyl}methyl)-2-naphthalenecarbonitrile (Enantiomer 2);
- 6-Fluoro-4-({3-[4-(4-fluorophenyl)-4-piperidinyl]-2-oxo-1-pyrrolidinyl}methyl)-2-naphthalenecarbonitrile (Enantiomer 2);
- 7-Fluoro-4-({3-[4-(4-fluorophehyl)-4-piperidinyl]-2-oxo-1-pyrrolidmyl}methyl)-2-naphthalenecarbonitrile (Enantiomer 1);
- 6-Fluoro-4-({3-[4-(4-fluorophenyl)-4-piperidinyl]-2-oxo-1-pyrrolidinyl}methyl)-2-naphthalenecarbonitrile (Enantiomer 1);
- 1-[(3,5-Dichlorophenyl)methyl]-3-[4-(4-fluorophenyl)-1-methyl-4-piperidinyl]-2-pyrrolidinone;
- 1-[1-(3-Chloro-1-naphthalenyl)ethyl]-3-[4-(4-fluorophenyl)-1-methyl-4-piperidinyl]-2-pyrrolidinone (Diastereoisomer 2 Chain Enantiomer 1);
- 1-[1-(3,5-Dichlorophenyl)ethyl]-3-[4-(4-fluorophenyl)-1-methyl-4-piperidinyl]-2-pyrrolidinone (Diastereoisomer 1 Chain Enantiomer 1);
- 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 1);
- 1-[1-(3,5-Dichlorophenyl)ethyl]-3-[4-(4-fluorophenyl)-1-methyl-4-piperidinyl]-2-pyrrolidinone (Diastereoisomer 1 Chain Enantiomer 2);
- 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 2);
- 1-[(3,5-Dichlorophenyl)methyl]-3-[4-(4-fluorophenyl)-1-methyl-4-piperidinyl]-2-pyrrolidinone (Enantiomer 1);
- 1-[(3,5-Dichlorophenyl)methyl]-3-[4-(4-fluorophenyl)-1-methyl-4-piperidinyl]-2-pyrrolidinone (Enantiomer 2);
- 4-({3-[4-(4-Fluorophenyl)-1-methyl-4-piperidinyl]-2-oxo-1-pyrrolidinyl}methyl)-2-naphthalenecarbonitrile (Enantiomer 1);
- 4-({3-[4-(4-Fluorophenyl)-1-methyl-4-piperidinyl]-2-oxo-1-pyrrolidinyl}methyl)-2-naphthalenecarbonitrile (Enantiomer 2);
- 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 2);
- 6-Fluoro-4-({3-[4-(4-fluorophenyl)-1-methyl-4-piperidinyl]-2-oxo-1-pyrrolidinyl}methyl)-2-naphthalenecarbonitrile;
- 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 1);
- 6-Fluoro-4-({3-[4-(4-fluorophenyl)-1-methyl-4-piperidinyl]-2-oxo-1-pyrrolidinyl}methyl)-2-naphthalenecarbonitrile (Enantiomer 1);
- 1-[(3,5-Dichlorophenyl)methyl]-3-[4-(4-fluorophenyl)-1-methyl-4-piperidinyl]-1H-pyrrole-2,5-dione;
- 1-[(3,5-Dichlorophenyl)methyl]-3-[4-(4-fluorophenyl)-1-methyl-4-pipendinyl]-5-methylidene-1,5-dihydro-2H-pyrrol-2-one;
or a phamaceutically acceptable salt (e.g hydrochloride, fumarate or citrate) or a solvate or amorphous or crystalline forms thereof.

Examples of the compounds of the present invention include:
- 1-[(3,5-Dichlorophenyl)methyl]-3-[4-(4-fluorophenyl)-4-piperidinyl]-1,5-dihydro-2*H-*pyrrol-2-one;
- 1-[(3,5-Dichlorophenyl)methyl]-3-[4-(4-fluorophenyl)-1-methyl-4-piperidinyl]-1,5-dihydro-2*H*-pyrrol-2-one;
- 1-[1-(3,5-Dichlorophenyl)ethyl]-3-[4-(4-fluorophenyl)-4-piperidinyl]-1,5-dihydro-2*H-*pyrrol-2-one (Chain Enantiomer 1);
- 1-[(1*S*)-1-(3-Chloro-1-naphthalenyl)ethyl]-3-[4-(4-fluorophenyl)-4-piperidinyl]-1,5-dihydro-2*H*-pyrrol-2-one;
- 1-[1-(3,5-Dichlorophenyl)ethyl]-3-[4-(4-fluorophenyl)-1-methyl-4-piperidinyl]-1,5-dihydro-2H-pyrrol-2-one (Chain Enantiomer 1);
- 1-[1-(3,5-Dichlorophenyl)ethyl]-3-[4-(4-fluorophenyl)-1-methyl-4-piperidinyl]-1,5-dihydro-2*H*-pyrrol-2-one (Chain Enantiomer 2);
- 1-[(1*R*)-1-(3-Chloro-1-naphthalenyl)ethyl]-3-[4-(4-fluorophenyl)-1-methyl-4-piperidinyl]-1,5-dihydro-2*H*-pyrrol-2-one;
- 4-({3-[4-(4-Fluorophenyl)-1-methyl-4-piperidinyl]-2-oxo-2,5-dihydro-1*H*-pyrrol-1-yl}methyl)-2-naphthalenecarbonitrile;
and phamaceutically acceptable salts (e.g hydrochloride, fumarate or citrate) and solvates or amorphous or crystalline forms thereof.

Specific compounds of the invention are:
- 1-[(3,5-Dichlorophenyl)methyl]-3-[4-(4-fluorophenyl)-1-methyl-4-piperidinyl]-1,5-dihydro-2*H*-pyrrol-2-one;
- 1-[(3,5-Dichlorophenyl)methyl]-3-[4-(4-fluorophenyl)-1-methyl-4-piperidinyl]-1,5-dihydro-2*H*-pyrrol-2-one hydrochloride;
- 1-[(3,5-Dichlorophenyl)methyl]-3-[4-(4-fluorophenyl)-1-methyl-4-piperidinyl]-1,5-dihydro-2*H*-pyrrol-2-one fumarate;
- 1-[(3,5-Dichlorophenyl)methyl]-3-[4-(4-fluorophenyl)-1-methyl-4-piperidinyl]-1,5-dihydro-2*H*-pyrrol-2-one citrate;
or crystalline forms thereof.

The compounds of the invention are antagonists of tachykinin receptors, including substance P and other neurokinins, both in vitro and in vivo and are thus of use in the treatment of conditions mediated by tachykinins, including substance P and other neurokinins.

Tachykinins are a family of peptides that share a common carboxyl-terminal sequence (Phe-X-Gly-Leu-Met-NH2). They are actively involved in the physiology of both lower and advanced lifeforms. In mammalian lifeforms the main tachykinins are subtance P (SP), Neurokinin A (NKA) and Neurokinin B (NKB) which act as neurotransmitters and neuromodulators. Mammalian tachykinins may contribute to the pathophysiology of a number of human diseases.

Three types of tachykinins receptors have been identified, namely NK1 (SP-preferring), NK2 (NKA-preferring) and NK3 (NKB-preferring) which are widely distributed throughout the central nervous (CNS) and peripheral nervous system.

Particularly the compounds of the invention are antagonists of the NK1 receptor.

The compounds of the present invention also have activity as selective serotonin reuptake inhibitors (hereinafter referred to as SSRIs) and are thus of use in the treatment of conditions mediated by selective inhibition of the serotonin reuptake transporter protein.

Thus, the compounds of the present invention combine dual activity as tachykinin antagonists, including substance P and other neurokinins, and as SSRIs. In particular, the compounds of the invention combine dual activity as NK1 receptor antagonists and as SSRIs.

NK₁-receptor binding affinity has been determined in vitro in a binding Scintillation proximity assay (SPA) by measuring the compounds' ability to displace [¹²⁵I]Tyr8-Substance P (SP) from recombinant human NK₁ receptors stably expressed in Chinese Hamster Ovary (CHO) cell membranes prepared by using a modification of the method described by Beattie D.T. et al. (Br. J. Pharmacol, 116:3149-3157, 1995). Briefly, polystrene Leadseeker WGA-SPA beads (Amersham Biosciences) were mixed with cell membranes in a bead/membrane ratio of 50:1 (w/w) in assay buffer (75 mM Tris pH 7.8, 75 mM NaCl, 4 mM MnCl2, 1 mM EDTA, 0.05% Chaps, 1 mM PMSF). The mixture was placed on ice for 30 minutes to allow the formation of membrane/bead complex before BSA was added to a final concentration of 1%. After another 30 minutes incubation on ice, the bead/membrane complex was washed twice and suspended in assay buffer. [¹²⁵I]Tyr8-Substance P (2200 Ci/mmol, PerkinElmer) was then added to the bead/membrane complex with a final concentration of 0.4 nM. 30 ul of the resulting mixture was then dispensed to each well of Nalgen NUNC 384-well plate with 1 ul compound pre-dispensed in DMSO. The plates were then sealed and pulse centrifuged at 1100 rpm. After 3 hours incubation at room temperature with shaking, the plates were centrifuged for 2 min at 1100 rpm and measured in Viewlux imager (PerkinElmer) for 5 minutes with a 618-nm filter. Inhibition of [¹²⁵I]Tyr8-Substance P binding to NK₁-receptors was measured by the reduction of luminescent signal. IC₅₀ values of each compound were determined by an 11-point 3x-dilution inhibition curve. pKᵢ values were calculated using the K_{D} of [¹²⁵I]Tyr8-Substance P determined in a separate experiment.

For representative compounds of the invention the NK₁-receptor binding affinity has also been determined in vitro using conventional filtration techniques by measuring the compounds' ability to displace [³H] -substance P SP from recombinant human NK₁ receptors expressed in CHO cell membranes prepared as described above. Briefly, ligand binding was performed in 0.2 ml of 50 mM HEPES, pH 7.4, containing 3 mM MnCl₂, 0.02% BSA, 0.5 nM [³H]-Substance P (30-56 Ci/mmol Amersham), a final membrane protein concentration of 30-50 µg/ml, and the test compounds. The incubation proceeded at room temperature for 40 min and was stopped by filtration. Non-specific binding was determined using excess of substance P (1 µM) and represents about 6-10% of the total binding.

Compounds of the invention were further characterised in a functional assay using FLIPR technology for the determination of their effect to inhibit the intracellular calcium increase induced by SP in both Human-NK₁-CHO cells and human U2OS cells transducted with NK₁ BacMan virus. Briefly, 10K-15K cells/well were seeded in 384 well Greiner bio-one plate in culture medium (DMEM with 10% FBS), incubated overnight in CO2 at 37°C. For human U2OS cells, 1% (v/v) BacMan virus carrying NK₁ gene was mixed with cells before plating. After aspirating the medium, cells were loaded with cytoplasmic calcium indicator Calcium 3 dye (Molecular Devices Co.) in 30ul/well buffer (Hank's balanced salts with 20 mM Hepes) and incubated in CO2 at 37°C for 60 minutes. 10ul/well assay buffer (Hank's balanced salts with 20 mM Hepes) containing different concentrations of compounds was then added to the cells for another 30 minutes incubation at 37°C. Finally, 10ul/well SP in assay buffer containing 0.1% BSA was added to the cells and fluorescence signal read on a FLIPR system. IC50 values of each compound were determined by an 11-point 3X-dilution inhibition curve. The potency of the antagonist (fpKᵢ value or pK_{B} value) was calculated from pIC50 by the Cheng-Prusoff equation or calculated from Schild's analysis.

SERT binding affinity has been determined in vitro by the compounds' ability to displace [³H]-citalopram from hSERT-LLCPK cell membranes. For the binding reaction, a final concentration of 0.25 nM of [³H] citalopram (84 Ci/mmol, Amersham) was incubated with 3-5µg/ml of cell membrane and the compound to be tested at different concentrations (7 concentration points in duplicate) in 50 mM Tris HCl, pH 7.7, containing 120 mM NaCl , 5 mM KCl, 10µM pargyline and 0.1% ascorbic acid. The reaction was performed for 120 min at 22°C and was terminated through GF/B Unifilter (pre-soaked in 0.5 % PEI) using a Cell Harvester (Tomtec). Scintillation fluid was added to each filtered spot and radioactivity was determined using a scintillation counter (TopCount (Packard)). Non-specific binding was determined using paroxetine (10µM) and represents about 2-5% of the total binding. Competition experiments were conducted with duplicate determination for each point. Msat601 software package was used to elaborate the competition binding data. IC₅₀ values were converted to Kᵢ values using the Cheng-Prusoff equation and by using the K_{D} of [³H]citalopram determined in separate experiments.

For preferred compounds of the invention, the inhibitory activity of the compounds at the human serotonin transporter (hSERT) has been determined in vitro using porcine LLCPK cells (ATCC.) stably transfected with the hSERT (hSERT-LLCPK). The cells have been plated onto 96-well plates (10000 cells/well). After 24 hr, cells have been washed in uptake buffer (Hank's balanced salt solution + 20 mM Hepes) and pre-incubated for 10 minutes at 30°C with 50 µl of buffer containing the test compounds. 50 µl of 50 nM [³H] Serotonin (5-HT) solution (final concentration: 25 nM [³H] 5-HT) have been added and plates have been incubated for 7 min at 30°C, during which cells take up radiolabelled 5-HT. Aspirating the solution and rapidly washing the cells with cold buffer has terminated the uptake. The amount of radioactive 5-HT incorporated in the cells has then been measured by adding the scintillation cocktail directly onto the cells and reading the plate in the Top Count. The data have been digitally processed to obtain the pIC₅₀ values of the uptake inhibitors.

The action of the compounds of the invention at the NK₁ receptor and/or serotonin transporter may be determined by using conventional animal models.

Thus, the ability to bind at the NK₁ receptor and/or serotonin transporter was determined using the guinea pig pup isolation calls model as described by Pettijohn, Psychol. Rep., 1979 and Rupniak et al., Neuropharmacology, 2000.

The anti-anxiety activity obtained by the administration of a compound according to the invention can be demonstrated in the gerbil social interaction model, according to the method described by Cheeta et al. (Cheeta S. et al., 2001. Brain Research 915: 170-175).

Compounds of the invention are useful in the treatment of CNS disorders and psychotic disorders, in particular in the treatment or prevention of depressive states and/or in the treatment of anxiety as defined in, but not restricted to, Diagnostic Statistical of Mental Disorder (DSM) IV edition edit by American Psychiatric Association and/or national Classification Diseases 10th revision (ICD-10).). The various subtypes of the disorders mentioned herein are contemplated as part of the present invention. Numbers in brackets after the listed diseases below refer to the classification code in DSM-IV.

Within the context of the present invention, the term psychotic disorder includes Schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9): Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90).

Anxiety disorders including Social Anxiety Disorder, Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00):

Compounds of the invention are also useful in the treatment of Sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and

Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type.

Compounds of the invention may be also useful in the treatment of Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder

Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic-Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown)

Substance-Related Disorders such as Anabolic Steroids, Nitrate Inhalants and Nitrous Oxide.

Compounds of the invention may be also useful in the treatment of Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

Compounds of the invention may be also useful in the treatment of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

Compounds of the invention may be also useful in the treatment of eating disorders such as Anorexia Nervosa (307.1) including the subtypes Restricting Type and Binge-Eating/Purging Type; Bulimia Nervosa (307.51) including the subtypes Purging Type and

Nonpurging Type; Obesity; Compulsive Eating Disorder; and Eating Disorder Not Otherwise Specified (307.50).

The compounds of the invention may be also useful in the treatment Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender identity Disorder in Adolescents or Adults (302.85); and Sexual Disorder Not Otherwise Specified (302.9).

Compounds of the invention may be useful as analgesics. In particular, they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; burns; scar pain; itch and thalamic pain such as post stroke thalamic pain.

Compounds of the invention may be also useful in the treatment or prevention of the cognitive disorders. Cognitive disorders include dementia, amnestic disorders and cognitive disorders not otherwise specified.

Furthermore, compounds of the invention may be also useful as memory and/or cognition enhancers in healthy humans with no cognitive and/or memory deficit. Enhancement of memory and/or cognition including the treatment of memory and/or cognition impairment in other diseases such as schizophrenia, bipolar disorder, depression, other psychiatric disorders and psychotic conditions associated with cognitive impairment, e.g. Alzheimer's disease.

Compounds of the invention may be also useful as anti-inflammatory agents. In particular, they are useful in the treatment of inflammation in asthma, influenza, chronic bronchitis and rheumatoid arthritis; in the treatment of inflammatory diseases of the gastrointestinal tract such as Crohn's disease, ulcerative colitis, inflammatory bowel disease and non-steroidal anti-inflammatory drug induced damage; inflammatory diseases of the skin such as herpes and eczema; inflammatory diseases of the bladder such as cystitis and urge incontinence and dental inflammation.

Compounds of the invention may be also useful in the treatment of overactive bladder disorders including symptoms of urinary frequency, with or without urge incontinence, nocturia and urgency.

Compounds of the invention may be also useful in the treatment of allergic disorders, in particular allergic disorders of the skin such as urticaria, and allergic disorders of the airways such as rhinitis.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; anti-metabolites, e.g. cytarabine, methotrexate and 5-fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer; poisons; toxins such as toxins caused by metabolic disorders or by infection, e.g. gastritis, or released during bacterial or viral gastrointestinal infection; pregnancy; vestibular disorders, such as motion sickness, vertigo, dizziness and

Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia.

Compounds of the invention are also useful in the treatment of gastrointestinal disorders such as irritable bowel syndrome, gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia (such as ulcer-like dyspepsia, dysmotility-like dyspepsia and unspecified dyspepsia) chronic constipation; skin disorders such as psoriasis, pruritis and sunburn; vasospastic diseases such as angina, vascular headache and Reynaud's disease; cerebral ischeamia such as cerebral vasospasm following subarachnoid haemorrhage; fibrosing and collagen diseases such as scleroderma and eosinophilic fascioliasis; disorders related to immune enhancement or suppression such as systemic lupus erythematosus and rheumatic diseases such as fibrositis; and cough.

The compounds of the invention may be also useful in premenstrual dysphoric disorder (PMDD), in chronic fatigue syndrome and Multiple sclerosis.

Compounds of the invention have been found to exhibit anxiolytic and antidepressant activity in conventional tests. For example, in Guinea pig pups separation-induced vocalisations (Molewijk et al., 1996) and in the gerbil social interaction model, according to the method described by Cheeta et al. (Cheeta S. et al., 2001. Brain Research 915: 170-175).

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by tachykinins (including substance P and other neurokinins) and/or by selective inhibition of serotonin reuptake.

There is also provided as a further aspect of the invention the use of a compound of formula (I) or a pharmaceutically acceptable salt or solvate thereof in the treatment of conditions mediated by tachykinins (including substance P and other neurokinins) and/or by selective inhibition of the serotonin reuptake transporter protein.

In a further aspect there is provided the use of a compound of formula (I) or a pharmaceutically acceptable salt or solvate thereof in the preparation of a medicament for use in the treatment of depression and /or anxiety.

In a further aspect there is provided the use of a compound of formula (I) or a pharmaceutically acceptable salt or solvate thereof in the use in the treatment of depression and/or anxiety.

In an alternative or further aspect there is provided a method for the treatment of a mammal, including man, in particular in the treatment of conditions mediated by tachykinins, including substance P and other neurokinins and/or by selective inhibition of the serotonin reuptake transporter protein comprising administration of an effective amount of a compound of formula (I) or a pharmaceutically acceptable salt thereof.

In a further aspect of the present invention is provided a method for the treatment of a mammal, including man, in particular for the treatment of depression and/or anxiety which method comprises administration of an effective amount of a compound of formula (I) or a pharmaceutically acceptable salt or solvate thereof.

It will be appreciated that reference to treatment is intended to include prophylaxis as well as the alleviation of established symptoms.

Compounds of formula (I) may be administered as the raw chemical but the active ingredient is preferably presented as a pharmaceutical formulation.

Accordingly, the invention also provides a pharmaceutical composition which comprises at least one compound of formula (I) or a pharmaceutically acceptable salt thereof and formulated for administration by any convenient route. Such compositions are preferably in a form adapted for use in medicine, in particular human medicine, and can conveniently be formulated in a conventional manner using one or more pharmaceutically acceptable carriers or excipients.

Thus, compounds of formula (I) may be formulated for oral, buccal, parenteral, topical (including ophthalmic and nasal), depot or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.

Thus, for parenteral administration a daily dose will typically be in the range of 1 to about 100 mg, preferably 1 to 80 mg per day. For oral administration a daily dose will typically be within the range 1 to 300 mg e.g. 1 to 100 mg.

Compounds of formula (I), and salts and solvates thereof, may be prepared by the general methods outlined hereinafter. In the following description, the groups R, R₁,R₂, R₃, R₄, R₅, R₆, R₇, R₈, R₉, R₁₀, n, p, q and r have the meaning as previously defined for compounds of formula (I) unless otherwise stated.

Compounds of formula (I), wherein ---- is a single bond, R₃ represents hydrogen or C₁₋₄ alkyl and R₄ represents hydrogen, may be prepared by reductive N-alkylation of a compound of formula (III), 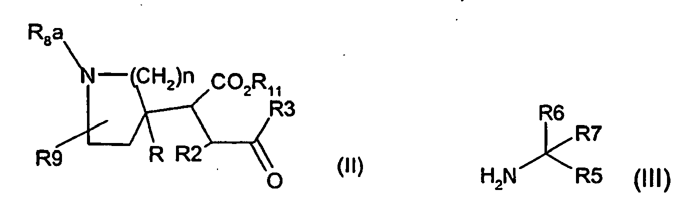 with the carbonylic compound (II), in which R₁₁ is C₁₋₄ alkyl (e.g methyl or ethyl), R₃ is hydrogen or C₁₋₄ alkyl and R₈ₐ has the meaning defined in formula (I) or is a nitrogen protecting group, to form an amino derivative (IV), 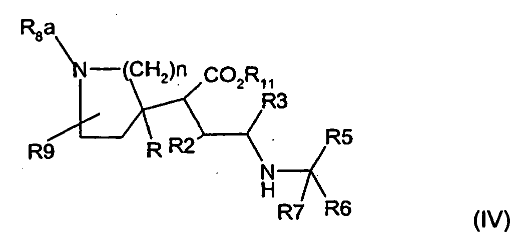 followed by cyclisation reaction in the presence of an alkoxy metal base e.g. sodium methoxy and where necessary followed by removal of the nitrogen protecting group.

The reductive N-alkylation may be carried out in an aprotic solvent such as dichloroethane or acetonitrile and in the presence of a suitable metal reducing agent such as sodium borohydride or sodium triacetoxyborohydride.

The cyclisation reaction is conveniently carried out in a solvent such as an alkanol e.g. methanol or ethanol at a temperature within the range 20° to 60°C.

If desired, compounds of formula (IV) may be isolated before the cyclisation reaction takes place.

Compounds of formula (I), wherein ---- is a single bond, R₃ is hydroxy and R₄ is hydrogen, may be prepared by oxidative cleavage of an allyl derivative of formula (V), 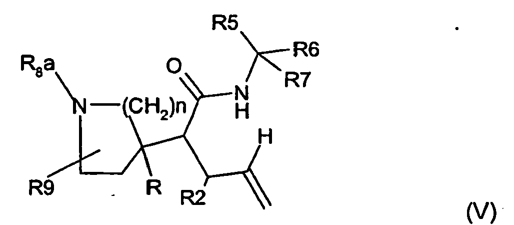 in which R₈ₐ is defined as in formula (II), to form the aldehyde (VI), followed by in situ cyclisation thereof and where necessary followed by removal of the nitrogen protecting group.

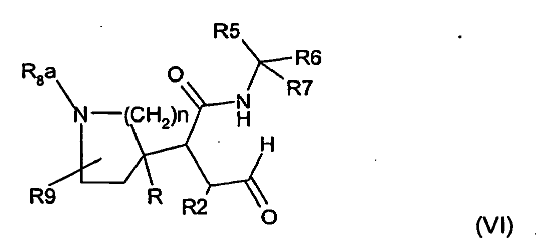

The oxidation may be carried out using conventional oxidating agents known in the art for converting an allyl group into a carbonyl group.

Thus, for example, the oxidative cleavage to form aldehyde (VI) is conveniently carried out using osmium tetroxide or potassium osmiate, followed by reaction with sodium periodate in water miscibile solvents (e.g tetrahydrofuran) and water preferably at room temperature. Alternative methods include ozonolysis using ozone followed by treatment with a suitable reducing agent such as dimethyl sulfide or trimethyl phosphite.

The cyclisation may be carried out by stirring the above mixture overnight at room temperature.

If desired, compounds of formula (VI) may be isolated before the cyclisation takes place.

Compounds of formula (I), wherein ---- is a single bond and R₃ together with R₄ represents =O, may be prepared by cyclisation of a compound of formula (VII), 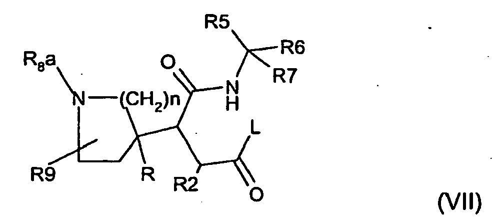 wherein R₈ₐ is defined as in formula (II) and L is a suitable leaving group.

Suitable leaving groups for this reaction (J.March, Advanced Organic Chemistry, 4th Edition, John Wiley and Sons, 1992, pp. 351-356) include, but are not limited to: halides e.g. chloro, bromo, iodo; C₁₋₄alkoxy.

The cyclisation reaction is conveniently carried out in the presence of a suitable base such as NaH and in an aprotic solvent such as THF and at a temperature ranging from 0°C to 80°C.

Alternatively, compounds wherein ---- is a single bond, R₈ represents (CH₂)rR₁₀ in which r is an integer from 1 to 4 and R₃ together with R₄ represents =O, may be prepared by reductive alkylation of a compound of formula(VIIA), 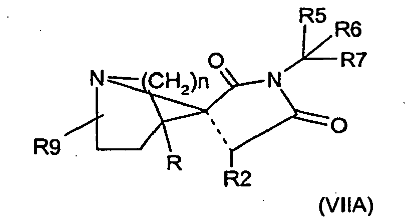 with an aldehyde, CH(O)(CH₂)ₘR₁₀ (VIIIa), wherein m is an integer from 0 to 3.

Alternatively, compounds of formula (I), wherein ----is a single bond may be prepared by N-alkylation of a compound of formula (VIII), 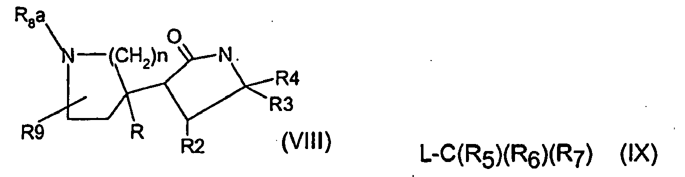 wherein R₈ₐ is defined as in formula(II) with a compound of formula (IX) in which L is a suitable leaving group as above defined. The N-alkylation may be carried out by in an aprotic solvent such as dichloroethane N,N dimethyl formamide or acetonitrile and in the presence of a base such as for example sodium hydride and conveniently at a temperature within the range 0 to 40°C.

Alternatively, compounds of formula (I), wherein ----is a double bond, R₃ represents hydrogen or C₁₋₄ alkyl and R₄ is hydrogen, may be prepared by oxidation of a compound of formula (Va) to obtain a compound of formula(VIa), which may be isolated if desired, followed by cyclisation with a strong acid such as sulfuric acid, trifluoro acetic, hydrochloric acid or p-toluensulfonic acid, by heating at temperature between 20°-80° C.

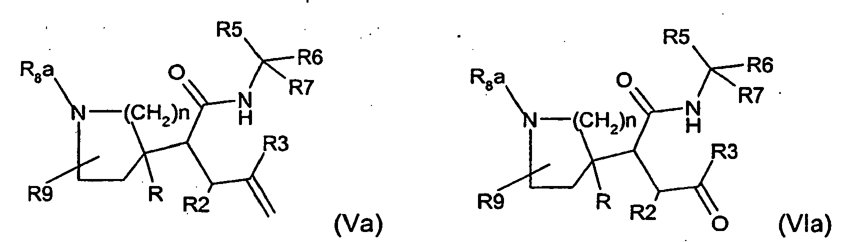

The oxidation may be carried out using the condition above described to convert compounds of formula (V) to compounds of formula (VI).

Compounds of formula(I) may be converted into other compounds of formula(I) . Thus, for example and by way of illustration rather than limitation, compounds of formula(I), wherein ----is a double bond, R₃ represents hydrogen and R₄ is hydrogen, may be prepared by reaction of a compound of formula(I) wherein ----- is a single bond, R₃ represents hydroxy, R₈ has the meaning defined in formula (I) or is a nitrogen protecting group, with an acid such as sulfuric acid, trifluoro acetic, hydrochloric acid or p-toluensulfonic acid.

For example, compounds of formula (I) wherein ---- is a double bond and R₃ is hydrogen, hydroxy, C₁₋₄alkyl or R₃ together with R₄ represents =O may be prepared by reaction of a compound of formula (I) wherein --- is a single bond, R₈ has the meaning defined in formula (I) or is a nitrogen protecting group and R₃ is hydrogen, hydroxy protected group, C₁₋₄alkyl or R₃ together with R₄ represents =O, with a suitable brominating agent followed by treatment with a base such as sodium ethoxide or methoxide and by removal of any protecting group.

A suitable brominating agent to be used in this reaction is N-bromosuccinimide.

For example, in a further embodiment, compounds of formula(I), wherein R₈ is (CH₂)rR₁₀ in which r is an integer from 1 to 4, may be prepared by reductive alkylation of a compound of formula(I), wherein R₈ is hydrogen, with an aldehyde, CH(O)(CH₂)ₘR₁₀ (VIIIa), wherein m is an integer from 0 to 3. The reductive N-alkylation may be carried out in an aprotic solvent such as dichloroethane or acetonitrile and in the presence of a suitable reducing agent such as sodium borohydride or sodium triacetoxyborohydride.

In a further embodiment, compounds of formula(I), wherein R₈ is (CH₂)rR₁₀ wherein r is 0 and R₁₀ is C₃₋₇ cycloalkyl, may be prepared by alkylation of a compound of formula (I), wherein R₈ is hydrogen, with a compound L- R₁₀ (IX a), wherein L is a suitable leaving group such as halogen (e.g iodine, chlorine or bromide).
The reaction is conveniently carried out in a solvent such as NN-dimethylformamide or tetrahydrofuran.
Examples of aprotic solvents are dichloromethane, NN-dimethylformamide, dimethylsulfoxide, tetrahydrofuran and the like.

For example, compounds of formula(I), wherein R₄ together with R₃ represents =CH₂, may be prepared by reaction of a compound of formula(I) in which R₃ and R₄ are hydrogen with formaldehyde in the presence of a suitable reducing agent such as sodium borohydride or sodium triacetoxyborohydride and a base such as sodium hydroxide.

Compounds of formula(II) may be prepared by oxidative cleavage of an allyl compound of formula (X), wherein R₈ₐ and R₁₁ have the meaning defined as in formula (II).

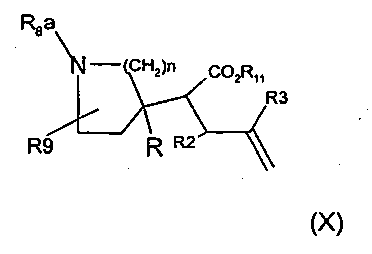

The oxidation is conveniently carried out in the presence of osmium tetroxide followed by reaction with sodium periodate. Alternative methods include ozonolysis using ozone followed by treatment with a suitable reducing agent such as dimethyl sulfide or trimethyl phosphite.
The reaction is carried out in a solvent such as N,N dimethylformamide or aqueous tetrahydrofuran at a temperature ranging from 0° to 25°C.

Compounds of formula (V) may be prepared by reaction of a compound of formula (X), wherein R₈ₐ and R₁₁ have the meaning defined in formula(II), with a strong base such as lithium hydroxide or sodium hydroxide to obtain a carboxylic acid of formula (XI) followed by reaction of an activated derivative thereof with a compound of formula (III) 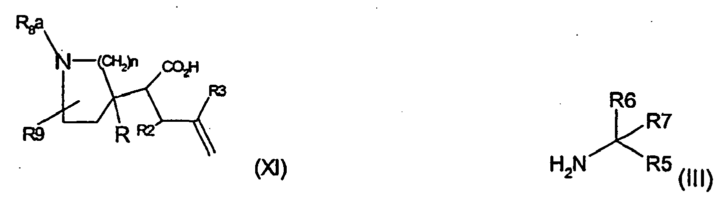 followed where necessary by removal of any nitrogen protecting group.
Suitable activated derivatives of the carboxyl group include the acyl halide, mixed anhydride, activated ester such as thioester or the derivative formed between the carboxylic acid group and a coupling agent such as that used in peptide chemistry, for example carbonyl diimidazole, O-(benzotriazol-1-yl)-N,N,N'N'-tetramethyluronium tetrafluoroborate or dicyclohexylcarbodiimide.
The reaction is preferably carried out in an aprotic solvent such as hydrocarbon, halohydrocarbon such as dichloromethane or an ether such as tetrahydrofuran, NN-dimethylformamide.
The activated derivatives of the carboxylic acid (XI) may be prepared by conventional means. A particular suitable activated derivative for use in this reaction is O-(benzotriazol-1-yl) -N,N,N',N'-tetramethyluronium tetrafluoroborate.
The reaction is suitably carried out in a solvent such as NN-dimethylformamide.

Compounds of formula (VII) may be prepared from (XII) by conventional means for 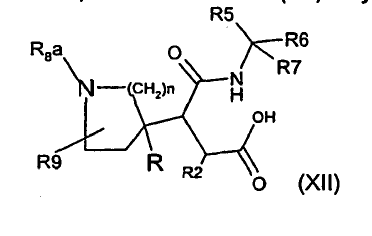 converting carboxylic groups into leaving groups.
Thus, for example, a compound of formula (VII) in which L is a chlorine atom may be prepared by treating a compound of formula (XII) with thionyl chloride in an aprotic solvent such as tetrahydrofuran and optionally in the presence of a tertiary organic base.

Compounds of formula (VIIA) may be prepared by reaction of a compound of formula(I) wherein R₈ is a nitrogen protecting group, ---- is a single bond, R₄ is hydrogen and R₃ is hydroxy with N₂SO₃ in the presence of inorganic acid (e.g hydrogen chloride).

Compounds of formula (XII) may be prepared by oxidation of a compound of formula (V).

The oxidation may be carried out using conventional oxidasing agents known in the art for converting an allyl group into a carboxyl group,using for example manganese dioxide.

Compounds of formula (X) may be prepared by reaction of a compound of formula (XIII), 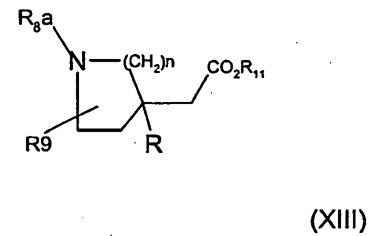 wherein R₈ₐ and R₁₁ have the meaning defined as in formula (II), with an allyl derivative CH2=C(R3)HC(R2)L (XIV) wherein L is a suitable leaving group such as halogen (e.g iodine, bromide).
The reaction is conveniently carried out in an aprotic solvent such as tetrahydrofuran in the presence of a base such as lithium bis trimethylsililamide at a temperature ranging from -70 to -60°C.

Compounds of formula (XIII) may be prepared by reaction of an activated derivative of compounds of formula (XV), wherein R₈ₐ has the meaning defined as in formula (II), with methanol or ethanol.

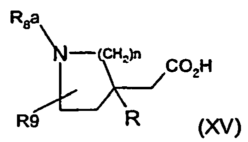

A particular suitable activated derivative for use in this reaction is O-(benzotriazol-1-yl) - N,N,N',N'-tetramethyluronium tetrafluoroborate.

Compounds of formula (XV) may be preprared from a cyano derivative (XVI), 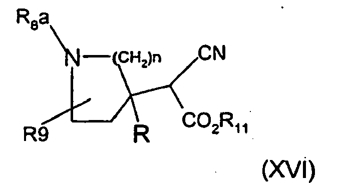 wherein R₈ₐ and R₁₁ have the meaning defined as in formula (II), by reaction with an acid, such as for example concentrated sulfuric acid.The reaction is conveniently carried out in a solvent such as acetic acid in the presence of water and by heating the reaction mixture up to 150°C.

Compounds of formula (XVI) may be prepared by reaction of a compound of formula (XVII), wherein R₈ₐ and R₁₁ have the meaning defined in formula (II), with a compound of formula (XVIII), wherein L is a suitable halogen (i.e bromine).

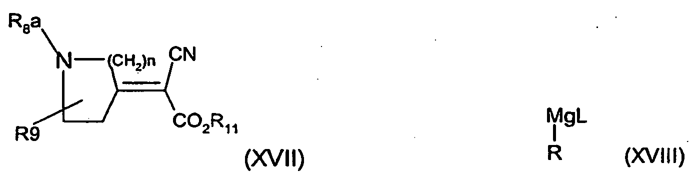

The reaction conveniently takes place in an aprotic solvent such as a hydrocarbon (e.g. toluene), ethers (e.g. tetrahydrofuran) and at a temperature within the range 0-25°C, optionally in the presence of cupper(I) salts such as for example cupper iodide.

Compounds of formula (XVII) may be prepared by reaction of a compound of formula (XIX) with a cyano derivative (XX), wherein R₈ₐ has the meaning defined in formula (I) or is a nitrogen protecting group.

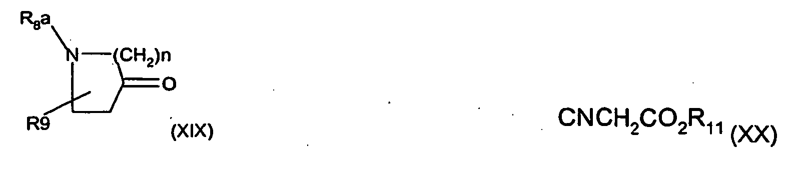

In a further embodiment. compounds of formula (XI) may be prepared by reaction of a compound of formula(XXI), wherein R₈ₐ has the meaning defined in formula (I) or is a nitrogen protecting group, 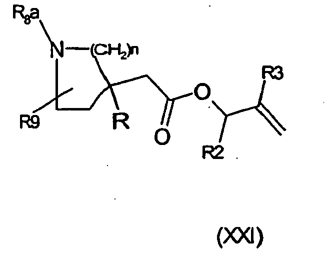 with a base such as lithium bis (trimethylsilyl)amide in the presence of a catalytic amount of a Palladium(0) complex such as tetrakis(triphenylphosphine)palladium.
The reaction is conveniently carried out in an aprotic solvent such tetrahydrofuran at a temperature within the range of -25° to 0°C.

Compounds of formula (XXI) may be prepared by reaction of a compound of formula (XXII), wherein R₈ₐ has the meaning defined in formula (I) or is a nitrogen protecting group, 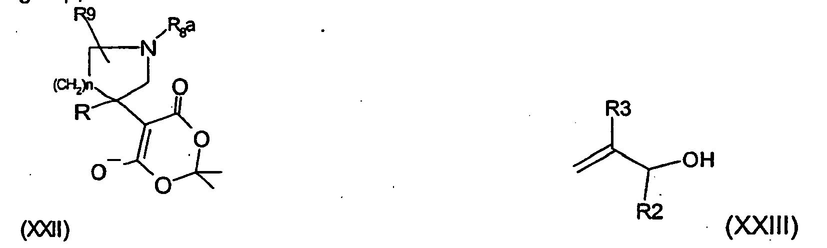 with an alcohol of formula (XXIII). The reaction conveniently takes place in an aprotic solvent such as a hydrocarbon (e.g toluene), or N,N dimethylformamide by heating. Compounds of formula (XXII) may be prepared by reaction of a compound of formula(XXIV) with a Grignard reagent of formula (XVIII).

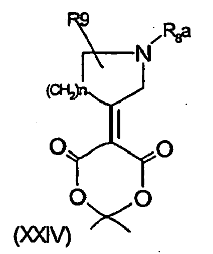

The reaction conveniently takes place in an aprotic solvent such as a hydrocarbon (e.g. toluene), ethers (e.g. tetrahydrofuran) and at a temperature within the range 0-25°C, optionally in the presence of cupper(I) salts such as for example cupper iodide.

Compounds of formula(XXIV) may be prepared by reaction of a compound of formula

XIX with Meldrums acid in a sovent such as alcohol (e.g methanol).

Compounds of formula (VIII) may be prepared by cleavage of a compound of formula (XXV), wherein R₈ₐ has the meaning defined in formula (I) or is a nitrogen protecting group,

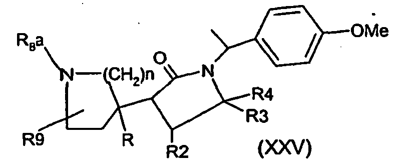

The reaction may be carried out in the presence of Cerio ammonium nitrate in an aprotic organic solvent such as acetonitrile.
Compounds (XXV) may be prepared by cyclisation of a compound of formula (XXVI) wherein R₈ₐ has the meaning defined in formula (I) or is a nitrogen protecting group.

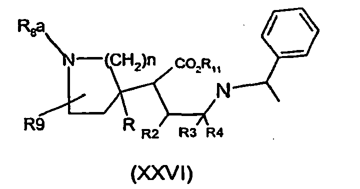

Cyclisation may be carried out in a solvent such an alcohol (e.g methanol) and in the presence of a suitable base such as sodium methoxy.
Compounds of formula (XXVI), wherein R₄ is hydrogen, may be prepared by reductive N-alkylation of 1-(4 methoxyphenyl)ethyl amine (XXVII) and a compound of formula(II). The reaction is conveniently carried out in an aprotic solvent such as dichloroethane or acetonitrile and in the presence of a suitable metal reducing agent such as sodium borohydride or sodium triacetoxyborohydride.

In a further embodiment compounds of formula (I), wherein R₂, R₃ and R₄ are hydrogen and ----- is a double bond, may be prepared from alcohol derivatives of formula (XXVIII), wherein R₈ₐ has the meaning defined in formula (I) or is a nitrogen protecting group, by conventional means for converting hydroxy group into a halide group followed by its elimination to form the corresponding double bond.

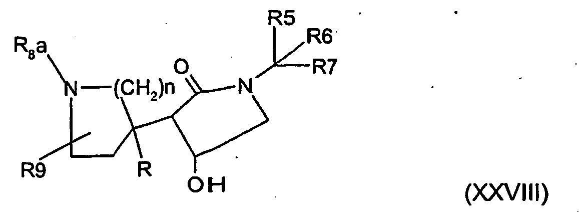

The reaction for converting (XXVIII) to halides may be carried out using for example halides of non-metallic elements such as thionyl chloride, phosphorus trichloride or phosphorus tribromide.
These reactions may be carried out in an aprotic solvent such as methylene chloride and at a temperature ranging from -5°C and 30°C.

Compounds of formula(XXVIII) may be prepared by reduction of a keto derivative (XXIX), wherein R₈ₐ has the meaning defined in formula (I) or is a nitrogen protecting group, 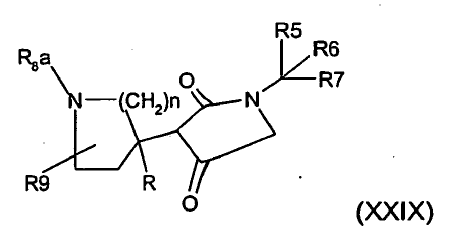 using the known method in the art to reduce a keto group to a hydroxy group thus for example using a borohydride reducting agent such as sodium borohydride, sodium cyano borohydride. The reaction is conviently carried out in a solvent such alkanol for example in aqueous isopropanol.
Compounds of formula(XXIX) may be prepared by decarboxylation of a compound of formula (XXX)

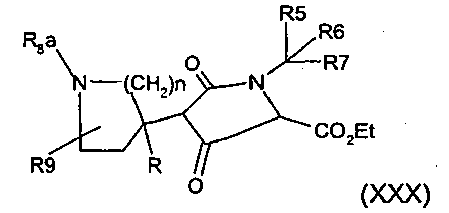

The decarboxylation may occur in the presence of an inorganic acid such as aqueous hydrochloric acid at a temperature ranging from 58 to 106°C.

Compounds of formula(XXX) may be prepared by cyclisation of a compound of formula (XXXI), wherein R₈ₐ has the meaning defined in formula (I) or is a nitrogen protecting group.

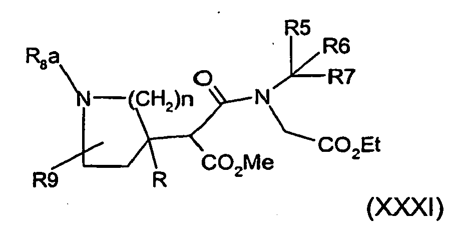

The cyclisation reaction is carried out in the presence of an alkoxy metal base e.g. sodium ethoxy in a protic solvent such as alcohol (e.g ethanol) at a temperature ranging from 0° to 5°C.

Compounds of formula(XXXI) may be prepared from esterification of an acid of formula (XXXII), 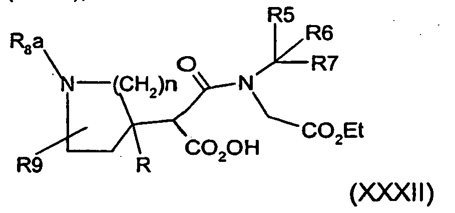 using the conventional method to convert an acid to methylester such as for example by reaction with dimethyl sulphate in the presence of an organic base such as potassium carbonate. The reaction may be carried out in an aprotic solvent such as NN dimethylformamide.
Compounds of formula(XXXII) may be prepared by reaction of a compound of formula (XXII) with a compound of formula (XXXIII)

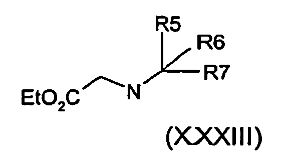

The reaction is suitably carried out in an aprotic solvent such as NN dimethylformamide by heating at 55°-58°C.

Amines (III) and enantiomers thereof (III) and compounds of formulae (XIX), (XX), (XXVII) and (XXXIII) are commercially available compounds or may be prepared by analogous methods to those used for known compounds.

It will be appreciated by those skilled in the art that it may be necessary to protect certain reactive substituents during some of the above procedures. Standard protection and deprotection techniques, such as those described in Greene T.W. Protective groups in organic synthesis, New York, Wiley (1981), can be used. Thus, for example when R 8a is a nitrogen protecting group, example of suitable groups include alkoxycarbonyl e.g. t-butoxycarbonyl, benzyloxycarbonyl, arylsulphonyl e.g. phenysulphonyl or 2-trimethylsilylethoxymethyl . Carboxylic acid groups can be protected as esters. Examples of suitable hydroxy protecting reagents include acetic anhydride, benzoic anhydride or a trialkylsilyl chloride. Aldehyde or ketone groups can be protected as acetals, ketals, thioacetals or thioketals. Deprotection of such groups is achieved using conventional procedures well known in the art. For example, protecting groups such as t-butyloxycarbonyl may be removed using an acid such as hydrochloric or trifluroroacetic acid in a suitable solvent such as dichloromethane, diethylether, isopropanol or mixtures thereof.

When a specific enantiomer of a compound of general formula (I) is required, this may be obtained for example by resolution of a corresponding enantiomeric mixture of a compound of formula (I) using conventional methods.
Thus, for example, specific enantiomers of the compounds of formula (I) may be obtained from the corresponding enantiomeric mixture of a compound of formula (I) using chiral HPLC procedure.

Alternatively, enantiomers of a compound of general formula (I) may be synthesed from the appropriate optically active intermediates using any of the general processes described herein.
Thus, in a one embodiment of the invention the enantiomers of the compound of formula (I) may be prepared by reaction of a chiral amine (III) using any of the processes described above for preparing compounds of formula (I) from amine (III).
The chiral amine (III) may be prepared from the corresponding racemic amine (III) using any conventional procedures such as salt formation with a suitable optically active acid such as for example di-p-toluoyl-D-tartaric acid, (*S*)-methoxyphenylacetic acid or di-p-toluoyl-L-tartaric acid, or using chiral HPLC procedure.

In a further embodiment the enantiomers of the compound of formula (I) may be prepared by reaction of a chiral amine (XXVII) using any of the processes described above for preparing compounds of formula (I) from amine (XXVII).

Where it is desired to isolate a compound of formula (I) as a salt, for example a pharmaceutically acceptable salt, this may be achieved by reacting a compound of formula (I) in the form of the free base with an appropriate amount of suitable acid and in a suitable solvent such as an alcohol (e.g. ethanol or methanol), an ester (e.g. ethyl acetate) or an ether (e.g. diethyl ether, *tert*-butylmethyl ether or tetrahydrofuran).

### Biology Data

The affinity of the compound of the invention for the NK₁ receptor was determined using the NK₁ receptor binding affinity method (Scintillation proximity assay (SPA)) measured *in vitro* by the compounds' ability to displace [¹²⁵I]Tyr8-Substance P (SP) from recombinant human NK₁ receptors expressed in Chinese Hamster Ovary (CHO) cell membranes. The affinity values are expressed as negative logarithm of the inhibition constant (pKᵢ) of displacer ligands. The pKᵢ values obtained as the average of at least two determinations with representative compounds of the invention are within the range of 8.6 to 6.6. Particularly pKᵢ values obtained as the average of at least two determinations with Examples N°1,3,5,6,11,14,15 and 16 are within the range of 8.65 to 8.07.

The affinity of the preferred compounds of the invention for the NK₁ receptor was also determined using the NK₁ receptor binding affinity method measuring *in vitro* by the compounds' ability to displace [³H] - substance P (SP) from recombinant human NK₁ receptors expressed in Chinese Hamster Ovary (CHO) cell membranes. The affinity values are expressed as negative logarithm of the inhibition constant of displacer ligands (pKᵢ). The pKᵢ values obtained as the average of at least two determinations with representative compounds of the invention are within the range of 9.32 to 8.72.

The antagonism of the compounds of the invention towards human NK₁ receptors was determined in a functional assay using FLIPR technology measuring their effect to inhibit the intracellular calcium increase induced by SP in both Human-NK₁-CHO cells and human U2OS cells transducted with NK₁ BacMan virus. The potency of the antagonist is expressed as fpKᵢ. or pK_{b}. The fpKi values obtained as the average of at least two determinations with representative compounds of the invention are within the range of 7.83 to 5.01. Particularly, fpKi values obtained as the average of at least two determinations with Examples N°1,3,5,6,11,14,15 and 16 are within the range of 7.83 to 5.79 with pK_{b} values within the range of 8.65 to 6.

The affinity of the compounds of the invention for the serotonin transporter was determined using the hSERT binding affinity method and measuring *in vitro* the compounds' ability to displace [³H]-citalopram from recombinant human serotonin transporter expressed in Porcine Epithelial Kidney LLCPK cell membranes. The affinity values are expressed as negative logarithm of the inhibition constant of displacer ligands (pKᵢ). The pKᵢ values obtained as the average of at least two determinations with representative compounds of the invention are within the range of 9.4 to 6.5. Particularly, pKᵢ values obtained as the average of at least two determinations with Examples

N°1,3,5,6,11,14,15 and 16 are within the range of 8.90 to 8.04.

The potency of the compounds of the invention for inhibiting the uptake of 5-HT through the serotonin transporter was determined using the hSERT uptake method and measuring *in vitro* the compounds' ability to displace [³H] 5-HT uptake from recombinant human serotonin transporter expressed in Porcine Epithelial Kidney LLCPK cells. The potency values are expressed as negative logarithm of the inhibition constant of displacer ligands that causes 50% inhibiton of the maximal 5-HT response (pIC₅₀). The pIC₅₀ values obtained as the average of at least two determinations with representative compounds of the invention are within the range of 7.5 to 5.7.

### Pharmacy examples

### Tablets

Tablets may be prepared by the normal method such as direct compression or wet granulation.
The tablets may be film coated with a suitable film forming material such for example Opadry using standard technique.

### Example A

**Tablets (Direct compression)/ Capsules**

| | |
|---|---|
| Active ingredient | 20.0mg |
| Dibasic Calcium Phospate | 123.253 mg |
| Crospovidone | 4.5mg |
| Magnesium Stearate | 1.5mg |
| Colloidal Silicon Dioxide | 0.75mg |

The active ingredient is blended with the other excipients. The blend can be used to fill gelatin capsules or compressed to form tablets using appropriate punches. The tablets can be coated using conventional techniques and coatings.

### Example B

**Tablets /Capsules (Wet granulation)**

| | |
|---|---|
| Active ingredient | 20.0mg |
| PVP | 3 mg |
| Avicel | 120.25mg |
| Crospovidone | 4.5mg |
| Magnesium Stearate | 1.5mg |
| Colloidal Silicon Dioxide | 0.75mg |

The Active ingredient and the intragranular excipients (PVP, Avicel, Crospovidone) are mixed at high main agitator (impeller) for a few minutes. The resulting mixture are wetted, adding the liquid binder (water) by spraying it into the powder while both agitators, impeller and chopper, are running at a low speed. The particles are let growing as resulting from the mechanical energy supplied (both agitators running at high speed) and dried by granulator chamber walls warming. The granules thus obtained are sieved and the other extragranular excipients (Magnesium Stearate, Colloidal Silicon Dioxide) are added and then mixed. The resulting mixture is compressed to obtained tablets or encapsulated to obtain capsules.

### Example C

**Tablets /Capsules(Dry Granulation)**

| | |
|---|---|
| Active ingredient | 20.0mg |
| PVP | 2 mg |
| Avicel | 121mg |
| Crospovidone XL | 4.5mg |
| Magnesium Stearate | 1.5mg |
| Colloidal Silicon Dioxide | 1mg |

The Active ingredient and the intragranular excipients (PVP, Avicel, Crospovidone) are mixed and the mixture is compated by compression with flat faced punches or by passing through two grooved rollers revolving toward each other, in order to obtain the "slugs" The other extragranular excipients (Magnesium Stearate, Colloidal Silicon Dioxide) are added and then mixed. The resulting mixture is compressed to obtained tablets or encapsulated to obtain capsules.

### Example D

### Infusion

| | | |
|---|---|---|
| Active ingredient | 2-50 | mg/mBuffer |
| solution pH 4.5 suitable for infusion (e.g. sodium chloride in NaCl 0.9% or 5% dextrose) | qs to 100ml | |

The formulation may be packed in glass vials or plastic bag.

In the Intermediates and Examples unless otherwise stated:
Melting points (map.) were determined on a Buchan map. apparatus and are uncorrected. r.t. refers to room temperature. Infrared spectra (IR) were measured in chloroform or nujol solutions on a FT-IR instrument.
Proton Magnetic Resonance (NMR) spectra were recorded on Varian instruments at 300, 400 or 500 MHz, on Bruker instrument at 300 MHz, chemical shifts are reported in ppm (δ) using the residual solvent line as internal standard. Splitting patterns are designed as s, singlet; d, doublet; t, triplet; q, quartet; m, multiplet; b, broad. The NMR spectra were recorded at temperature ranging from 25 to 90°C; when more than one conformer was detected the chemical shifts for the most abundant one is reported.
Mass spectra (MS) were taken on a 4 II triple quadrupole Mass Spectrometer (Micromass UK) or on a Agilent MSD 1100 Mass Spectrometer, operating in ES (+) and ES (-) ionization mode or on a Agilent LC/MSD 1100 Mass Spectrometer, operating in ES (+) and ES (-) ionization mode coupled with HPLC instrument Agilent 1100 Series HPLC (LC/MS -ES /+):refers to analysis performed on a Supelcosil ABZ +Plus (33x4.6 mm, 3µm) (mobile phase: 100% [water +0.1% HCO₂H] for 1 min, then from 100% [water +0.1% HCO₂H] to 5% [water +0.1% HCO₂H] and 95% [CH₃CN ] in 5 min, finally under these conditions for 2 min; T=40°C; flux= 1 mL/min;
HPLC (LC/MS -ES /-): refers to analysis performed on a Supelcosil ABZ +Plus (33x4.6 mm, 3µm) (mobile phase: 100% [water +0.05% NH₃] for 1 min, then from 100% [water +0.05% NH₃ to 5% [water +0.05% NH₃] and 95% [CH₃CN] in 5 min, finally under these conditions for 2 min; T=40°C; flux= 1 mL/min]. In the mass spectra only one peak in the molecular ion cluster is reported.
The X-ray powder diffraction pattern of a crystalline form of the compound of the invention was obtained by loading the sample into the diffractometer (Siemens D5005 X-ray diffractometer equipped with q/q goniometer, scintillation counter and graphite monochromator. The diffractometer was set up with the instrumental parameters given below:
   Instrumental parameters
   Monochromatic radiation: Cu - 1.54056/1.54439
   2θ range: 2°-45° 2θ
   Generator voltage/current: 40kV/50mA
   Step size: 0.02° 2θ
   Time per step: 2 sec-1
   Rotation: on
   Divergence/Antiscattering slit: variable
   Sample holder: round cavity or low-background plate.

The spectrum obtained was analysed using the data evaluation software EVA 7.0.
Optical rotations were determined at 20°C with a Jasco DIP360 instrument (I=10 cm, cell volume = 1 mL, λ = 589 nm). Flash silica gel chromatography was carried out over silica gel 230-400 mesh supplied by Merck AG Darmstadt, Germany or over Varian Mega Be-Si pre-packed cartridges or over pre-packed Biotage silica cartridges.
T.I.c. refers to thin layer chromatography on 0.25 mm silica gel plates (60F-254 Merck) and visualized with UV light. For phase separations performed by using microfiltration devices: phase separation cartridge with polypropylene frit by Whatman or Alltech. SCX means: SCX-cartridges (loading 0.75mmol\g) by Varian.
Solutions were dried over anhydrous sodium sulphate.
Methylene chloride was redistilled over calcium hydride and tetrahydrofuran was redistilled over sodium.
The following abbreviations are used in the text: AcOEt = ethyl acetate, CH = cyclohexane, DCE = dichloroethane, DCM = methylene chloride, DIPEA = N,N-diisopropylethylamine, DMF = N,N'-dimethylformamide, Et2O = diethyl ether, EtOH = ethanol, MeOH = methanol, TEA = triethylamine, THF = tetrahydrofuran, TFA = trifluoroacetic acid, CH3CN= acetonitrile, TBTU = O-(benzotriazol-1-yl)-N,N,N'N'-tetramethyluronium tetrafluoroborate, std= saturated.

**Enantiomer 1 or enantiomer 2** means a compound of the invention or an intermediate thereof as a single enantiomer whose configuration was not determined.
**Chain enantiomer 1 or chain enantiomer 2** refers to a compound of the invention or an intermediate thereof (i.e (Ib),(IV), (V) or (VI)) wherein R₆ and R₇ are not the same group, having a single undetermined configuration at the carbon atom shown as ** in the formula (Ib),(IV), (V) or (VI).

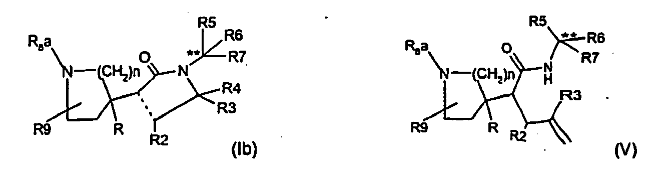

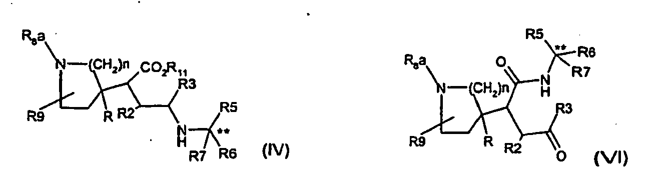

**Diastereoisomer 1 or diastereoisomer 2** refers to a compound of the invention or an intermediate thereof having at leat two stereogenic center and wherein ---- is a single bond, having a single but undetermined configuration at the carbon atom shown as *** in the formula(Ic)

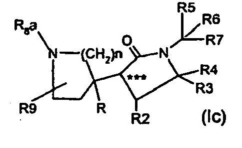

### Intermediate 1

### [1-(3,5-Dichlorophenyl)ethyl]amine

A solution of 3,5-dichlorobenzaldehyde (54.3 g) in dry THF (300 mL) was added dropwise to lithium bis(trimethylsilyl)-amide (1M solution in THF - 340 mL) at -30°C under a Nitrogen atmosphere. The resulting orange mixture was allowed to warm to - 5°C under stirring in a Nitrogen atmosphere in 1 h, then it was cooled down to -60°C and methyllithium (1.6M solution in Et2O - 290 mL) was added keeping the internal temperature of the reaction mixture < -55°C.
The resulting dark violet reaction mixture was stirred for 1 h at -60°C under a Nitrogen atmosphere, then it was carefully quenched at -60°C with aqueous 2N hydrochloric acid solution (20 mL) followed by aqueous 6N hydrochloric acid solution to pH = 2. The reaction mixture was concentrated *in vacuo* and the aqueous residue was washed with 1:1 CH/Et2O (500 mL). The separated aqueous phase was then made basic (pH = 14) at 0°C with NaOH pellets. The basic aqueous phase was extracted with Et2O (4 x 400 mL), the collected organic layers were dried and concentrated *in vacuo* to give the title compound (60 g) as a yellow oil.
T.I.c.: DCM/MeOH 9:1, Rf=0.5 (detection with ninhydrine).
NMR (CDCl₃): δ (ppm) 7.25 - 7.15 (m, 3H); 4.05 (q, 1H); 1.35 (d, 3H).
MS (ES/+): m/z= 190 [M+H]⁺.

### Intermediate 2 and Intermediate 3

### [1-(3,5-Dichlorophenyl)ethyl]amine (Enantiomer 1)and [1-(3,5-Dichlorophenyl)ethyl]amine (Enantiomer 2)

A solution of (*S*)-methoxyphenylacetic acid (23 g) in acetone (140 mL) was added to a solution of intermediate **1** (25 g) in acetone (140 mL). The thick suspension was heated at 56°C for 1 h then it was stirred at r.t. overnight. The slurry was filtered and the solid residue washed with acetone (200 mL). The solid (47 g) was triturated in acetone (500 mL) by heating to reflux for 1 h, cooling to r.t. and stirring overnight. The suspension was filtered and the solid residue (29 g) washed with acetone (500 mL) and triturated three times as described above to give (*S*)-methoxyphenylacetic acid salt of [1-(3,5-dichlorophenyl)-ethyl]amine (16.6 g). The solid was stirred in a mixture of aqueous saturated sodium hydrogen carbonate solution (200 mL) and DCM (200 mL). The organic phase was separated, washed with brine (200 mL), dried and concentrated *in vacuo* to give the title compound intermediate **2** (8.2 g) as a colourless oil.
The same procedure was performed on a distinct batch of intermediate 1 (7.5 g) to obtain the title compound intermediate **2** (1.6 g); the mother liquors from the precipitation were evaporated *in vacuo* to give a residue (9.5 g), which was treated with aqueous saturated sodium hydrogen carbonate solution (50 mL) and extracted with DCM (50 mL). The colourless oil thus obtained (5 g) was treated with (*R*)-methoxyphenylacetic acid (4.3 g) in acetone as described above (one precipitation and two triturations) to give (*R*)-methoxyphenylacetic acid salt of [1-(3,5-dichloro-phenyl)-ethyl]amine (3.26 g). The solid was stirred in a mixture of aqueous saturated sodium hydrogen carbonate solution (50 mL) and DCM (50 mL). The organic phase was washed with brine (50 mL), dried and concentrated *in vacuo* to give the title compound intermediate **3** (1.6 g) as colourless oil.

### Intermediate 2: (Enantiomer 1)

NMR (CDCl₃): δ (ppm) 7.25 - 7.15 (m, 3H); 4.05 (q, 1H); 1.35 (d, 3H).
MS (ES/+): m/z= 190 [M+H]⁺.
HPLC (column: Chiral-AGP 15cm x 2mm, 5µm; injection volume=1µL; mobile phase: ammonium phosphate buffer 100mM pH=4.4 / MeOH isocratic 99/1 % v/v; flow rate= 0.13 mL/min; detection: λ=210 nm): retention time = 5.4 minutes; purity (a/a %) >98%.

### Intermediate 3: (Enantiomer 2)

NMR (CDCl₃): δ (ppm) 7.25 - 7.15 (m, 3H); 4.05 (q, 1H); 1.35 (d, 3H).
MS (ES/+): m/z= 190 [M+H]⁺.
HPLC (column: Chiral-AGP 15cm x 2mm, 5µm; injection volume=1µL; mobile phase: ammonium phosphate buffer 100mM pH=4.4 / MeOH isocratic 99/1 % v/v; flow rate= 0.13 mL/min; detection: λ=210 nm): retention time = 6.2 minutes; purity (a/a %) >99%.

### Intermediate 4

### [1-(3-Chloro-1-naphthalenyl)ethyl]amine

A solution of 3-chloro-naphthalenecarbaldehyde (1.93 g) in dry THF (12 mL) was added dropwise to lithium bis(trimethylsilyl)-amide (1M solution in THF - 10.1 mL) at -30°C under a Nitrogen atmosphere. The resulting yellow mixture was stirred under a Nitrogen atmosphere from -30°C to -5°C for 1 h, then it was cooled down to -60°C and methyllithium (1.6M solution in Et2O - 11 mL) was added keeping the internal temperature of the reaction mixture < -55°C.
The resulting dark violet reaction mixture was stirred for 40 minutes at -50°C under a Nitrogen atmosphere, then it was carefully quenched at -50°C with aqueous 2N hydrochloric acid solution (30 mL) until pH = 2. The reaction was concentrated *in vacuo* and the aqueous residue was washed with 1:1 CH/Et2O (50 mL). The separated aqueous phase was then made basic (pH = 14) at 0°C with NaOH pellets. This basic aqueous phase was extracted with Et2O (3 x 60 mL), the collected organic layers were dried and concentrated *in vacuo* to give the title compound (1.12 g) as a yellow oil.
T.I.c.: AcOEt/MeOH 8:2, Rf=0.25 (detection with ninhydrine).
NMR (d₆-DMSO): δ (ppm) 8.14 (dd, 1H); 7.94 - 7.85 (m, 2H); 7.73 (d, 1H); 7.58 - 7.50 (m, 2H); 4.80 (q, 1H); 1.35 (d, 3H).
MS (ES/+): m/z= 189 [M-NH₂]⁺.

### Intermediate 5 and Intermediate 6

### [(1R)-1-(3-Chloro-1-naphthalenyl)ethyl]amine and [(1S)-1-(3-Chloro-1-naphthalenyl)ethyl]amine

To a solution of intermediate **4** (1.12 g) in acetone (10 mL), a solution of (*S*)-methoxyphenylacetic acid (0.9 g) in acetone (10 mL) was added. The thick suspension was heated at 56°C for 40 minutes then it was stirred at r.t. overnight. The slurry was filtered and the solid residue washed with acetone (10 mL). The solid (0.87 g) was triturated in acetone (10 mL) by heating to reflux for 1 h, cooling to r.t. and stirring overnight. The suspension was filtered and the solid residue (0.6 g) washed with acetone (10 mL) and triturated once again as described above to give (*S*)-methoxyphenylacetic acid salt of [(1*R*)-1-(3-Chloro-1-naphthalenyl)ethyl]amine (0.45 g). The solid was stirred in a mixture of aqueous saturated sodium hydrogen carbonate solution (20 mL) and DCM (20 mL). The organic phase was washed with brine (20 mL), dried and concentrated in *vacuo* to give the title compound intermediate **5** (0.25 g) as a colourless oil.

The mother liquors from the precipitation and first trituration were collected, concentrated *in vacuo,* treated with aqueous saturated sodium hydrogen carbonate solution (20 mL) and extracted with DCM (20 mL). The colourless oil thus obtained (1 g) was treated with (*R*)-methoxyphenylacetic acid (0.8 g) in acetone (8 mL) as described above (one precipitation and two triturations) to give (*R*)-methoxyphenylacetic acid salt of [(1*S*)-1-(3-Chloro-1-naphthalenyl)ethyl]amine (0.43 g). A portion of this solid (200 mg) was stirred in a mixture of aqueous saturated sodium hydrogen carbonate solution (10 mL) and DCM (10 mL). The organic phase was washed with brine (20 mL), dried and concentrated in *vacuo* to give the title compound intermediate **6** (0.100 g) as colourless oil.

### Intermediate 5:

NMR (d₆-DMSO): δ (ppm) 8.14 (dd, 1H); 7.94 - 7.85 (m, 2H); 7.73 (d, 1H); 7.58 - 7.50 (m, 2H); 4.80 (q, 1H); 1.35 (d, 3H).
MS (ES/+): m/z=189 [M-NH₂]⁺.
SFC (Gilson) analytical conditions: column: Chiralcel OD 25 x 4.6mm; mobile phase: CO₂ / Ethanol +0.1% Isopropanol 92/8 v/v; flow rate= 2.5 mUmin; P = 180 bar; T = 35°C; detection: λ=225 nm): retention time = 13.8 minutes; purity (a/a %) >99%.

### Intermediate 6:

NMR (d₆-DMSO): δ (ppm) 8.14 (dd, 1H); 7.94 - 7.85 (m, 2H); 7.73 (d, 1H); 7.58 - 7.50 (m, 2H); 4.80 (q, 1H); 1.35 (d, 3H).
MS (ES/+): m/z=189 [M-NH₂]⁺.
SFC (Gilson) analytical conditions: column: Chiralcel OD 25 x 4.6mm; mobile phase: CO₂ / Ethanol + 0.1% Isopropanol 92/8 v/v; flow rate= 2.5 mUmin; P = 180 bar; T = 35°C; detection: λ=225 nm): retention time = 12.4 minutes; purity (a/a %) >99%.

### Intermediate 7

### 1-(3-Chloro-1-naphthalenyl)methanamine

The 3-chloro-1-naphthalenecarbaldehyde (2 g) dissolved in dry THF (12 mL) was added dropwise to lithium bis(trimethylsilyl)amide 1 M in THF (11.5 mL) previously cooled at - 40°C. The resulting yellow mixture was stirred from -40°C to -20°C over 1.5h; then it was cooled down at -50°C and lithium aluminium hydride 1M in Et2O (10.6 mL) was added; the mixture was stirred at -40°C for 2h then it was quenched with HCl 2N (10 mL) and allowed to reach room temperature. The reaction mixture was diluted with further aqueous HCl 2N solution (20 mL) and extracted with CH/Et₂O 1/1 (50 mL). The acidic aqueous phase was basified at 0°C with NaOH pellets until pH=14, then it was extracted with diethyl ether (2 x 150 mL). The organic phase was dried and concentrated *in vacuo* to give the title compound (1.78 g) as a white solid.
T.I.c.: DCM/MeOH 8:2, Rf=0.43 (detection with ninhydrine).
MS (ES/+): m/z= 175 [M-NH₂]⁺.

### Intermediate 8

### 4-(Aminomethyl)-2-naphthalenecarbonitrile

The 4-(hydroxymethyl)-2-naphthalenecarbonitrile (200mg) is dissolved, under inert atmosphere, into a 3 necked flask equipped with a reflux condenser, with the solvent mixture DMF (4 mL) and CCl4 (1mL). PPh3 and NaN3 are added in sequence and then the reaction mixture is heated at 90°C for 2h (disappearance of the starting material). H2O (5mL) is added , followed by AcOEt (20mL); the phase are separated and organic phase is washed with brine (10mL). The crude obtained after evaporation of the solvents is dissolved in THF (3mL) and H2O (1 mL) and the reaction mixture is stirred overnight at r.t. before being worked up by dilution with AcOEt (100 mL) and washing with H20 (15mL) and then with brine (15mL). The crude obtained after evaporation of the solvents is purified by flash chromatography (from DCM to DCM/MeOH 95/5) affording 0.138g of the desired compound as a pale yellow oil.

NMR (CDCl₃): δ (ppm) 8.14 (s, 1H); 8.08 (d, 1H); 7.89 (d, 1H); 7.69 - 7.60 (m, 2H); 7.66 (s, 1H); 4.36 (s, 2H).

### Intermediate 9

### 4-(Bromomethyl)-2-naphthalenecarbonitrile

To a solution of 4-(hydroxymethyl)-2-naphthalenecarbonitrile (200 mg) in dry DCE (5 mL), CBr₄ (542.8 mg) and (Ph)₃P were added, the resulting mixture was stirred at r.t. for 40 min and then quenched with 40 mL of water. The aqueous phase was washed with DCM (3 x 40 mL). The combined organic extracts were dried, concentrated, and purified by flash chromatography (CH/AcOEt from 8:2 to 1:1) to give the title compound (216 mg) as a white foam.
MS (ES/+): m/z=247 [M+H]⁺.

### Intermediate 10 and 11

### Methyl 4-bromo-7-fluoro-2-naphthalenecarboxylateand methyl 4-bromo-6-fluoro-2-naphthalenecarboxylate

Isoamylnitrite (3.56 mL) dissolved in dimethoxyethane (18 mL) and a solution of 2-amino-4-fluorobenzoic acid (4.11 g) in dimethoxyethane (18 mL) were both added in separate streams at matching rate over 90 min to a refluxing solution of 3-bromo-coumalic acid methyl ester (3 g) in dimethoxyethane (25 mL) and catalytic amount of trifluoroacetic acid (21 mg). The reaction mixture was heated under reflux for a further 1 h after the end of the additions.Then the temperature was decreased to 50°C and toluene (40 mL) was added. The mixture was then cooled to r.t., the phases were separated and the organic one was extracted with aqueous 0.5 M NaOH (20 mL), aqueous 5% sodium metabisolfite (20 mL), water (20 mL), aqueous 2M HCl (20 mL) and finally water (20 mL).
Solvent was then removed by evaporation under reduced pressure to give a crude which was purified by Biotage Flash Chromatography eluting with CH:AcOEt= 95:5 to give the title compound **10** (625 mg) and title compound **11** (547 mg) as yellow oils.

### Intermediate 10

T.I.c.: CH/AcOEt 7:3, Rf=0.67.
NMR (CDCl₃): δ (ppm) 8.48 (s, 1H); 8.31 (s, 1 H); 8.27 (dd, 1H); 7.56 (dd, 1H); 7.46 (td, 1H);3.96 (s, 3H).

### Intermediate 11

T.I.c.: CH/AcOEt 7:3, Rf=0.60.
NMR (CDCl₃): δ (ppm) 8.53 (s, 1H); 8.36 (s, 1H); 7.94 (dd, 1H); 7.88 (d, 1H); 7. 34 (td, 1H); 3.96 (s, 3H).

### Intermediate 12

### 4-Bromo-7-fluoro-2-naphthalenecarboxylic acid

Intermediate **10** (970 mg) was dissolved in THF (20 mL) and water (10 mL) and then LiOH·H₂O (577 mg) was added. The mixture was heated at 80°C for 2h. Then it was cooled to r.t. and aqueous 2M HCl solution was added. The aqueous phase was extracted with AcOEt and the organic extracts were dried and evaporated *in vacuo* to give the title compound (850 mg) as a yellow solid.
NMR (d₆-DMSO): δ (ppm) 13.4 (bs, 1H); 8.63 (s, 1H); 8.23 (dd, 1H); 8.18 (s, 1H); 8.07 (dd, 1H);7.71 (td,1H).

### Intermediate 13

### 4-bromo-6-fluoro-2-naphthalenecarboxylic acid

Intermediate **11** (3.89 g) was dissolved in THF (60 mL) and water (30 mL) and then LiOH·H₂O (2.32 g) was added. The mixture was heated at 80°C for 2h. Then it was cooled to r.t. and aqueous 2M HCl solution was added. The aqueous phase was extracted with AcOEt and the organic extracts were dried and evaporated *in vacuo* to give the title compound (3.4 g) as a yellow solid.
HPLC (LC/MS -ES /-): t_{R} = 4.00 min
MS (ES/-): m/z=267 [M-H]⁻

### Intermediate 14

### 4-Bromo-7-fluoro-N-hydroxy-2-naphthalenecarboxamide

Intermediate **12** (850 mg) was dissolved in DMF (3 mL) and then TBTU (1.32 g) and DIPEA (1.9 mL) were added. The mixture was stirred for 30 min under a Nitrogen atmophere and then hydroxylamine hydrochloride (286 mg) was added; after stirring for 2 h aqueous std NH₄Cl solution was added and the aqueous phase was extracted with AcOEt. The organic phase was then washed with aqueous std NaHCO₃ solution, dried and evaporated *in vacuo* to give a crude which was triturated with pentane to afford the title compound (360 mg) as a withish solid.
MS (ES/+): m/z=284 [M+H]⁺.

### Intermediate 15

### 4-Bromo-7-fluoro-2-naphthalenecarbonitrile

Intermediate **14** (360 mg) was suspended in fluoro benzene (11 mL) under Nitrogen atmosphere at r.t. and phosphorous tribromide (358 µL) was dropped on the mixture over 5 min. The suspension was refluxed at 80°C for 18h; then it was cooled to r.t. and aqueous std NaHCO₃ solution was added and the aqueous phase extracted with AcOEt The organic extracts were collected, dried and evaporated *in vacuo* to give a crude which was purified by biotage flash cromathography eluting with CH:AcOEt=98:2 to afford the title compound (200 mg) as a pale brown solid.
NMR (d₆-DMSO): δ (ppm) 8.66 (s, 1H); 8.32 (dd, 1H); 8.28 (d, 1H); 8.01 (dd, 1H): 7.84 (dt, 1H).

### Intermediate 16

### 4-Bromo-6-fluoro-2-naphthalenecarbonitrile

A solution of intermediate **13** (3.2 g), TBTU (4.58 g) and DIPEA (3.19 mL) in anhydrous DMF (50 ml) was stirred at r.t. for 1 h under a Nitrogen atmosphere. 1,1,1,3,3,3-Hexamethyldisilazane (5.02 mL) was added and the mixture stirred at r.t. overnight. The mixture was washed with aqueous 5% NaHCO₃ solution, with an aqueous 2M HCl solution, and then the organic layer was dried, concentrated *in vacuo* to obtain a compound intermediate (3.15 g) which was dissolved in thionyl chloride (45 mL) and refluxed for 2 h under a Nitrogen athmosphere. Then the solvent was removed *in vacuo* to obtain the title compound as a pale brown solid (1.66 g).
NMR (CDCl₃): δ (ppm) 8.18 (s, 1H); 7.94 (d, 1H); 7.93 (s, 1H); 7.91 (d, 1H); 7.43 (td, 1H).

### Intermediate 17

### 4-Ethenyl-7-fluoro-2-naphthalenecarbonitrile

A solution of intermediate **15** (25 mg), TETRAKIS (triphenylphosphine) Palladium (0) (5 mg), tributyl(ethenyl)stannane (32 µL) and one crystal of hydroquinone in dry toluene (1 mL) was heated at 110°C for 4h. The mixture was then cooled to r.t. and aqueous std NaHCO₃ solution and AcOEt were added; the organic phase was separated, washed with aqueous 10% KF solution, dried and evaporated *in vacuo* to give the crude. It was then purified by flash cromatography eluting by CH:AcOEt=9:1, to give the title compound (14 mg) as a yellow solid.
NMR (d₆-DMSO): δ (ppm) 8.51 (s, 1H); 8.40 (dd, 1H); 7.98 (d, 1H); 7.92 (dd, 1H); 7.70 (td, 1H); 7.57 (dd, 3H); 6.07 (d, 1H); 5.65 (d, 1H).

### Intermediate 18

### 4-Ethenyl-6-fluoro-2-naphthalenecarbonitrile

A solution of intermediate **16** (1.66 g), TETRAKIS (triphenylphosphine) Palladium (0) (485 mg), tributyl(ethenyl)stannane (2.34 mL) and one crystal of hydroquinone in dry toluene (50 mL) was heated at 110°C for 4h. The mixture was then cooled to r.t. and aqueous std NaHCO₃ solution and AcOEt were added; the organic phase was separated, washed with aqueous 10% KF solution, dried and evaporated *in vacuo* to give the crude. It was then purified by flash cromatography eluting by CH:AcOEt=95:5 to 9:1, to give the title compound (1.21 g) as a yellow solid.
T.I.c.: CH/AcOEt 95:5, Rf=0.39.
NMR (CDCl₃): δ (ppm) 8.13 (s, 1 H); 7.91 (dd, 1H); 7.72 (dd, 1H); 7.71 (s, 1H); 7.38 (td, 1H); 7.25 (dd, 1H); 5.83 (d, 1H); 5.61 (d, 1H).

### Intermediate 19

### 7-Fluoro-4-formyl-2-naphthalenecarbonitrile

Intermediate **17** (14 mg) was dissolved in THF (1.5 mL) and water (0.3 mL); aqueous 4% osmium tetroxide solution (22 µL) and sodium periodate (30 mg) were added and the solution was vigorously stirred at r.t. and under Nitrogen atmosphere for 4 h. Then a 5% solution of sodium methabisolfite in aqueous std NaHCO₃ solution was added; the organic phase was extracted with AcOEt, dried and evaporated *in vacuo* to give the title compound (14 mg) as a pale yellow solid.
NMR (d₆-DMSO): δ (ppm) 10.38 (s, 1H); 9.23 (dd, 1H); 8.90 (s, 1H); 8.50 (s, 1H); 8.03 (dd, 1H);7.87 (td, 1H).

### Intermediate 20

### 6-Fluoro-4-formyl-2-naphthalenecarbonitrile

Intermediate **18** (100 mg) was dissolved in THF (3 mL) and water (1 mL); aqueous 4% osmium tetroxide solution (310 µL) and sodium periodate (217 mg) were added and the solution was vigorously stirred at r.t. and under Nitrogen atmosphere for 4 h. Then a 5% solution of sodium methabisolfite in aqueous std NaHCO₃ solution was added; the organic phase was extracted with AcOEt, dried and evaporated *in vacuo* to give the title compound (99 mg) as a pale yellow solid.
NMR (CDCl₃): δ (ppm) 10.31 (s, 1H); 9.00 (dd, 1H); 8.44 (s, 1H); 8.14 (s, 1H): 8.01 (dd, 1H);7.50 (td, 1H).

### Intermediate 21

### 4-(Bromomethyl)-7-fluoro-2-naphthalenecarbonitrile

Intermediate **19** (540 mg) was dissolved in MeOH (30 mL) under a Nitrogen athmosphere, the solution was cooled at 0°C and NaBH₄ (102 mg) was added portionwise. After 1 h, aqueous NH4Cl std solution was added and the solution was stirred for ½ h. Then AcOEt was additioned and the organic phase was separated, dried and concentrated *in vacuo* to give a crude which was purified by flash chromatography by CH:AcOEt= 9:1 to afford the a compound intermediate {MS (ES/+): m/z=202 [M+H]⁺} (383 mg) as a white solid.
A portion of this compound (200 mg) was suspended in DCE (10 mL) at r.t. and under a Nitrogen athmosphere; then triphenylphosphine (524 mg) and carbontetrabromide (498 mg) were added and the solution was stirred under these conditions for 2h. Water was added and the aquoeus phase was extracted with DCM. The organic extracts were dried and evaporated in vacuo to give a crude which was purified by flash chromatography eluting with CH:AcOEt= 99:1 to 9:1 to afford the title compound (130 mg) as a white solid.
NMR (CDCl₃): δ (ppm) 8.2 (dd, 1H); 8.15 (s, 1H); 7.63 (s, 1H); 7.56 (dd, 1H); 7.53 (td, 1H); 4.86 (s, 2H).

### Intermediate 22

### 4-(Bromomethyl)-6-fluoro-2-naphthalenecarbonitrile

Intermediate **20** (8300 mg) was dissolved in MeOH (50 mL) under a Nitrogen athmosphere, the solution was cooled at 0°C and NaBH₄ (158 mg) was added portionwise. After 1 h, aqueous NH4Cl std solution was added and the solution was stirred for ½ h. Then AcOEt was additioned and the organic phase was separated, dried and concentrated *in vacuo* to give a crude which was purified by flash chromatography by CH:AcOEt= 9:1 to afford the a compound intermediate {MS (ES/+): m/z=202 [M+H]⁺} (383 mg) as a white solid.
A portion of this compound (180 mg) was suspended in DCE at r.t. and under a Nitrogen athmosphere; then triphenylphosphine (473 mg) and carbontetrabromide (450 mg) were added and the solution was stirred under these conditions for 2h. Water was added and the aquoeus phase was extracted with DCM. The organic extracts were dried and evaporated in vacuo to give a crude which was purified by flash chromatography eluting with CH:AcOEt= 99:1 to 9:1 to afford the title compound (124 mg) as a white solid.
NMR (CDCl₃): δ (ppm) 8.2 (s, 1H); 7.96 (dd, 1H); 7.78 (dd, 1H); 7.7 (s, 1H); 7.43 (td, 1H); 4.83 (s, 2H).

### Intermediate 23

### 4-[(1-Cyano-1-ethoxycarbonyl)-methylene]-piperidine-1-carboxylic acid tert-butyl ester

Ethyl cyanoacetate (13.9 mL), ammonium acetate (4.64 g) and acetic acid (6.9 mL) were added, under a Nitrogen atmosphere, to a solution of 1,1-dimethylethyl 4-oxo-1-piperidinecarboxylate (20 g) in anhydrous toluene (200 mL) in a round bottom flask equipped with a Dean Stark apparatus. The mixture was heated to 110°C for 2 h, then to 85°C overnight and finally to 130°C for 4 h. The mixture was allowed to cool to r.t. and washed with aqueous 1M sodium hydroxide solution, water and brine. The organic layer was dried and concentrated *in vacuo* to a residue, which was purified by flash chromatography (CH/AcOEt 8:2) to give the title compound (15.54 g) as a yellow oil.
T.I.c.: CH/AcOEt 8:2, Rf=0.35 (detection with ninhydrine).
IR (nujol, cm⁻¹): 2229 (C≡N), 1720 and 1694 (C=O).
NMR (CDCl₃): δ (ppm) 4.29 (q, 2H); 3.61 (t, 2H); 3.55 (t, 2H); 3.13 (t, 2H); 2.78 (t, 2H); 1.49 (s, 9H); 1.36 (t, 3H).
MS (ES/+): m/z=295 [M+H]⁺.

### Intermediate 24

### 4-(1-Cyano-1-ethoxycarbonyl-methyl)-4-(4-fluorophenyl)-piperidine-1-carboxylic acid tert-butyl ester

A solution of 4-fluorophenyl magnesium bromide (1.0M in THF, 49 mL) was added dropwise to a mixture of intermediate **23** (8 g) and copper iodide (1.57 g) in anhydrous THF (65 mL) previously cooled to 0°C under a Nitrogen atmosphere. The mixture was stirred under these conditions for 1 h and then allowed to warm to r.t. and stirred at 23°C for 2 h. The mixture was cooled to 0°C, treated with aqueous 3M hydrochloric acid solution until pH=5 and extracted with AcOEt (3 x 100 mL). The combined organic extracts were washed with an aqueous saturated ammonium chloride solution (200 mL), dried and concentrated *in vacuo*. The residue was purified by flash chromatography (CH/AcOEt 85:15) to give the title compound (9.8 g) as a yellow oil.
T.I.c.: CH/AcOEt 6:4, Rf=0.35 (detection with ninhydrine).
NMR (CDCl₃): δ (ppm) 7.31 (dd, 2H); 7.06 (t, 2H); 3.95 (q, 2H); 3.87 (bs, 2H); 3.54 (t, 1H); 2.85 (bt, 2H); 2.53 (dt, 2H); 1.9 (m, 2H); 1.4 (s, 9H); 1.02 (t, 3H).
MS (ES/+): m/z=391 [M+H]⁺.

### Intermediate 25

### 4-Carboxymethyl-(4-fluoro-phenyl)-piperidine-1-carboxylic acid tert-butyl ester

A mixture of intermediate **24** (0.448 g) in acetic acid (2 mL), conc. sulfuric acid (1 mL) and water (1 mL) was heated to 140°C overnight. The solution was allowed to cool to r.t. and dropped into an aqueous 2.5M sodium hydroxide solution (50 mL). Then, di-tert-butyl-dicarbonate (500 mg) was added and the resulting mixture was stirred at r.t. for 5 h. It was cooled to 0°C and treated with aqueous 6N hydrochloric acid solution until pH=3-4 and then extracted with AcOEt (20 mL). The organic phase was dried, concentrated *in vacuo* and the residue was purified by flash chromatography (CH/AcOEt 7:3) to give the title compound (210 mg) as a pale yellow foam.
T.I.c.: CH/AcOEt 1:1, Rf=0.25 (detection with ninhydrine). NMR (d₆-DMSO): δ (ppm) 11.78 (bs, 1H); 7.4 (dd, 2H); 7.15 (t, 2H); 3.45 (m, 2H): 3.11 (m, 2H); 2.54 (s, 2H); 2.04 (m, 2H); 1.89 (m, 2H); 1.37 (s, 9H).
MS (ES/-): m/z=336 [M-H]⁻.

### Intermediate 26

### 1,1-Dimethylethyl 4-(4-fluorophenyl)-4-[2-(methyloxy)-2-oxoethyl]-1-piperidinecarboxylate

To a solution of intermediate **25** (20 g) in dry DCM (250 mL) under a Nitrogen atmosphere at r.t., DIPEA (26 mL) and TBTU (20.9 g) were added. The mixture was stirred at r.t. for 40 minutes, then dry MeOH (10 mL) was added and the brown dark mixture was stirred at r.t. for 14 h.
The reaction mixture was diluted with DCM (200 mL) and washed with aqueous 5% sodium hydrogen carbonate solution (2 x 250 mL) and then with brine (250 mL); the organic phase was dried, concentrated *in vacuo* to give the title compound (28.6 g) as a brown dark oil.
T.Ic.: CH/AcOEt 1:1, Rf=0.72 (detection with ninhydrine).
MS (ES/+): m/z = 374 [M+Na]⁺.

### Intermediate 27

### 1,1-Dimethylethyl 4-(4-fluorophenyl)-4-{1-[(methyloxy)carbonyl]-3-buten-1-yl}-1-piperidinecarboxylate

To a solution of crude intermediate **26** (38.7 g) in dry THF (300 mL) at -60°C and under a Nitrogen atmosphere, lithium bis(trimethylsilyl)-amide (1 M solution in THF -120 mL) was added dropwise. The resulting mixture was warmed up to -15°C and stirred at that temperature for 2 h, then cooled again to -60°C for the addition of allylbromide (11 mL). The reaction mixture was allowed to warm to r.t. and stirred for 3 h. The reaction mixture was quenched at 5°C with water (25 ml), THF was evaporated *in vacuo* and the residue oil was dissolved in Et2O (400 mL) and washed with aqueous saturated NH₄Cl solution (300 mL) and then with brine (2 x 200 mL). The organic phase was dried, concentrated in *vacuo* and the brown dark oil residue (38.5 g) was purified by flash chromatography (Biotage Flash 75L, CH/AcOEt 9:1). The title compound was obtained as a yellow oil (26 g).
T.I.c.: CH/AcOEt 8:2, Rf=0.39 (detection with ninhydrine).
MS (ES/+): m/z = 414 [M+Na]⁺.

### Intermediate 28

### 2-[1-{[(1,1-Dimethylethyl)oxy]carbonyl}-4-(4-fluorophenyl)-4-piperidinyl]-4-pentenoic acid

### Method A

To a solution of intermediate **27** (38.7 g) in isopropanol (200 mL) was added an aqueous lithium hydroxide solution (25 g in 200 mL). The resulting suspension was refluxed for 24 h, further lithium hydroxide (12.5 g in 100 mL of water) was added and the suspension refluxed for further 48 h.
Isopropanol was evaporated in vacuo and the basic aqueous phase (pH = 14) was extracted with Et2O (2 x 300 mL). The aqueous phase was acidified at 0°C with aqueous 5N hydrochloric acid solution (230 mL) until pH= 4.5. The acidic aqueous phase was then extracted with AcOEt (3 x 600 mL); the collected organic phases were dried and concentrated *in vacuo* to give the title compound (29 g) as a white solid.
T.I.c.: CH/AcOEt 8:2, Rf=0.06 (detection with ninhydrine).
MS (ES/+): m/z = 400 [M+Na]⁺
MS (ES/-): m/z = 376 [M-H]⁻.

### Method B

Intermediate **64** (0.76g) was taken up with of dry THF (4 ml) under N₂. The solution was cooled to -20°C and a THF solution of lithium bis(trimethylsilyl)amide (2.41 ml) was added dropwise over 10 min maintaining the temperature between -20°C and -15°C The reaction was stirred for 15 min between -20°C and -15°C and Pd(PPh₃)₄ (0.23g) was added in one portion . The reaction was then allowed to reach room temperature and monitored by HPLC.
The solution is diluted with Ethyl ether 3ml then a solution of potassium carbonate 5ml is added. The organic is collected and concentrated to foam to give the title compound (0.624g).
NMR (DMSO): δ (ppm) 12.1 (broad, 1H), 7.40 (dd, 2H), 7.16 (t, 2H), 5.55 (m, 1H), 4.90 (m, 2H), 3.78 (bm, 2H), 2.8-2.4 (bm, 2H), 2.42 (m, 2H), 2.24 (d, 1H), 2.05 (m, 1H), 1.91 (m, 2H), 1.63 (m, 1H), 1.35 (s, 9H).

### Intermediate 29

### 1,1-Dimethylethyl 4-[1-({[(3,5-dichlorophenyl)methyl]amino}carbonyl)-3-buten-1-yl]-4-(4-fluorophenyl)-1-piperidinecarboxylate

To a solution of intermediate **28** (29 g) in dry DMF (280 mL), DIPEA (33 mL) and TBTU (27.1 g) were added, the solution became dark and after 45 minutes of stirring at r.t., 3,5-dichlorobenzylamine (14.2 g) was added; the reaction mixture turned to orange; it was stirred for 1 h at r.t. under a Nitrogen atmosphere then it was diluted with AcOEt (800 mL) and washed with ice/water 1/1 (4 x 400 mL). The organic phase was dried and concentrated *in vacuo* to give the crude title compound (38.8 g) as a pale orange foam. This material was purified by flash chromatography (Biotage Flash 75L, CH/AcOEt 85:15) to obtain the title compound (38.6 g) as a white foam.
T.I.c.: CH/AcOEt 7:3, Rf=0.45 (detection with ninhydrine).
MS (ES/+): m/z=557 [M+Na]⁺.

### Intermediate 30

### 1,1-Dimethylethyl 4-[1-({[1-(3,5-dichlorophenyl)ethyl]amino}carbonyl)-3-buten-1-yl]-4-(4-fluorophenyl)-1-piperidinecarboxylate (Chain Enantiomer 1)

DIPEA (0.7 mL) and TBTU (0.835 g) were added to a solution of intermediate **28** (0.77 g) in anhydrous DMF (12 mL) under a Nitrogen atmosphere. After stirring for 45 minutes, intermediate 2 (0.44 g) was added. The mixture was stirred at r.t. for 14 h, then it was diluted with AcOEt and washed with cold water. The organic layer was dried, concentrated *in vacuo* and the residue was purified by flash chromatography (CH/AcOEt from 9:1 to 7:3) to give the title compound (0.955 g) as a colourless oil.
T.I.c.: CH/AcOEt 8:2, Rf=0.35 (detection with ninhydrine).
MS (ES/+): m/z=571 [M+Na]⁺.

Following the same procedure described for intermediate 30, intermediates 31 and 32 were obtained.

### Intermediate 31

### 1,1-Dimethylethyl 4-[1-({[1-(3,5-dichlorophenyl)ethyl]amino}carbonyl)-3-buten-1-yl]-4-(4-fluorophenyl)-1-piperidinecarboxylate (Chain Enantiomer 2)

Starting from intermediate **28** (0.77 g) and using intermediate 3, 0.9 g of the title compound were obtained.
T.I.c.: CH/AcOEt 8:2, Rf=0.33 (detection with ninhydrine).
MS (ES/+): m/z=571 [M+Na]⁺.

### Intermediate 32

### 1,1-Dimethylethyl 4-[1-({[(1R)-1-(3-chloro-1-naphthalenyl)ethyl]amino}carbonyl)-3-buten-1-yl]-4-(4-fluorophenyl)-1-piperidinecarboxylate

Starting from intermediate **28** (313 mg) and using intermediate 5, 320 mg of the title compound were obtained.
T.I.c.: CH/AcOEt 7:3, Rf=0.45 (detection with ninhydrine).
MS (ES/+): m/z=587 [M+Na]⁺.

### Intermediate 33

### 1,1-Dimethylethyl 4-[1-({[(1S)-1-(3-chloro-1-naphthalenyl)ethyl]amino}carbonyl)-3-buten-1-yl]-4-(4-fluorophenyl)-1-piperidinecarboxylate

To a solution of intermediate **28** (320 mg) in dry DMF (6 mL), DIPEA (0.296 mL) and TBTU (354 mg) were added, the solution became dark and after 1 h of stirring at r.t., intermediate **6** (0.192 mg) dissolved in DMF (1 mL) was added; the reaction mixture was stirred for 4 h at r.t. under a Nitrogen atmosphere then it was diluted with AcOEt (10 mL) and washed with ice/water 1/1. The organic phase was dried and concentrated *in vacuo* to give the crude title compound that was purified by flash chromatography (elution with CH/AcOEt 95:5 to 8:2) to obtain the title compound (0.451 g) as a white foam.
T.I.c.: CH/AcOEt 7:3, Rf=0.4 (detection with ninhydrine).
MS (ES/+): m/z=587 [M+Na]⁺.

### Intermediate 34

### 1,1-Dimethylethyl 4-[1-({[(3-chloro-1-naphthalenyl)methyl]amino}carbonyl)-3-buten-1-yl]-4-(4-fluorophenyl)-1-piperidinecarboxylate

To a solution of intermediate **28** (400 mg) in dry DMF (4 mL), DIPEA (0.46 mL) and TBTU (374 mg) were added, after 20 minutes of stirring at r.t., 3-chloronaphtylamine (213 mg) was added; the reaction mixture was stirred for 14 h at r.t. under a Nitrogen atmosphere then it was diluted with AcOEt (25 mL) and washed with ice/brine (3 x 30 mL). The organic phase was dried and concentrated *in vacuo* to give the crude title compound that was purified by flash chromatography (elution with CH to CH/AcOEt 8:2) to obtain the title compound (500 mg) as a white solid.
T.I.c.: CH/AcOEt 7:3, Rf=0.34 (detection with ninhydrine).
MS (ES/+): m/z=573 [M+Na]⁺.

### Intermediate 35

### 1,1-Dimethylethyl 4-[1-({[(3-cyano-1-naphthalenyl)methyl]amino}carbonyl)-3-buten-1-yl]-4-(4-fluorophenyl)-1-piperidinecarboxylate

To a solution of intermediate **28** (269 mg) in dry DMF (3 mL), DIPEA (0.30 mL) and TBTU (230 mg) were added, after 20 minutes of stirring at r.t., 3-cyanonaphtylamine (130 mg) was added; the reaction mixture was stirred for 14 h at r.t. under a Nitrogen atmosphere then it was diluted with AcOEt (25 mL) and washed with ice/brine (3 x 30 mL). The organic phase was dried and concentrated *in vacuo* to give the crude title compound that was purified by flash chromatography (elution with CH to CH/AcOEt 1:1) to obtain the title compound (165 mg) as a white solid.
T.I.c.: CH/AcOEt 7:3, Rf=0.27 (detection with ninhydrine).
MS (ES/+): m/z = 564 [M+Na]⁺.

### Intermediate 36

### 1,1-Dimethylethyl 4-(4-fluorophenyl)-4-{1-[(methyloxy)carbonyl]-3-oxopropyl}-1-piperidinecarboxylate

To a suspension of intermediate **27** (2 g) in THF (9 mL) and water (3 mL) osmium tetraoxide 4% wt solution in water (0.3 mL) was added. The mixture was stirred at r.t. for 15 min during which it became dark, then NalO₄ (2.18 g) was added and the brownish reaction mixture was stirred at r.t. for 2 h. After this time it was diluted with water and extracted with AcOEt. The organic layer was washed with water and brine, dried and concentrated *in vacuo.* The residue was purified by flash chromatography (CH/AcOEt from 8:2 to 1:9).

MS (ES/+): m/z=416 [M+Na]⁺.

### Intermediate 37

### 1,1-Dimethylethyl 4-{1-[(3,5-dichlorophenyl)methyl]-2-oxo-3-pyrrolidinyl}-4-(4-fluorophenyl)-1-piperidinecarboxylate

3,5-Dichlorobenzylamine (117 mg) was added to a solution of intermediate **36** (180 mg) in dry dichloroethane (5 mL) under a Nitrogen atmosphere. After stirring for 1 h at r.t. NaBH(OAc)₃ (169 mg) was added and the resulting mixture was stirred at r.t. overnight. Then it was diluted with DCM, washed with an aqueous saturated sodium hydrogen carbonate solution, dried and concentrated *in vacuo* to give a crude oil, which was purified by flash chromatography (Biotage Flash 25M, CH/AcOEt 4:1). The two fractions corresponding to products with Rf=0.36 and Rf=0.50 (CH/AcOEt 1:1, detection with ninhydrine) were collected to give, after evaporation, an oil (40 mg) which was then dissolved, under a Nitrogen atmosphere, in dry MeOH (5 mL) and to which sodium methoxide (4.5 mg) was added. The mixture was refluxed for 5 h, then it was allowed to cool to r.t. and solvent was removed *in vacuo*. The residue was dissolved in AcOEt, washed with an aqueous saturated sodium hydrogen carbonate solution, dried and concentrated *in vacuo*. The crude product thus obtained was purified by flash chromatography (CH/AcOEt 9:1) to give the title compound (35 mg) as a colourless oil.
T.I.c.: CH/ AcOEt 1:1, Rf=0.5 (detection with ninhydrine).
NMR (CDCl₃): δ (ppm) 7.31 (dd, 2H); 7.2 (t, 1H); 6.98 (t, 2H); 6.83 (d, 2H); 4.25 (d, 1H); 4.04 (d, 1H); 3.76 (b, 2H); 3.01 (td, 1H); 2.88 (m, 1H); 2.82 (bm, 1H); 2.63 (dd, 1H); 2.41 (b, 1H); 2.18 (bd, 1H); 2.05-2.15 (m, 2H); 1.94 (b, 1H); 1.81 (b, 1H); 1.69 (m, 1H); 1.4 (s, 9H).
MS (ES/+): m/z=543 [M+Na]⁺.

### Intermediate 38

### 1,1-Dimethylethyl 4-{3-{[(1S)-1-(3-chloro-1-naphthalenyl)ethyl]amino}-1-[(methyloxy)carbonyl]propyl}-4-(4-fluorophenyl)-1-piperidinecarboxylate

To a solution of intermediate **36** (45 mg) in dry CH₃CN (7 mL) intermediate **6** (23.5 mg) was added, the resulting mixture was stirred at r.t. for 15 minutes then NaBH(OAc)₃ (35 mg) was added portionwise (exothermic reaction observed). The reaction mixture was stirred at r.t. for 1 h, then CH₃CN was evaporated *in vacuo* and the residue was dissolved in DCM (100 mL) and washed with water (80 mL) and then with brine (2 x 100 mL). The organic phase was dried, concentrated *in vacuo* and the residue was purified by flash chromatography (CH/AcOEt from 9:1 to 8:2) to give the title compound (745 mg) as a white solid.
HPLC(LC/MS -ES /+): t_{R} = 4.9 min
MS (ES/+): m/z=583 [M+H]⁺

### Intermediate 39

### 1,1-Dimethylethyl 4-{3-{[1-(3,5-dichlorophenyl)ethyl]amino}-1-[(methyloxy)carbonyl]propyl}-4-(4-fluorophenyl)-1-piperidinecarboxylate (Chain Enantiomer 1)

To a solution of intermediate **36** (300 mg) in dry CH₃CN (8 mL) intermediate **2** (144.3 mg) was added, the resulting mixture was stirred at r.t. for 15 minutes then NaBH(OAc)₃ (241.7 mg) was added portionwise (exothermic reaction observed). The reaction mixture was stirred at r.t. for 1 h, then CH₃CN was evaporated *in vacuo* and the residue was dissolved in DCM (100 mL) and washed with water (80 mL) and then with brine (2 x 100 mL). The organic phase was dried, concentrated *in vacuo* and the residue was purified by flash chromatography (CH/AcOEt from 8:2 to 1:1) to give the title compound (351 mg) as a white foam.
T.I.c.: CH/AcOEt 1:1, Rf=0.48 (detection with ninhydrine)
MS (ES/+): m/z=567 [M+H]⁺

### Intermediate 40

### 1,1-Dimethylethyl 4-{3-{[1-(3,5-dichlorophenyl)ethyl]amino}-1-[(methyloxy)carbonyl]propyl}-4-(4-fluorophenyl)-1-piperidinecarboxylate (Chain Enantiomer 2)

To a solution of intermediate **36** (300 mg) in dry CH₃CN (8 mL) intermediate **3** (144.3 mg) was added, the resulting mixture was stirred at r.t. for 15 minutes then NaBH(OAc)₃ (241.7 mg) was added portionwise (exothermic reaction observed). The reaction mixture was stirred at r.t. for 1 h, then CH₃CN was evaporated *in vacuo* and the residue was dissolved in DCM (100 mL) and washed with water (80 mL) and then with brine (2 x 100 mL). The organic phase was dried, concentrated *in vacuo* and the residue was purified by flash chromatography (CH/AcOEt from 8:2 to 1:1) to give the title compound (390 mg) as a white foam.
T.I.c.: CH/AcOEt 1:1, Rf=0.48 (detection with ninhydrine)
MS (ES/+): m/z=567 [M+H]⁺

### Intermediate 41

### 1,1-Dimethylethyl 4-(4-fluorophenyl)-4-[1-[(methyloxy)carbonyl]-3-({(1R)-1-[4-(methyloxy)phenyl]ethyl}amino)propyl]-1-piperidinecarboxylate

To a solution of intermediate **36** (318 mg) in dry CH₃CN (10 mL) (R)-1-(4-Methoxyphenyl)ethyl-amine (122 mg) was added, the resulting mixture was stirred at r.t. for 15 minutes then NaBH(OAC)₃ (257.6 mg) was added portionwise (exothermic reaction observed). The reaction mixture was stirred at r.t. for 1 h, then CH₃CN was evaporated *in vacuo* and the residue was dissolved in DCM (150 mL) and washed with water (100 mL) and then with brine (2 x 100 mL). The organic phase was dried, concentrated *in vacuo* to give the title compound (461 mg) as a foam.
MS (ES/+): m/z=529 [M+H]⁺.

### Intermediate 42 and intermediate 43

### 1,1-Dimethylethyl 4-{1-[(1S)-1-(3-chloro-1-naphthalenyl)ethyl]-2-oxo-3-pyrrolidinyl}-4-(4-fluorophenyl)-1-piperidinecarboxylate (Diastereoisomer 1)1,1-Dimethylethyl 4-{1-[(1S)-1-(3-chloro-1-naphthalenyl)ethyl]-2-oxo-3-pyrrolidinyl}-4-(4-fluorophenyl)-1-piperidinecarboxylate (Diastereoisomer 2)

A mixture of intermediate **38** (48 mg) and NaOMe (6.68 mg) in dry MeOH (5 mL) was processed by microwave irradiation at 150°C (2 cycles of 12 min, one cycle of 15 min and 2 cycles of 30 min). Then MeOH was evaporated *in vacuo* and the residue was dissolved in AcOEt (100 mL) and washed with water (80 mL). The organic phase was dried and concentrated *in vacuo* to give an oil. This material was dissolved in dry DCM (6 mL) and then Di-tert-butyl dicarbonate (18.6 mg) was added to this mixture. After stirring for 1 h at r.t., DCM was evaporated *in vacuo.* The crude was purified by flash chromatography (CH/AcOEt from 9:1 to 8:2) to give intermediate 42 (21 mg) and intermediate 43 (19 mg).

### Intermediate 42

T.I.c.: CH/AcOEt 7:3, Rf=0.29 (detection with ninhydrine).
HPLC (LC/MS -ES /+):t_{R} = 6.9 min
MS (ES/+): m/z=495 [M-tBut]⁺

### Intermediate 43

T.I.c.: CH/AcOEt 7:3, Rf=0.21 (detection with ninhydrine).
HPLC (LC/MS -ES /+):t_{R} = 6.8 min
MS (ES/+): m/z=495 [M-tBut]⁺

### Intermediate 44 and intermediate 45

### 1,1-Dimethylethyl 4-{1-[1-(3,5-dichlorophenyl)ethyl]-2-oxo-3-pyrrolidinyl}-4-(4-fluorophenyl)-1-piperidinecarboxylate (Diastereoisomer 1 Chain Enantiomer 1) 1,1-Dimethylethyl 4-{1-[1-(3,5-dichlorophenyl)ethyl]-2-oxo-3-pyrrolidinyl}-4-(4-fluorophenyl)-1-piperidinecarboxylate Diastereoisomer 2 Chain Enantiomer 1)

A mixture of intermediate **39** (351 mg) and NaOMe (67 mg) in dry MeOH (4 mL) was processed by microwave irradiation at 150°C (3 cycles of 30 min and one cycle of 20 min). Then MeOH was evaporated *in vacuo* and the residue was dissolved in AcOEt (100 mL) and washed with water (80 mL). The organic phase was dried and concentrated in *vacuo* to give an oil. This material was dissolved in dry DCM (6 mL) and then Di-tert-butyl dicarbonate (135.2 mg) and TEA (0.13 mL) were added to this mixture. After stirring for 1 h at r.t., DCM was evaporated *in vacuo*. The crude was purified by flash chromatography (CH/AcOEt from 9:1 to 1:1) to give **intermediate 44** (125 mg) {first spot to be eluted having MS (ES/+): m/z=535 [M+H]⁺} and **intermediate 45** (165 mg) {second spot to be eluted having MS (ES/+): m/z=535 [M+H]⁺}.

### Intermediate 46 and intermediate 47

### 1,1-Dimethylethyl 4-{1-[1-(3,5-dichlorophenyl)ethyl]-2-oxo-3-pyrrolidinyl}-4-(4-fluorophenyl)-1-piperidinecarboxylate (Diastereoisomer 1 Chain Enantiomer 2) 1,1-Dimethylethyl 4-{1-[1-(3,5-dichlorophenyl)ethyl]-2-oxo-3-pyrrolidinyl}-4-(4-fluorophenyl)-1-piperidinecarboxylate (Diastereoisomer 2 Chain Enantiomer 2)

A mixture of intermediate **40** (390 mg) and NaOMe (74.4 mg) in dry MeOH (4 mL) was processed by microwave irradiation at 150°C (3 cycles of 30 min and one cycle of 20 min). Then MeOH was evaporated *in vacuo* and the residue was dissolved in AcOEt (100 mL) and washed with water (80 mL). The organic phase was dried and concentrated in *vacuo* to give an oil. This material was dissolved in dry DCM (6 mL) and then Di-tert-butyl dicarbonate (150.4 mg) and TEA (0.14 mL) were added to this mixture. After stirring for 1 h at r.t., DCM was evaporated *in vacuo*. The crude was purified by flash chromatography (CH/AcOEt from 9:1 to 1:1) to give **intermediate 46** (125 mg) {first spot to be eluted having MS (ES/+): m/z=535 [M+H]⁺} and **intermediate 47** (165 mg) {second spot to be eluted having MS (ES/+): m/z=535 [M+H]⁺}.

### Intermediate 48 and intermediate 49

### 1,1-Dimethylethyl 4-(4-fluorophenyl)-4-(1-{(1R)-1-[4-(methyloxy)phenyl]ethyl}-2-oxo-3-pyrrolidinyl)-1-piperldinecarboxylate (Diastereoisomer 1)1,1-Dimethylethyl 4-(4-fluorophenyl)-4-(1-{(1R)-1-[4-(methyloxy)phenyl]ethyl}-2-oxo-3-pyrrolidinyl)-1-piperidinecarboxylateDiastereoisomer 2)

To a solution of intermediate **41** (740 mg) in dry Toluene (10.mL) Pyridine (1.13 mL) was added, the mixture was heated at 130°C and it was stirred at this temperature over weekend. Then toluene was evaporated *in vacuo* and the residue was dissolved in AcOEt (150 mL) and washed with water (100 mL). The organic phase was dried, concentrated in *vacuo* to give an oil. The crude was purified by flash chromatography (CH/AcOEt from 9:1 to 8:2) to give intermediate 48 (201.5 mg) and intermediate 49 (218.5 mg).

### Intermediate 48

HPLC(LC/MS -ES /+):t_{R} = 6.3 min
MS (ES/+): m/z=442 [M-tBut]⁺

### Intermediate 49

HPLC(LC/MS -ES /+):t_{R} = 6.6 min
MS (ES/+): m/z=442 [M-tBut]⁺

### Intermediate 50

### 1,1-Dimethylethyl 4-{1-[(3,5-dichlorophenyl)methyl]-5-hydroxy-2-oxo-3-pyrrolidinyl}-4-(4-fluorophenyl)-1-piperidinecarboxylate

To a suspension of intermediate **29** (0.72 g) in THF (37.5 mL) and water (12.5 mL) osmium tetraoxide 4% wt solution in water (0.8 mL) was added. The mixture was stirred for 20 minutes during which it became dark, then NalO₄ (1.15 g) was added portionwise in 10 minutes and the brownish reaction mixture was stirred at r.t. for 12 h, thus becoming a milky suspension. The reaction mixture was diluted with AcOEt (250 mL) and washed with water (4 x 50 mL) and then with brine (40 mL). The organic phase was dried and concentrated *in vacuo* to give a brown-grey foam (0.635 g). This material was purified by flash chromatography (Biotage Flash 40S, CH/AcOEt 6:4 to 1:1) affording the title compound (0.335 g) as a white foam.
T.I.c.: CH/AcOEt 1:1, Rf=0.33 (detection with ninhydrine)
NMR (CDCl₃): δ (ppm) 7.32 (dd, 2H); 7.21 (s, 1H); 6.98 (t, 2H); 6.78 (s, 2H); 4.61 (d, 1H); 4.45 (bt, 1H); 3.91 (d, 1H); 3.85 - 3.68 (bm, 2H); 3.05 (t, 1H); 2.95 - 2.78 (bm, 2H); 2.25 - 2.15 (m, 3H); 2.11 - 1.60 (m, 3H); 1.39 (s, 9H).
MS (ES/+): m/z=559 [M+Na]⁺ .

### Intermediate 51

### 1,1-Dimethylethyl 4-[1-({[3,5-dichlorophenyl)methyl]amino}carbonyl)-3-oxopropyl]-4-(4-fluorophenyl)-1-piperidinecarboxylate (Chain Enantiomer 1)

To a suspension of intermediate **30** (0.07 g) in THF (2 mL) and water (0.5 mL) osmium tetraoxide 4% wt solution in water (3.2 mL) was added. The mixture was stirred r.t. for 1 h during which it became dark, then NalO₄ (0.082 g) was added portionwise and the brownish reaction mixture was stirred at r.t. for 1 h. After this time it was diluted with water and extracted with AcOEt. The organic layer was dried and concentrated *in vacuo*. The residue was purified by flash chromatography (CH/AcOEt from 9:1 to 7:3) to afford the title compound as a yellow oil (0.047 g). T.I.c.: CH/AcOEt 6:4, Rf=0.37 (detection with ninhydrine).
NMR (CDCl₃): δ (ppm) 9.70 (s, 1H); 7.24 (s, 2H); 7.13 (dd, 2H); 6.96 (s, 1H); 6.93 (t, 2H); 5.40 (bq, 1H); 3.96 - 3.82 (m, 2H); 3.02 (dd, 1H); 2.71 - 2.35 (m, 4H); 2.11 - 1.85 (m, 2H); 1.74 -1.57 (m, 2H); 1.39 (s, 9H); 1.33 (d, 3H).
MS (ES/+): m/z=573 [M+Na]⁺.

### Intermediate 52

### 1,1-Dimethylethyl 4-[1-({[(1S)-1-(3-chloro-1-naphthalenyl)ethyl]amino}carbonyl)-3-oxopropyl]-4-(4-fluorophenyl)-1-piperidinecarboxylate

To a suspension of intermediate **33** (0.45 g) in THF (4 mL) and water (1 mL) osmium tetraoxide 4% wt solution in water (0.05 mL) was added. The mixture was stirred r.t. for 40 minutes during which it became dark, then NalO₄ (0.35 g) was added portionwise and the brownish reaction mixture was stirred at r.t. for 1.15 h. After this time it was diluted with AcOEt and extracted with water. The organic layer was dried and concentrated *in vacuo.*
The residue was purified by flash chromatography (CH/AcOEt from 9:1 to 6:4) to afford the title compound as a yellow oil (0.128 g).
MS (ES/+): m/z=589 [M+Na]⁺.

### Intermediate 53

### 1,1-Dimethylethyl 4-[1-({[(3-chloro-1-naphthalenyl)methyl]amino}carbonyl)-3-oxopropyl]-4-(4-fluorophenyl)-1-piperidinecarboxylate

To a suspension of intermediate **34** (500 mg) in THF (3.75 mL) and water (1.25 mL) osmium tetraoxide 4% wt solution in water (0.057 mL) was added. The mixture was stirred at r.t. for 15 minutes during which it became dark, then NalO₄ (388 mg) was added portionwise and the brownish reaction mixture was diluted with water (2.5 mL) and THF (2.5 mL) and stirred at r.t. for 1.5 h. After this time it was diluted with AcOEt (30 mL) and washed with water (30 mL), then with brine (30 mL). The organic layer was dried and concentrated *in vacuo.* The residue was purified by flash chromatography (elution from CH to CH/AcOEt 1:1) to afford the title compound as a white solid (210 mg).
T.I.c.: CH/AcOEt 6:4, Rf= 0.16 (detection with ninhydrine).
MS (ES/+): m/z=575 [M+Na]⁺.

### Intermediate 54

### 1,1-Dimethylethyl 4-[1-({[(3-cyano-1-naphthalenyl)methyl]amino}carbonyl)-3-oxopropyl]-4-(4-fluorophenyl)-1-piperidinecarboxylate

To a suspension of intermediate **35** (165 mg) in THF (3 mL) and water (1 mL), osmium tetraoxide 4% wt solution in water (0.020 mL) was added. The mixture was stirred at r.t. for 15 minutes during which it became dark, then NalO₄ (130 mg) was added portionwise and the brownish reaction mixture was stirred at r.t. for 1.5 h. After this time it was diluted with AcOEt (30 mL) and washed with water (30 mL), then with brine (30 mL). The organic layer was dried and concentrated *in vacuo*. The residue was purified by flash chromatography (elution from CH/AcOEt 7:3 to CH/AcOEt 1:1) to afford the title compound as a white solid (120 mg).
T.I.c.: CH/AcOEt 6:4, Rf= 0.08 (detection with ninhydrine).
MS (ES/+): m/z = 566 [M+Na]⁺.

### Intermediate 55

### 1,1-Dimethylethyl 4-(4-fluoro-phenyl)-4-(2-oxo-3-pyrrolidinyl)-1-piperidinecarboxylate (Enantiomer 1)

A suspension of intermediate **48** (30 mg), Ammonium cerium(IV) nitrate (99.4 mg), Silica gel (142 mg) and a 3:1 mixture of CH₃CN/water (1.2 mL) was stirred for 10 min under Nitrogen atmosphere at r.t. and then quenched with an aqueous saturated sodium sulfite solution (1 mL) and an aqueous saturated sodium carbonate solution (1 mL). The liquid was decanted from the insoluble ceriun carbonate salts, and the aqueous phase washed with DCM (3 x 5 mL). The combined organic extracts were dried, concentrated *in vacuo*, and purified by flash chromatography (CH/AcOEt from 8:2 to 0:10) to give the title compound (21 mg) as a white foam.
HPLC (LC/MS -ES /+):t_{R} = 5.7 min
MS (ES/+): m/z=307 [M-tBut]⁺

### Intermediate 56

### 1,1-Dimethylethyl 4-(4-fluorophenyl)-4-(2-oxo-3-pyrrolidinyl)-1-piperidinecarboxylate (Enantiomer 2)

A suspension of intermediate **49** (192 mg), Ammonium cerium(IV) nitrate (641.2 mg), Silica gel (923 mg) and a 3:1 mixture of CH₃CN/water (8 mL) was stirred for 10 min under Nitrogen atmosphere at r.t. and then quenched with an aqueous saturated sodium sulfite solution (2 mL) and an aqueous saturated sodium carbonate solution (2 mL). The liquid was decanted from the insoluble cerium carbonate salts, and the aqueous phase washed with DCM (3 x 10 mL). The combined organic extracts were dried, concentrated *in vacuo*, and purified by flash chromatography (CH/AcOEt from 8:2 to 1:1) to give the title compound (105 mg) as a white foam.
HPLC(LC/MS -ES /+):t_{R} = 5.5 min
MS (ES/+): m/z=307 [M-tBut]⁺

### Intermediate 57

### 1,1-Dimethylethyl 4-{1-[(3-cyano-1-naphthalenyl)methyl]-2-oxo-3-pyrrolldinyl}-4-(4-fluorophenyl)-1-piperidinecarboxylate (Enantiomer 1)

NaH (12.6 mg) was added to a solution of intermediate 55 (57 mg) in dry DMF (3.0 mL) at 0°C under Nitrogen atmosphere. The resulting mixture was stirred at 0°C for 30 minutes; then intermediate **9** (43 mg) was added. The reaction mixture was stirred at 0°C for 35 min and then quenched water and brine; the aqueous phase was washed with AcOEt (3 x 30 mL). The combined organic extracts were dried, concentrated *in vacuo*, and purified by flash chromatography (CH/AcOEt from 8:2 to 6:4) to give the title compound (41 mg) as a white foam.
T.I.c.: CH/AcOEt 1:1, Rf=0.39 (detection with ninhydrine)
HPLC(LC/MS -ES /+):t_{R} = 6.6 min
MS (ES/+): m/z=428 [M-BOC]⁺

### Intermediate 58

### 1,1-Dimethylethyl 4-{1-[(3-cyano-1-naphthalenyl)methyl]-2-oxo-3-pyrrolidinyl}-4-(4-fluorophenyl)-1-piperidinecarboxylate (Enantiomer 2)

NaH (11.5 mg) was added to a solution of intermediate 56 (52 mg) in dry DMF (3.0 mL) at 0°C under Nitrogen atmosphere. The resulting mixture was stirred at 0°C for 30 minutes; then intermediate **9** (39 mg) was added. The reaction mixture was stirred at 0°C for 35 min and then quenched water and brine; the aqueous phase was washed with AcOEt (3 x 30 mL). The combined organic extracts were dried, concentrated *in vacuo*, and purified by flash chromatography (CH/AcOEt from 8:2 to 6:4) to give the title compound (41 mg) as a white foam.
T.I.c.: CH/AcOEt 1:1, Rf=0.39 (detection with ninhydrine)
MS (ES/+): m/z=528 [M+H]⁺.

### Intermediate 59

### 1,1-Dimethylethyl 4-{1-[(3-cyano-6-fluoro-1-naphthalenyl)methyl]-2-oxo-3-pyrrolidinyl}-4-(4-fluorophenyl)-1-piperidinecarboxylate (Enantiomer 2)

NaH (11 mg) was added to a solution of intermediate **22** (49 mg) in dry DMF (3.0 mL) at 0°C under Nitrogen atmosphere. The resulting mixture was stirred at 0°C for 30 minutes; then intermediate **21** (39 mg) was added. The reaction mixture was heated at 40°C for ½ h and then quenched with ice; the aqueous phase was washed with AcOEt (3 x 30 mL), the collected organic extracts were washed with brine and dried, concentrated *in vacuo*, and purified by flash chromatography (CH/AcOEt from 9:1 to 8:2) to give the title compound (28 mg) as a white foam.
HPLC(LC/MS -ES /+):t_{R} = 6.41 min
MS (ES/+): m/z=468 [M+Na]⁺

### Intermediate 60

### 1,1-Dimethylethyl 4-{1-[(3-cyano-7-fluoro-1-naphthalenyl)methyl]-2-oxo-3-pyrrolidinyl}-4-(4-fluorophenyl)-1-piperidinecarboxylate (Enantiomer 2)

NaH (11 mg) was added to a solution of intermediate **56** (49 mg) in dry DMF (3.0 mL) at 0°C under Nitrogen atmosphere. The resulting mixture was stirred at 0°C for 30 minutes; then intermediate **22** (39 mg) was added. The reaction mixture was heated at 40°C for ½ h and then quenched with ice; the aqueous phase was washed with AcOEt (3 x 30 mL), the collected organic extracts were washed with brine and dried, concentrated *in vacuo*, and purified by flash chromatography (CH/AcOEt from 9:1 to 8:2) to give the title compound (19 mg) as a white foam.
HPLC(LC/MS -ES /+):t_{R} = 6.31 min
MS (ES/+): m/z= 568 [M+Na]⁺

### Intermediate 61

### 1,1-Dimethylethyl 4-{1-[(3-cyano-6-fluoro-1-naphthalenyl)methyl]-2-oxo-3-pyrrolidinyl}-4-(4-fluorophenyl)-1-piperidinecarboxylate (Enantiomer 1)

NaH (9 mg) was added to a solution of intermediate **55** (41 mg) in dry DMF (5.0 mL) at 0°C under Nitrogen atmosphere. The resulting mixture was stirred at 0°C for 30 minutes; then intermediate **21** (30 mg) was added. The reaction mixture was heated at 40°C for 2 h and then quenched with ice; the aqueous phase was washed with AcOEt (3 x 30 mL), the collected organic extracts were washed with brine and dried, concentrated *in vacuo*, and purified by flash chromatography (CH/AcOEt from 9:1 to 7:3) to give the title compound (52 mg) as a white foam.
HPLC(LC/MS -ES /+):t_{R} = 6.43 min
MS (ES/+): m/z=568 [M+Na]⁺

### Intermediate 62

### 1,1-Dimethylethyl 4-{1-[(3-cyano-7-fluoro-1-naphthalenyl)methyl]-2-oxo-3-pyrrolidinyl}-4-(4-fluorophenyl)-1-piperidinecarboxylate (Enantiomer 1)

NaH (9 mg) was added to a solution of intermediate **55** (41 mg) in dry DMF (5.0 mL) at 0°C under Nitrogen atmosphere. The resulting mixture was stirred at 0°C for 30 minutes; then intermediate **22** (30 mg) was added. The reaction mixture was heated at 40°C for 2 h and then quenched with ice; the aqueous phase was washed with AcOEt (3 x 30 mL), the collected organic extracts were washed with brine and dried, concentrated *in vacuo*, and purified by flash chromatography (CH/AcOEt from 9:1 to 7:3) to give the title compound (21 mg) as a white foam.
HPLC(LC/MS -ES /+):t_{R} = 6.41 min
MS (ES/+): m/z= 568 [M+Na]⁺

### Intermediate 63

### 1'-[(3,5-Dichlorophenyl)methyl]-4-(4-fluorophenyl)-2'H,5'H-spiro[1-azabicyclo[2.2.1]heptane-7,3'-pyrrolidine]-2',5'-dione

NalO4 (29g) was dissolved in water (200 mL). The mixture was left stirring overnight and the next morning was filtered. Intermediate **29** (29g) was charged in the reactor, then, THF (150mL) was added, and K₂OsO₄ (261mg) in water (43mL). The mixture was heated at 40 °C (internal temperature), and the solution was stirred for 30 min. Then the solution of NalO₄ in water was added dropwise during 5h.
The reaction was cooled at r.t. Then aqueous std Na2SO3 (100mL) was added and the solution was stirred under these conditions for 1h. Then AcOEt (300 mL) was added and the phases separated. The organic phase was concentrated up to a volume of 130 mL then 14ml of HCl 5M solution in AcOEt was added. The mixture was heated at reflux for 2hr. Then it was cooled down at r.t. and aqueous 2.5M NaOH (348 mL) was added. The phases were separated and the organic one was concentrated *in vacuo* to a crude oil. The oil was then diluted with AcOEt (160mL) and acetonitrile was added at 0deg. A solid precipitated to give 6g of the title compound.
NMR (DMSO): δ (ppm) 7.53 (bs, 1H), 7.26 (dd, 2H), 7.05 (d, 2H), 6.93 (t, 2H), 4.47 (d+d, 2H), 3.66 (m, 1H), 3.02 (m, 1H), 2.98 (d, 1H), 2.68 (d, 1H), 2.65 (m, 2H), 2.58 (m, 1H), 2.36 (m, 1H), 1.69 (m, 1H), 1.59 (m, 1H)

### Intermediate 64

### 1,1-dimethylethyl4-(4-fluorophenyl)-4-[2-oxo-2-(2-propen-1-yloxy)ethyl]-1-piperidinecarboxylate

To a solution of 1-piperidinecarboxylic acid, 4-(2,2-dimethyl-4,6 dioxo-1,3-dioxan-5-yl)-4-(4-fluorophenyl)-1,1-dimethyl ester (0.288 g) in DMF (5 ml) Allyl alcohol (0.15ml) is added at rt. The reaction mixture is heated at 100-105C for 16h. The reaction is cooled at rt and concentrated to give the title compound as oil (0.171g).
NMR (DMSO): δ (ppm) -7.39 (dd, 2H), 7.12 (t, 2H), 5.65 (m, 1H), 5.06 (m, 2H), 4.28 (m, 2H), 3.49 (bm, 2H), 3.12 (bm, 2H), 2.67 (s, 2H), 2.07 (m, 2H), 1.88 (m, 2H), 1.38 (s, 9H).

### Intermediate 65

### Ethyl N-[(3,5-dichlorophenyl)methyl]glycinate, hydrochloride salt

To a solution of glycine ethyl ester hydrochloride (35.7 g) in acetonitrile (90 ml), in a 3-necked flask equipped with air-driven mechanical stirrer, thermometer, and addition funnel, 3,5-dichlorobenzyl chloride (5.0 g) and triethylamine (35.7 ml) were added at 20-25 °C, maintaining good stirring. The reaction was then heated to reflux (82 ± 3 °C) under stirring for 30-60 min. When reaction was complete, after cooling to 20-25 °C, the reaction mixture was filtered and the cake was rinsed with toluene. The filtrate and the toluene rinses were combined and concentrated to 90ml. This solution was washed with water and then cooled to about 5 ± 3 °C in an ice-water bath. Conc. HCl (2.3 ml) was then added dropwise over about 5 minutes at 5-10 °C. The mixture was stirred at 5-10 °C for about 30 - 90 minutes. The white precipitate was filtered off, rinsed with toluene and tert-butyl methyl ether, and dried at 40-50 °C under vacuum to obtain the title compound (6.62 g).
¹H NMR (DMSO-d₆): δ (ppm) 10.17 (broad s, NH/NH2⁺); 7.71 (fine doublet, 2 H); 7.68 (fine d, 1H); 4.16-4.20 (m, 4 H); 3.94 (br s, 2 H); 1.23 (t, 3H).

### Intermediate 66

### 1,1-dimethylethyl 4-{2-{[(3,5-dichlorophenyl)methyl][2-(ethyloxy)-2-oxoethyl]amino}-1-[(methyloxy)carbonyl]-2-oxoethyl}-4-(4-fluorophenyl)-1-piperidinecarboxylate

Piperidinecarboxylic acid, 4-(2,2-dimethyl-4,6 dioxo-1,3-dioxan-5-yl)-4-(4-fluorophenyl)-1,1-dimethyl ester (26.8 g)) and intermediate 65 (18.4 g) were added to dimethylformamide (148 ml) in necked flask, equipped with air-driven mechanical stirrer, thermometer, and condenser . The mixture was stirred at 20 - 25 °C for about 15 minutes to obtain a well-dispersed slurry and then heated to 55 ± 3 °C over 15 - 30 minutes and stirred at 55 ± 3 °C for about 2 - 3 hours. The reaction was cooled to 20 ± 5 °C.and then K₂CO₃ (5.1g.) and Me₂SO₄ (11.7g.) were added under stirring at 20 ± 5 °C. When reaction was complete, the reaction mixture was filtered through celite and the celite was washed with tert-butyl methyl ether (TBME, 276ml). The TBME was washed with 10% aqueous KHSO₄ (180ml) and then with water (180mL) and the TBME layer (containing 38.37gr of the title compound) was collected and concentrated to a minimum volume.

Mass spec. (m + Na⁺) = 661.2

### Intermediate 67

### 1-[(3,5-dichlorophenyl)methyl]-3-[4-(4-fluorophenyl)-4-piperidinyl]-2,4-pyrrolidinedione, hydrochloride salt

A solution of intermediate 66 (85.46g) in *tert*-butyl methyl ether (TBME, 310 ml) was concentrated to a minimum stirring volume under reduced pressure at <30 °C and then ethanol (510 ml) was added. After further concentration to ca 475mL, the mixture was cooled (0 °C) and a solution of sodium ethoxide in ethanol (21wt% in EtOH, 23ml) over 10-20 min was added under nitrogen, while maintaining the temperature at 0-5 °C. The mixture was stirred for an additional 10-20 min at 0-5 °C. When the cyclization was complete, the mixture was acidified with 1*N* HCl (160 ml) to a pH of 1-3 while maintaining the tem perature below 25 °C. The mixture was then concentrated to ca. 250mL at ca. 25-43°C under reduced pressure. After adding water (350mL) the mixture was re-concentrated to ca. 350mL under reduced pressure. 350 ml of concentrated HCI was added maintaining the temperature below 35 °C. The mixture was heated to 58-63 °C over ca. 30 min and stirred for an additional 30-45 min, and then heated to reflux over 30-45 min and stirred for 1-1.5 h at 100-106 °C. When the deethoxycarbonylation is complete, the mixture was cooled to ca. 60 °C over ca. 30 min. and THF (80ml) was added at 57-60 °C until the mixture becomes a clear solution. After cooling to 51-53 °C over ca.15 min and seeds of the title compound was added. The mixture was stirred at 51-53 °C for ca. 50 min and then cooled to 20 °C over and stirred at 20 °C overnight. The resulting slurry was filtered and the filter cake was washed with water (85 ml). After drying the wet cake under vacuum for 4-6 h at ca. 50 °C the title compound (54 g) was obtained as an off-white solid.
¹H NMR (DMSO-d₆): δ (ppm) 11.62 (s, 1 H, O-H enol form); 8.86 (s, 2 H, NH₂); 7.47 (t, 1 H), 7.34. (m, 2 H); 7.15 (d, 2 H), 7.11 (m, 2 H); 4.42 (s, 2 H); 3.84 (s, 2 H); 3.25 (m, 2 H); 3.11 (m, 2 H); 2.90 (m, 2 H); 1.91 (m, 2 H).
MS (ES/+): m/z = 437, 435 [M + 1]⁺.

### Intermediate 68

### 1-[(3,5-dichlorophenyl)methyl]-3-[4-(4-fluorophenyl)-4-piperidinyl]-4-hydroxy-2-pyrrolidinone

Sodium borohydride (794 mg,) was added to a stirred solution of intermediate 67 (3.30 g) in 50 mL of 2-propanol at room temperature. After 5 min at room temperature, the mixture was cooled to 5 °C and then water (10 mL) was slowly added over 10 min period while keeping the temperature below 20 °C. The mixture was stirred for 15 h at room temperature and then sodium borohydride (265 mg) was added. The mixture was stirred for 4.5 h at room temperature. Sodium borohydride (133 mg) and water (10 mL) were added and mixture was stirred for 3 h at room temperature. The mixture was acidified with 6N HCl to pH = 1 and stirred for 30 min. Ethyl acetate (50 mL) was added and the mixture was basified with NaOH (25 w %) to pH = 13. The organic layer was separated and then washed with 17 mL of brine. The organic layer was concentrated under reduced pressure to leave an oily solid. Ethyl acetate (50 mL) was added and the mixture was re-concentrated under reduced pressure to afford 3.24 g of title compound as an off-white solid.
¹H NMR (DMSO-d₆): δ (ppm) 7.48 (m, 3 H); 7.25 (d, 2 H); 7.07 (t, 2 H); 4.86 (br s, 1H, O-H); 4.42 (d, 1 H); 4.27 (d, 2 H); 3.70 (br s, 1 H); 3.18 (dd, 1 H); 2.70 (m, 4 H); 2.53 (m, 2 H); 2.28 (t, 1H); 2.18 (br t, 1 H); 2.04 (d, 1 H).
MS (ES/+): m/z = 439, 437 [M + 1]⁺.

### Intermediate 69

### 1-[(3,5-dichlorophenyl)methyl]-3-[4-(4-fluorophenyl)-1-methyl-4-piperidinyl]-4-hydroxy-2-pyrrolidinone

Intermediate 68 (2.01g,) was placed into a flask equipped with a stir bar, temperature probe, and condenser. Then water (10ml) and a 37% formaldehyde solution. (0.74ml) were added and stirred at room temperature for about 5 minutes. 88% formic acid (12 ml) was added slowly to the reaction which was heated to about 100°C for 4-12 hours. Once the reaction was completed, it was cooled to room temperature and 1 M NaOH until the pH is -9. was added slowly followed by ethyl acetate (20ml). The organic layers were separed and after evaporation of the solvent the title compound was obtained 1.1g as a tan colored solid.
Analytical: mass spec. (m + H) = 451.2
NMR: H¹ DMSO-d₆ 400MHz: 7.48 (m, 3H), 7.25 (m, 2H), 7.08 (t, 2H), 4.87 (d, 1H), 4.42 (d, 1 H), 4.29 (d, 1H), 3.68 (s, 1H), 3.19 (dd, 1 H), 2.92 (t, 1 H), 2.73 (d, 1 H), 2.61 (m, 1H), 2.58 (m, 1H), 2.53 (m, 1H), 2.47 (m, 1 H), 2.33 (t, 1 H), 2.10 (d, 1H), 1.98 (s, 3H), 1.92 (t, 1 H), 1.69 (t, 1H).

### Example 1

### 1-[(3,5-Dichlorophenyl)methyl]-3-[4-(4-fluorophenyl)-4-piperidinyl]-1,5-dihydro-2H-pyrrol-2-one

### Method A

To intermediate **50** (0.22 g), TFA (15 mL) was added and the solution was heated at 60°C under a Nitrogen atmosphere for 3 h. The reaction mixture was evaporated *in vacuo*, the residue dissolved in DCM (30 mL) and slowly added to an aqueous 2.5M sodium hydroxide solution, previously cooled to 0°C. The organic phase was separated and the basic aqueous solution was extracted with DCM (40 mL); the collected organic phases were dried and concentrated *in vacuo* to give the crude compound (0.17 g) as a yellow oil, which was purified by flash chromatography (DCM, then DCM/MeOH from 95:5 to 7:3) to give the title compound (104 mg) as a white solid.
T.I.c.: DCM/MeOH 9:1, Rf=0.15 (detection with ninhydrine).
NMR (CDCl₃): δ (ppm) 7.34 (dd, 2H); 7.23 (t, 1 H); 6.99 (t, 2H); 6.97 (m, 2H); 6.68 (t, 1H); 4.46 (s, 2H); 3.75 (dd, 2H); 2.89 (t, 4H); 2.53 (m, 2H); 2.23 (m, 2H).
MS (ES/+): m/z=419 [M+H]⁺.

### Method B

A mixture of NalO4 (15 Kg) in 108L of water was stirred overnight and then was filtered.

Intermediate 29 (15Kg) is charged in the reactor, then, THF (75L.), water (22.5L) and K₂OsO₄ (0.001 eq, 10.35g) were added. The mixture was heated at 40 °C , and stirred for 30 min and then the solution of NalO₄ (15Kg) in water (108 L) was added dropwise during 5hrs. The reaction was cooled down at r.t.and then 60L of H₂O are added follow by the addition of 90L of CH₂Cl₂. The mixture was stirred overnight.
The phases are separated. Then 0.255Kg of functionalized silica gel (Thiol-3) were added and the mixture was stirred for 2hr under reflux. Once the mixture is cooled down to r.t. Then the solution is filtrated slowly through a CUNO filter (R55S) and the filter washed with 45L of CH2Cl2.
All the organics were collected and transfer to a clean reactor and concentrated until 67.5L. Water was then added (7.5L) and the reaction was vigorously stirred under N₂ and TFA (30L) was added in one portion. The reaction is stirred at 20°C for 30 min. Further TFA (30L) was added and the solution was heated to 55°C (internal temperature) and monitored via HPLC. After 5h the reaction mixture was cooled to 10°C, DCM (150L) and methanol (75L) were successively added and the reaction mixture was cooled to 10°C. A solution of 5M aq. NaOH was added, maintaining the temperature below 20°C, until pH 12-13. The phases were separated and the aqueous layer was extracted with DCM (45L). The combined organic layers were washed with water (15L), the collected organic layer was concentrated under vacuum (400 mmHg, 27°C) to 60L and iPrOAc (120L) was added. The solution is concentrated to 60L under vacuum at 55°C and then cooled down to 10°C. Seeds are added and 2.25L of water is added dropwise. The mixture is agitated overnight. The solid is filtered and put in the oven at 40oC under vacuum to obtain 8.358Kg of the **title compound.**
NMR (DMSO): δ (ppm) 7.47 ppm (t, 1H). 7.35 ppm (dd, 2H), 7.08 ppm (d, 5H), 4.46 ppm (s, 2H), 3.93 ppm (s, 2H), 2.75-2.60 ppm (m, 4H), 2.5 ppm (bm, 2H), 1.94 ppm (m, 2H)

### Example 2

### 1-[(3,5-Dichlorophenyl)methyl]-3-[4-(4-fluorophenyl)-4-piperidinyl]-1,5-dihydro-2H-pyrrol-2-one hydrochloride

To a suspension of example **1** (15 mg) in dry Et2O (0.5 mL) at 0°C, hydrogen chloride (1M solution in Et2O - 39 µL) was added dropwise, the resulting thick suspension was stirred at 0°C for 15 minutes, then solvent was evaporated under a Nitrogen flux and the solid residue was triturated in pentane (3 x 1 mL) to obtain the title compound (16 mg) as a white solid.
NMR (d₆-DMSO): δ (ppm) 8.52 (bs, 2H); 7.52 (t, 1H); 7.42 (dd, 2H); 7.2 - 7.16 (m, 5H); 4.51 (s, 2H); 3.97 (s, 2H); 3.12 - 3.0 (m, 4H); 2.78 (d, 2H); 2.3 (m, 2H).
MS (ES/+): m/z=419 [M-HCl+H]⁺.

### Example 3

### 1-[(3,5-Dichlorophenyl)methyl]-3-[4-(4-fluorophenyl)-1-methyl-4-piperidinyl]-1,5-dihydro-2H-pyrrol-2-one

### Method A

To a solution of example 1 (16.1 g) in CH3CN (200 mL) 37% wt formaldehyde in water (6.5 mL) was added, the resulting mixture was stirred at r.t. for 15 minutes then NaBH(OAc)₃ (12.2 g) was added portionwise (exothermic reaction observed). The reaction mixture was stirred at r.t. for 1 h, then it was quenched with aqueous 5% sodium hydrogen carbonate solution (40 ml), CH3CN was evaporated and the residue was diluted with further aqueous 5% sodium hydrogen carbonate solution (200 mL) and extracted with DCM (3 x 200 mL). The collected organic phases were dried, concentrated *in vacuo* to give a crude white foam (16.5 g) which was purified by flash chromatography (Biotage Flash 75L, DCM , then DCM/MeOH from 95:5 to 8:2). The title compound (12.7 g) was obtained as a white solid.
T.I.c.: DCM/MeOH 9:1, Rf = 0.41 (detection with ninhydrine).
NMR (CDCl₃): δ (ppm) 7.35 (dd, 2H); 7.23 (t, 1 H); 6.98 (t, 2H); 6.96 (m, 2H); 6.76 (bt, 1 H); 4.46 (s, 2H); 3.76 (dd, 2H); 2.70 - 2.25 (bm, 8H); 2.23 (s, 3H).
MS (ES/+): m/z=433 [M+H]⁺.

### Method B

Thionyl chloride (0.67 ml) was added to a cooled solution (approximately -5°C) of dichloromethane (10 ml) and pyridine (1.86 ml). at such a rate to maintain the temperature lower than 0 °C. The resulting solution was stirred at around -5 °C for 10 minutes and a solution of intermediate 69 (2.0 g) in dichloromethane (10 ml).was slowly added maintaining the temperature lower than 0 °C. The resulting solution was then stirred and warmed to room temperature in 30-40 minutes and then stirred at room temperature until complete for about an additional 1.0 hour.. The reaction was quenched with water (20 ml). The mixture was settled and separated. To the dichloromethane layer was added 25% potassium carbonate aqueous solution. The mixture was stirred for 30 minutes and then settled and separated. The organic phase wa s concentrated to dryness to afford the title compound as an oil (1.5 - 1.7g, 1H-NMR: 7.31-7.20 (m, 2H), 7.16 (s, 1H), 6.93-6.89 (m, 4H), 6.71 (s, 1 H), 4.39 (s, 2H), 3.71 (s, 2H), 2.52-2.28 (br, m), 2.21 (s, 3H). 66
MS: M+1 433

### Example 4

### 1-[(3,5-Dichlorophenyl)methyl]-3-[4-(4-fluorophenyl)-1-methyl-4-piperidinyl]-1,5-dihydro-2H-pyrrol-2-one hydrochloride

To a suspension of example **3** (10.7 g) in dry Et2O (200 mL) at 0°C, hydrogen chloride (1M solution in Et2O - 26 mL) was added dropwise, the resulting thick suspension was stirred at 0°C for 1 h, then it was filtered under a Nitrogen atmosphere on a Gooch filter and washed with Et2O (2 x 100 mL) and with pentane (3 x 100 mL), then dried *in vacuo* at 40°C for 3 h and at r.t. for 14 h. The title compound (11.2 g) was obtained as a white solid.
NMR (d₆-DMSO): δ (ppm) 9.97 (b, 1 H); 7.51 (bs, 1 H); 7.40 (s, 1H); 7.36 (dd, 2H); 7.16 (d, 2H); 7.15 (t, 2H); 4.51 (s, 2H); 4.01 (s, 2H); 3.55 - 3.3 (m, 2H); 3.04 (bd, 1H); 2.96 (bdd, 1 H); 2.76 (d, 3H); 2.55 - 2.3 (m, 2H); 2.13 (bt, 2H).
MS (ES/+): m/z=433 [M-HCl+H]⁺.

### Example 5

### 1-[1-(3,5-Dichlorophenyl)ethyl]-3-[4-(4-fluorophenyl)-4-piperidinyl]-1,5-dihydro-2H-pyrrol-2-one (Chain Enantiomer 1)

TFA (0.8 mL) was added to intermediate **51** (0.019 g) and the mixture was allowed to stir at 60°C for 1 h. After this time the mixture was cooled to r.t. and concentrated *in vacuo*. The crude thus obtained was diluted with DCM and poured into an aqueous 2M sodium hydroxide solution previously cooled to 0°C. The organic layer was separated then was dried and concentrated *in vacuo* to give a residue which was purified by flash chromatography (DCM, then DCM/MeOH from 95:5 to 8:2) to give the title compound (0.014 g) as a white foam.
T.I.c.: DCM/MeOH 9:1, Rf=0.39 (detection with ninhydrine).
NMR (CDCl₃): δ (ppm) 7.37 (dd, 2H); 7.27 (m, 1H); 7.06 (d, 2H); 7.04 (t, 2H); 6.72 (s, 1 H); 5.40 (q, 1H); 3.88 (dd, 1H); 3.60 (dd, 1H); 2.96 (m, 4H); 2.62 (m, 2H); 2.27 (m, 2H); 1.56 (d, 3H).
MS (ES/+): m/z=433 [M+H]⁺.

### Example 6

### 1-[(1S)-1-(3-Chloro-1-naphthalenyl)ethyl]-3-[4-(4-fluorophenyl)-4-piperidinyl]-1,5-dihydro-2H-pyrrol-2-one

TFA (4 mL) was added to intermediate 52 (0.128 g) and the mixture was allowed to stir at r.t. for 20 minutes. TFA was evaporated and TFA (2 mL) was added. The mixture was stirred at 60°C for 1.15 h. After this time the mixture was cooled to r.t. and concentrated *in vacuo*. The crude thus obtained was diluted with DCM and poured into an aqueous 2M sodium hydroxide solution previously cooled to 0°C. The organic layer was separated then was dried and concentrated *in vacuo* to give a residue which was purified by flash chromatography (DCM, then DCM/MeOH from 95:5 to 8:2) to give the title compound (0.048 g) as a white foam.
NMR (CDCl₃): δ (ppm) 7.89 (d, 1 H); 7.82 (d, 1 H); 7.77 (d, 1 H); 7.51 (t, 1 H); 7.48 (d, 1 H); 7.37 (td, 1 H); 7.34 (dd, 2H); 7.02 (t, 2H); 6.59 (s, 1 H); 6.09 (q, 1H); 3.8 (dd, 1 H); 3.06 (dd, 1 H); 3.05-2.85 (bm, 4H); 2.68 (bd, 1 H); 2.6 (bd, 1 H); 2.24 (bm, 2H); 1.69 (d, 3H).
MS (ES/+): m/z=449 [M+H]⁺.

### Example 7

### 1-[(3-Chloro-1-naphthalenyl)methyl]-3-[4-(4-fluorophenyl)-4-piperidinyl]-1,5-dihydro-2H-pyrrol-2-one

TFA (10 mL) was added to intermediate 53 (210 mg) at 0°C and the mixture was allowed to stir at 0°C for 15 minutes. TFA was evaporated and then added again to reach the original volume. The mixture was stirred at 60°C for 2 h. After this time the mixture was cooled to r.t. and concentrated *in vacuo.* The crude thus obtained was diluted with DCM and poured into an aqueous 2.5M sodium hydroxide solution previously cooled to 0°C. The organic layer was separated, dried on sodium sulphate and concentrated *in vacuo* to give a residue which was purified by flash chromatography (elution: DCM, then DCM/MeOH from 95:5 to 70:30) to give the title compound (140 mg) as a white solid.
T.Lc.: DCM/MeOH 8:2, Rf = 0.08 (detection with ninhydrine).
NMR (CDCl₃): δ (ppm) 7.9 (d, 1 H); 7.77 (s, 1H); 7.74 (d, 1 H); 7.49 (t, 1 H); 7.38 (t, 1 H); 7.33 (dd, 2H); 7.25 (d, 1 H); 6.99 (t, 2H); 6.64 (s, 1 H); 4.92 (s, 2H); 3.63 (bs, 2H); 2.95 (bm, 4H); 2.62 (bm, 2H); 2.29 (bm, 2H).
MS (ES/+): m/z=435 [M+H]⁺.

### Example 8

### 4-({3-[4-(4-Fluorophenyl)-4-piperidinyl]-2-oxo-2,5-dihydro-1H-pyrrol-1-yl}methyl)-2-naphthalenecarbonitrile

TFA (12 mL) was added to intermediate **54** (120 mg) at 0°C and the mixture was allowed to stir at 0°C for 15 minutes. TFA was evaporated and then added again to reach the original volume. The mixture was stirred at 60°C for 1.5 h. After this time the mixture was cooled to r.t. and concentrated *in vacuo.* The crude thus obtained was diluted with DCM and poured into an aqueous 2.5M sodium hydroxide solution previously cooled to 0°C. The organic layer was separated, dried on sodium sulphate and concentrated *in vacuo* to give a residue which was purified by flash chromatography (elution: DCM, then DCM/MeOH from 9:1 to 7:3) to give the title compound (70 mg) as a white solid.
T.I.c.: DCM/MeOH 8:2, Rf = 0.06 (detection with ninhydrine).
NMR (CDCl₃): δ (ppm) 8.18 (s, 1 H); 8.02 (d, 1 H); 7.89 (d, 1 H); 7.6 (td, 1H); 7.55 (td, 1H); 7.41 (s, 1H); 7.33 (dd, 2H); 6.99 (td, 2H); 6.67 (s, 1H); 4.96 (s, 2H); 3.64 (bs, 2H); 2.92 (bm, 4H); 2.59 (bm, 2H); 2.27 (bm, 2H).
MS (ES/+): m/z = 426 [M+H]⁺.

### Example 9

### 1-[1-(3,5-Dichlorophenyl)ethyl]-3-[4-(4-fluorophenyl)-4-ppieridinyl]-1,5-dihydro-2H-pyrrol-2-one (Chain Enantiomer 2)

To a suspension of intermediate **31** (0.9 g) in THF (20 mL) and water (5 mL) osmium tetraoxide 4% wt solution in water (1.5 mL) was added. The mixture was stirred at r.t. for 1 h during which it became dark, then NalO₄ (1.4 g) was added and the brownish reaction mixture was stirred at r.t. for 14 h. After this time it was diluted with water and extracted with AcOEt. The organic layer was dried and concentrated *in vacuo*. The residue was purified by flash chromatography (CH/AcOEt from 9:1 to 7:3) and the fractions with Rf=0.37 (CH/AcOEt 6:4, detection with ninhydrine) and MS (ES/+): m/z=573 [M+Na]⁺ were collected to give an intermediate (0.4 g) to which TFA (0.8 mL) was added and the mixture was allowed to stir at 60°C for 2 h. After this time the mixture was cooled to r.t. and concentrated *in vacuo*. The crude thus obtained was diluted with DCM and poured into an aqueous 2M sodium hydroxide solution previously cooled to 0°C. The organic layer was separated then was dried and concentrated *in vacuo* to give a residue which was purified by flash chromatography (DCM, then DCM/MeOH from 95:5 to 8:2) to give the title compound (0.065 g) as a white foam.
T.I.c.: DCM/MeOH 9:1, Rf=0.39 (detection with ninhydrine).
NMR (CDCl₃): δ (ppm) 7.37 (dd, 2H); 7.27 (m, 1 H); 7.06 (d, 2H); 7.04 (t, 2H); 6.72 (s, 1 H); 5.40 (q, 1H); 3.88 (dd, 1H); 3.60 (dd, 1 H); 2.96 (m, 4H); 2.62 (m, 2H); 2.27 (m, 2H); 1.56 (d, 3H).
MS (ES/+): m/z=433 [M+H]⁺.

### Example 10

### 1-[(1R)-1-(3-Chloro-1-naphthalenyl)ethyl]-3-[4-(4-fluorophenyl)-4-piperidinyl]-1,5-dihydro-2H-pyrrol-2-one

To a suspension of intermediate **32** (0.32 g) in THF (16 mL) and water (4 mL) osmium tetraoxide 4% wt solution in water (1 mL) was added. The mixture was stirred at r.t. for 45 minutes, then NalO₄ (0.5 g) was added portion wise and the reaction mixture was stirred at r.t. for 1 h. After this time it was diluted with water and extracted with AcOEt. The organic layer was dried and concentrated *in vacuo*. The residue was purified by flash chromatography (CH/AcOEt from 9:1 to 7:3) and the fractions with Rf=0.78 and Rf=0.63 (CH/AcOEt 1:1, detection with ninhydrine) and both having MS (ES/+): m/z=589 [M+Na]⁺ were collected to give a mixture of intermediates (0.068 g) to which TFA (2 mL) was added and the mixture was allowed to stir at 60°C for 2 h. After this time the mixture was cooled to r.t. and concentrated *in vacuo*. The crude thus obtained was diluted with DCM and poured into an aqueous 2M sodium hydroxide solution previously cooled to 0°C. The organic layer was separated then was dried and concentrated *in vacuo* to give the title compound (0.05 g) as a white foam.
T.I.c.: DCM/MeOH 8:2, Rf=0.77 (detection with ninhydrine).

### Example 11

### 1-[1-(3,5-Dichlorophenyl)ethyl]-3-[4-(4-fluorophenyl)-1-methyl-4-piperidinyl]-1,5-dihydro-2H-pyrrol-2-one (Chain Enantiomer 1)

To a solution of example **5** (0.241g) in CH3CN (14 mL) 37%wt formaldehyde in water (0.130 mL) was added. The resulting mixture was stirred at r.t. for 15 minutes then NaBH(OAc)₃ (0.235 g) was added. The reaction mixture was stirred at r.t. for 1 h, then it was quenched with water, CH3CN was evaporated and the residue was diluted with water and extracted with DCM. The collected organic phases were dried, concentrated in vacuo to give a crude that was purified by flash chromatography (DCM, then DCM/MeOH from 98:2 to 9:1) to give the title compound (0.177 g) as a white solid.
T.I.c.: DCM/MeOH 85:15, Rf=0.54 (detection with ninhydrine).
MS (ES/+): m/z=447 [M+H]⁺.

### Example 12

### 1-[(1S)-1-(3-Chloro-1-naphthalenyl)ethyl]-3-[4-(4-fluorophenyl)-1-methyl-4-piperidinyl]-1,5-dihydro-2H-pyrrol-2-one

To a solution of example **6** (0.025 g) in CH3CN (2 mL) 37%wt formaldehyde in water (0.013 mL) was added. The resulting mixture was stirred at r.t. for 15 minutes then NaBH(OAc)₃ (0.024 g) was added. The reaction mixture was stirred at r.t. for 2 h, then it was quenched with water, CH3CN was evaporated and the residue was diluted with water and extracted with DCM. The collected organic phases were washed with an aqueous std NaHCO3 solution, dried, concentrated *in vacuo* to give a crude that was purified by flash chromatography (DCM, then DCM/MeOH from 98:2 to 9:1) to give the title compound (0.022 g) as a white foam.
T.I.c.: DCM/MeOH 9:1, Rf=0.6 (detection with ninhydrine).
NMR (CDCl₃): δ (ppm) 7.85 (d, 1 H); 7.82 (d, 1H); 7.77 (d, 1 H); 7.51 (t, 1 H); 7.47 (d, 1 H); 7.40-7.32 (m, 3H); 7.04 (t, 2H); 6.67 (bs, 1 H); 6.08 (q, 1 H); 3.82 (d, 1 H); 3.09 (d, 1 H); 3.00-2.55 (bm, 6H); 2.52 (bs, 3H); 2.0-1.8 (bm, 2H); 2.24 (bm, 2H); 1.69 (d, 3H).
MS (ES/+): m/z=463 [M+H]⁺.

### Example 13

### 1-[(3-Chloro-1-naphthalenyl)methyl]-3-[4-(4-fluorophenyl)-1-methyl-4-piperidinyl]-1,5-dihydro-2H-pyrrol-2-one

To a solution of example **7** (50 mg) in CH₃CN (2 mL) 37%wt formaldehyde in water (0.020 mL) was added. The resulting mixture was stirred at r.t. for 10 minutes then NaBH(OAc)₃ (37 mg) was added. The reaction mixture was stirred at r.t. for 1.5 h, then it was quenched with water, CH₃CN was evaporated and the residue was diluted with a 5% aqueous solution of NaHCO₃ and extracted with DCM (2 x 10 mL); the organic layers were dried, concentrated *in vacuo* to give a crude that was purified by flash chromatography (DCM, then DCM/MeOH from 95:5 to 70:30) to give the title compound (40 mg) as a white solid.
T.I.c.: DCM/MeOH 8:2, Rf = 0.35 (detection with ninhydrine).
NMR (CDCl₃): δ (ppm) 7.88 (d, 1 H); 7.76 (d, 1 H); 7.72 (d, 1H); 7.48 (t, 1H); 7.36 (tt, 1 H); 7.33 (dd, 2H); 7.23 (d, 1 H); 6.96 (t, 2H); 6.68 (s, 1 H); 4.91 (s, 2H); 3.62 (s, 2H); 2.56 (bt, 4H); 2.36 (bt, 4H); 2.22 (s, 3H).
MS (ES/+): m/z=449 [M+H]⁺.

Following the same procedure described for example 13, example 14, 15 and 16 were obtained.

### Example 14

### 1-[1-(3,5-Dichlorophenyl)ethyl]-3-[4-(4-fluorophenyl)-1-methyl-4-piperidinyl]-1,5-dihydro-2H-pyrrol-2-one (Chain Enantiomer 2)

Starting from example **9** (0.10 g), 0.025 g of the title compound were obtained.
T.I.c.: DCM/MeOH 85:15, Rf=0.5 (detection with ninhydrine).
NMR (CDCl₃): δ (ppm) 7.39 (dd, 2H); 7.28 (m, 1H); 7.08 - 7.06 (m, 4H); 6.82 (bs, 1 H); 5.36 (q, 1 H); 3.89 - 3.62 (dd, 2H); 3.3 - 2.2 (bm, 8H); 2.65 (s, 3H); 1.53 (d,3H).
MS (ES/+): m/z=447 [M+H]⁺.

### Example 15

### 1-[(1R)-1-(3-Chloro-1-naphthalenyl)ethyl)-3-[4-(4-fluorophenyl)-1-methyl-4-piperidinyl]-1,5-dihydro-2H-pyrrol-2-one

Starting from example **10** (0.050 g), 0.024 g of the title compound were obtained.
T.I.c.: DCM/MeOH 85:15, Rf=0.6 (detection with ninhydrine).
MS (ES/+): m/z=463 [M+H]⁺.

### Example 16

### 4-({3-[4-(4-Fluorophenyl)-1-methyl-4-piperidinyl]-2-oxo-2,5-dihydro-1H-pyrrot-1-yl}methyl)-2-naphthalenecarbonitrile

Starting from example **8** (0.049 g), 0.039 g of the title compound were obtained.
T.I.c.: DCM/MeOH 8:2 with addition of 1% of NH4OH, Rf=0.56 (detection with ninhydrine). NMR (CDCl₃): δ (ppm) 8.2 (s, 1 H); 8.0 (d, 1 H); 7.89 (d, 1 H); 7.57 (m, 2H); 7.41 (d, 1 H); 7.33 (m, 2H); 6.99 (t, 2H); 6.72 (s, 1H); 4.95 (s, 2H); 3.65 (m, 2H); 2.8-2.3 (bm, 8H); 2.31 (s, 3H).

### Example 17

### 1-[1-(3,5-Dichlorophenyl)ethyl]-3-[4-(4-fluorophenyl)-1-methyl-4-piperidinyl]-1,5-dihydro-2H-pyrrol-2-one hydrochloride(Chain Enantiomer 1)

Hydrogen chloride (1M solution in Et2O - 0.43 mL) was added to a solution of example **11** (0.176 g) in dry Et2O (4 mL) previously cooled to 0°C under a Nitrogen atmosphere. The mixture was stirred at 0°C for 15 minutes, then it was concentrated *in vacuo*. The residue was triturated with pentane (3 x 1 mL) to give the title compound (0.180 g) as a white solid.
NMR (d₆-DMSO): δ (ppm) 10.11/9.94 (2bs, 1H); 7.51 - 7.46 (m, 1H); 7.41 (bs, 1H); 7.32 (dd, 2H); 7.2 - 7.1 (m, 4H); 5.11 (m, 1 H); 4.14 - 3.74 (dd, 2H); 3.3 - 2.13 (bm, 8H); 2.77 (d, 3H); 1.52 (d, 3H).
MS (ES/+): m/z=447 [M-HCl+H]⁺.

### Example 18

### 1-[(1R)-1-(3-Chloro-1-naphthalenyl)ethyl]-3-[4-(4-fluorophenyl)-1-methyl-4-piperidinyl]-1,5-dihydro-2H-pyrrol-2-one hydrochloride

Hydrogen chloride (1 M solution in Et2O - 57 µL) was added to a solution of example **15** (0.024 g) in dry Et2O (1 mL) previously cooled to 0°C under a Nitrogen atmosphere. The mixture was stirred at 0°C for 15 minutes, then it was concentrated *in vacuo*. The residue was triturated with pentane (3 x 1 mL) to give the title compound (0.024 g) as a white solid.
NMR (d₆-DMSO): δ (ppm) 9.57 (bs, 1H); 8.01 (s, 1 H); 7.92 (d, 1 H); 7.88 (bd, 1 H); 7.55 (t, 1 H); 7.47 (s, 1 H); 7.40 (t, 1 H); 7.3 - 6.5 (bm, 5H); 5.87 (q, 1 H); 4.1 - 3.9 (bm, 1 H); 3.2 - 2.0 (bm, 12H); 1.61 (bs, 3H).
MS (ES/+): m/z=463 [M-HCl+H]⁺.

### Example 19

### 1-[(1S)-1-(3-Chloro-1-naphthalenyl)ethyl]-3-[1-(cyclopropylmethyl)-4-(4-fluorophenyl)-4-piperidinyl]-1,5-dihydro-2H-pyrrol-2-one

To a solution of example **6** (0.018 g) in CH3CN (2 mL) cyclopropancarboxaldehyde (0.015 mL) was added. The resulting mixture was stirred at r.t. for 15 minutes then NaBH(OAc)₃ (0.017 g) was added. The reaction mixture was stirred at r.t. for 2 h, then it was quenched with water, CH3CN was evaporated and the residue was diluted with water and extracted with DCM. The collected organic phases were washed with an aqueous saturated solution of NaHCO3, dried, concentrated in vacuo to give a crude that was purified by flash chromatography (DCM, then DCM/MeOH from 98:2 to 9:1) to give the title compound (0.017 g) as a white foam.
T.I.c.: DCM/MeOH 9:1, Rf=0.51 (detection with ninhydrine).
NMR (CDCl₃): δ (ppm) 7.67 (d, 1 H); 7.63 (d, 1 H); 7.58 (d, 1 H); 7.33 (t, 1 H); 7.28 (d, 1 H); 7.17 (m, 3H); 6.84 (t, 2H); 6.48 (s, 1 H); 5.89 (q, 1H); 3.62 (d, 1 H); 2.89 (d, 1 H); 2.7-2.1 (bm, 10H); 1.5 (d, 3H); 0.8 (bm, 1 H); 0.4 (bs, 2H); -0.01 (bs, 2H).
MS (ES/+): m/z=503 [M+H]⁺.

### Example 20

### 1-[(3-Chloro-1-naphthalenyl)methyl]-3-[1-(cyclopropylmethyl)-4-(4-fluorophenyl)-4-piperidinyl]-1,5-dihydro-2H-pyrrol-2-one

To a solution of example **7** (50 mg) in CH3CN (2 mL) cyclopropanecarboxaldehyde (0.043 mL) was added. The resulting mixture was stirred at r.t. for 10 minutes then NaBH(OAc)₃ (37 mg) was added. The reaction mixture was stirred at r.t. for 1.5 h, then it was quenched with water, CH₃CN was evaporated and the residue was diluted with a 5% aqueous solution of NaHCO₃ and extracted with DCM (2 x 10 mL); the organic layers were dried, concentrated *in vacuo* to give a crude that was purified by flash chromatography (DCM, then DCM/MeOH from 95:5 to 80:20) to give the title compound (22 mg) as a white solid.
T.I.c.: DCM/MeOH 8:2, Rf = 0.5 (detection with ninhydrine).
NMR (CDCl₃): δ (ppm) 7.87 (d, 1 H); 7.75 (d, 1 H); 7.72 (d, 1 H); 7.47 (t, 1H); 7.36 (t, 1H); 7.33 (dd, 2H); 7.22 (d, 1 H); 6.96 (t, 2H); 6.68 (s, 1 H); 4.91 (s, 2H); 3.61 (d, 2H); 2.62 (bd, 4H); 2.38 (bd, 4H); 2.22 (bs, 2H); 0.84 (b, 1H); 0.47 (d, 2H); 0.05 (d, 2H).
MS (ES/+): m/z=489 [M+H]⁺.

### Example 21

### 1-[(3,5-Dichlorophenyl)methyl]-3-[4-(4-fluorophenyl)-4-piperidinyl]-2-pyrrolidinone

TFA (200 µl) was added to a solution of intermediate 37 (35 mg) in dry DCM (1.8 mL) and the mixture was stirred for 3 h at r.t. Then it was diluted with DCM, washed with an aqueous std sodium hydrogen carbonate solution, dried and concentrated *in vacuo*. The residue was purified by SCX to give the title compound (22 mg) as a colourless oil.
T.I.c.: DCM/MeOH 2:8, Rf=0.16 (detection with ninhydrine).
NMR (d₆-DMSO): δ (ppm) 7.43 (t, 1 H); 7.36 (dd, 2H); 7.06 (t, 2H); 6.96 (d, 2H); 5.34 (bs, 1 H); 4.27 (d, 1H); 4.04 (d, 1 H); 2.94 (dd, 2H); 2.89 (m, 1 H); 2.72 (t, 1 H); 2.6 (m, 1 H); 2.58 (m, 1H); 2.49 (m, 1H); 2.41 (dd, 1H); 2.16 (bd, 1H);1.95 (m, 2H); 1.82 (m, 1H); 1.56 (m, 1 H).
MS (ES/+): m/z=421 [M+H]⁺.

### Example 22

### 1-[(1S)-1-(3-Chloro-1-naphthalenyl)ethyl]-3-[4-(4-fluorophenyl)-4-piperidinyl]-2-pyrrolidinone (Diastereoisomer 1)

TFA (0.8 mL) was added to a solution of intermediate **42** (21 mg) in dry DCM (3.2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (14.5 mg) as a white foam.
NMR (CDCl₃): δ (ppm) 7.95 (d, 1H); 7.76 (s, 1H); 7.75 (d, 1H); 7.53 (m, 2H); 7.38 (m, 2H); 7.27 (s, 1 H); 7.11 (t, 2H); 5.93 (q, 1 H); 3.32 (bt, 2H), 2.93 (m, 1 H); 2.77 (bt, 1 H); 2.62 (bm, 1H); 2.3 - 2.5 (m, 3H); 2.05 - 2.25 (m, 2H); 1.89 (bd, 1 H); 1.5 - 1.75 (m, 2H); 1.25 (d, 3H).

### Example 23

### 1-[(1S)-1-(3-Chloro-1-naphthalenyl)ethyl]-3-[4-(4-fluorophenyl)-4-piperidinyl]-2-pyrrolidinone (Diastereoisomer 2)

TFA (0.8 mL) was added to a solution of intermediate **43** (19 mg) in dry DCM (3.2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH); Solvent evaporation gave the title compound (14.3 mg) as a white foam.
NMR (CDCl₃): δ (ppm) 7.84 (d, 1H); 7.76 (s, 1H); 7.75 (d, 1H); 7.52 (t, 1H); 7.4 (t, 1H); 7.28 (s, 1 H); 7.23 (dd, 2H); 6.71 (t, 2H); 5.99 (q, 1H), 3.02 (bt, 1 H), 3.01 (m, 1H). 2.99 (m, 1H); 2.84 (bd, 1 H); 2.6 - 2.75 (m, 3H); 2.22 (bt, 1H); 2.17 (bd, 1H); 2.0 - 2.15 (m, 2H), 1.79 (m, 1H); 1.55 (d, 3H); 1.38 (m, 1H).

### Example 24

### 1-[1-(3,5-Dichlorophenyl)ethyl]-3-[4-(4-fluorophenyl)-4-piperidinyl]-2-pyrrolidinone (Diastereoisomer 1 Chain Enantiomer 1)

TFA (1.0 mL) was added to a solution of intermediate **44** (125 mg) in dry DCM (4.0 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (91 mg) as a white foam.
HPLC(LC/MS -ES /+):t_{R} = 4.6 min
MS (ES/+): m/z=435 [M+H]⁺
NMR (CDCl₃): δ (ppm) 7.33 (dd, 2H); 7.21 (t, 1 H); 7.03 (d, 2H); 7.02 (t, 2H); 5.24 (q, 1 H); 3.03 (m, 1 H); 2.99 (m, 1 H); 2.81 (bd, 1 H); 2.78 (t, 1 H), 2.66 (tm, 1 H), 2.63 (m, 1H), 2.56 (dd, 1H); 2.15 - 2.35 (bm, 3H); 1.94 (m, 1 H); 1.88 (m 1 H); 1.75 (m, 1 H); 1.11 (d, 3H).

### Example 25

### 1-[1-(3,5-Dichlorophenyl)ethyl]-3-[4-(4-fluorophenyl)-4-piperidinyl]-2-pyrrolidinone (Diastereoisomer 2 Chain Enantiomer 1)

TFA (1.5 mL) was added to a solution of intermediate **45** (165 mg) in dry DCM (6.0 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (101 mg) as a white foam.
HPLC(LC/MS -ES/+):t_{R} = 4.6 min
MS (ES/+): m/z=435 [M+H]⁺
NMR (CDCl₃): δ (ppm) 7.34 (dd, 2H); 7.24 (t, 1H); 6.99 (t, 2H); 6.82 (dd, 2H); 5.27 (q, 1H); 3.1 (m, 1 H); 3.08 (m, 1 H); 3.04 (m, 1 H); 2.84 (td, 1 H), 2.75 (t, 1 H), 2.68 (td, 1 H), 2.4 (td, 1H); 2.10 - 2.4 (bm, 3H); 1.99 (tm, 1 H); 1.67 (m 1 H); 1.41 (dd, 3H).

### Example 26

### 1-[1-(3,5-Dichlorophenyl)ethyl]-3-[4-(4-fluorophenyl)-4-piperidinyl]-2-pyrrolidinone (Diastereoisomer 1 Chain Enantiomer 2)

TFA (1.0 mL) was added to a solution of intermediate **46** (133 mg) in dry DCM (4.0 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (97 mg) as a white foam.
HPLC(LC/MS -ES /+):t_{R} = 4.6 min
MS (ES/+): m/z=435 [M+H]⁺
NMR (CDCl₃): δ (ppm) 7.28 (dd, 2H); 7.17 (t, 1H); 6.99 (t, 2H); 6.98 (d, 2H); 5.18 (q, 1H); 3.04 (m, 1 H); 3.02 (m, 1 H); 2.86 (bd, 1 H); 2.77 (t, 1 H), 2.59 (m, 1H), 2.56 (m, 1H), 2.52 (tm, 1 H); 2.0 - 2.4 (bm, 3H); 1.94 (td, 1 H); 1.85 (m 1 H); 1.69 (m, 1 H), 1.06 (d, 3H).

### Example 27

### 1-[1-(3,5-Dichlorophenyl)ethyl]-3-[4-(4-fluorophenyl)-4-piperidinyl]-2-pyrrolidinone (Diastereoisomer 2 Chain Enantiomer 2)

TFA (2.0 mL) was added to a solution of intermediate **47** (180 mg) in dry DCM (8.0 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (111 mg) as a white foam.
HPLC(LC/MS -ES /+):t_{R} = 4.6 min
MS (ES/+): m/z=435 [M+H]⁺
NMR (CDCl₃): δ (ppm) 7.34 (dd, 2H); 7.24 (t, 1H); 6.99 (t, 2H); 6.82 (dd, 2H); 5.27 (q, 1 H); 3.1 (m, 1 H); 3.08 (m, 1 H); 3.04 (m, 1 H); 2.84 (td, 1H), 2.75 (t, 1 H), 2.68 (td, 1 H), 2.4 (td, 1 H); 2.10 - 2.4 (bm, 3H); 1.99 (tm, 1 H); 1.67 (m 1 H); 1.41 (dd, 3H).

### Example 28

### 4-({3-[4-(4-Fluorophenyl)-4-piperidinyl]-2-oxo-1-pyrrolidinyl}methyl)-2-naphthalenecarbonitrile (Enantiomer 1)

TFA (0.75 mL) was added to a solution of intermediate **57** (40 mg) in dry DCM (3 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (25 mg) as a white foam.
NMR (CDCl₃): δ (ppm) 8.15 (s, 1 H); 8.08 (d, 1 H); 7.89 (dd, 1 H); 7.66 (td, 1 H); 7.61 (td, 1H); 7.27 (dd, 2H); 7.25 (s, 1H); 6.84 (td, 2H); 4.66 (s, 2H); 3.05 (bt, 2H); 2.92(bd, 1H), 2.6 - 2.85 (m, 4H); 2.18 - 2.35 (m, 3H); 2.02 (m, 1 H); 1.88 (m, 1 H); 1.63 (m, 1 H).

### Example 29

### 4-({3-[4-(4-Fluorophenyl)-4-piperidinyl]-2-oxo-1-pyrrolidinyl}methyl)-2-naphthalenecarbonitrile (Enantiomer 2)

TFA (0.75 mL) was added to a solution of intermediate **58** (33 mg) in dry DCM (3 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (18.5 mg) as a white foam.
NMR (CDCl₃): δ (ppm) 8.15 (d, 1 H); 8.05 (d, 1 H); 7.89 (dd, 1 H); 7.66 (td, 1 H); 7.61 (td, 1 H); 7.26 (dd, 2H); 7.24 (s, 1 H); 6.85 (td, 2H); 4.7 (d, 1 H); 4.61 (d, 1 H); 3.14 (bt, 2H); 3.11 (bd, 1 H), 2.62 - 2.91 (m, 4H); 2.26 - 2.40 (m, 3H); 2.07 (m, 1 H); 1.89 (m, 1 H); 1.62 (m, 1H).

### Example 30

### 7-Fluoro-4-({3-[4-(4-fluorophenyl)-4-piperidinyl]-2-oxo-1-pyrrolidinyl}methyl)-2-naphthalenecarbonitrile (Enantiomer 2)

TFA (0.5 mL) was added to a solution of intermediate **59** (28 mg) in dry DCM (2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (19 mg) as a white foam.
HPLC(LC/MS -ES /+):t_{R} = 4.08 min
MS (ES/+): m/z=446 [M+H]⁺

NMR (CDCl₃): δ (ppm) 8.17 (dd, 1 H); 8.08 (s, 1 H); 7.5 (dd, 1 H); 7.43 (td, 1H); 7.27 (dd, 2H); 7.22 (s, 1 H); 6.85 (td, 2H); 4.69 (d, 1 H); 4.55 (d, 1 H); 2.95 (bt, 2H); 2.85-2.50 (m, 4H), 2.3-2.10 (m, 3H); 2.00-1.50 (m, 4H).

### Example 31

### 6-Fluoro-4-({3-[4-(4-fluorophenyl)-4-piperidinyl]-2-oxo-1-pyrrolidinyl}methyl)-2-naphthalenecarbonitrile (Enantiomer 2)

TFA (0.5 mL) was added to a solution of intermediate **60** (19 mg) in dry DCM (2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (11 mg) as a white foam.
HPLC(LC/MS -ES /+):t_{R} = 4.06 min
MS (ES/+): m/z=446 [M+H]⁺

NMR (CDCl₃): δ (ppm) 8.14 (s, 1 H); 7.91 (td, 1 H); 7.83 (bd, 1 H); 7.41 (tt, 1H); 7.29 (s, 1 H); 7.26 (dd, 2H); 6.85 (td, 2H); 4.65 (d, 1H); 4.52 (d, 1 H); 2.99 (bt, 2H); 2.90-2.55 (m, 4H), 2.4 - 2.1 (m, 3H); 2.00 - 1.50 (m, 4H).

### Example 32

### 7-Fluoro-4-({3-[4-(4-fluorophenyl)-4-piperidinyl]-2-oxo-1-pyrrolidinyl}methyl)-2-naphthalenecarbonitrile (Enantiomer 1)

TFA (0.5 mL) was added to a solution of intermediate **61** (52 mg) in dry DCM (2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (31 mg) as a white foam.
HPLC(LC/MS -ES /+):t_{R} = 3.99 min
MS (ES/+): m/z=446 [M+H]⁺
NMR (CDCl₃): δ (ppm) 8.23 (dd, 1H); 8.14 (s, 1 H); 7.55 (dd, 1 H); 7.48 (m, 1 H); 7.33 (dd, 2H); 7.28 (d, 1H); 6.9 (t, 2H); 4.75 (d, 1 H); 4.6 (d, 1 H); 2.98 (m, 2H); 2.85-2.7 (m, 4H), 2.65 (td, 1H); 2.35 - 2.15 (m, 3H); 2.00-1.75 (m, 2H); 1.72 (m, 1H).

### Example 33

### 6-Fluoro-4-({3-[4-(4-fluorophenyl)-4-piperidinyl]-2-oxo-1-pyrrolidinyl}methyl)-2-naphthalenecarbonitrile (Enantiomer 1)

TFA (0.5 mL) was added to a solution of intermediate **62** (21 mg) in dry DCM (2 mL) at 0°C under Nitrogen atmosphere. The reaction mixture was stirred 1 h before being concentrated *in vacuo* at 0°C. The residue was purified on a SCX-cartridge (loaded with DCM, washed with MeOH, eluted with NH₃ 2 M in MeOH, followed by MeOH). Solvent evaporation gave the title compound (16 mg) as a white foam.
HPLC(LC/MS -ES /+):t_{R} = 3.95 min
MS (ES/+): m/z=446 [M+H]⁺

NMR (CDCl₃): δ (ppm) 8.09 (s, 1H); 7.86 (dd, 1H); 7.79 (dd, 1H); 7.36 (td, 1H); 7.24 (dd, 2H); 7.24 (s, 1H); 6.81 (t, 2H); 4.6 (d, 1H); 4.48 (d, 1H); 2.91 (m, 2H); 2.80-2.65 (m, 4H), 2.55 (td, 1H); 2.27 (m, 1H); 2.15 (bd, 2H); 1.95-1.75 (m, 2H); 1.59 (m, 1H).

### Example 34

### 1-[(3,5-Dichlorophenyl)methyl]-3-[4-(4-fluorophenyl)-1-methyl-4-piperidinyl]-2-pyrrolidinone

37% wt Formaldehyde in water (8 µL) and NaBH(OAc)₃ (16 mg) were added to a solution of example **21** in CH3CN (3 mL). After stirring for 2 h the same amounts of 37% wt formaldehyde in water and NaBH(OAc)₃ were added and the mixture was stirred at r.t. overnight. Then it was diluted with DCM (2 ml), washed with an aqueous saturated sodium hydrogen carbonate solution and filtered on a phase separation cartridge. The filtered was concentrated *in vacuo* and the crude product was purified by flash chromatography (DCM/MeOH 4:6) to give the title compound (15 mg) as a colourless oil.
T.I.c.: DCM/MeOH 4:6, Rf=0.15 (detection with ninhydrine).
NMR (d₆-DMSO): □ (ppm) 7.43 (t, 1 H); 7.34 (dd, 2H); 7.03 (t, 2H); 6.95 (s, 2H); 4.28 (d, 1H); 4.02 (d, 1H); 2.9 (dd, 1 H); 2.69 (m, 1 H); 2.4 - 2.52 (m, 2H); 2.41 (dd, 1 H); 2.0 - 2.2 (m, 2H); 2.0 (s, 3H); 1.8 - 2.05 (m, 5H); 1.61 (m, 1H).
MS (ES/+): m/z=435 [M+H]⁺.

### Example 35

### 1-[(1S)-1-(3-chloro-1-naphthalenyl)ethyl]-3-[4-(4-fluorophenyl)-1-methyl-4-piperidinyl]-2-pyrrolidinone (Diastereoisomer 2)

A solution of formaldehyde in water (37% w/w; 8.5 µL) was added to a stirred solution of Example **23** (9.2 mg) in CH₃CN (5 mL) under Nitrogen atmosphere at r.t.. After 15 min NaBH(OAC)₃ (7 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (2.4 mg) as a white foam.
HPLC(LC/MS -ES /+):t_{R} = 4.6 min
MS (ES/+): m/z=465 [M+H]⁺

### Example 36

### 1-[1-(3,5-Dichlororophenyl)ethyl]-3-[4-(4-fluorophenyl)-1-methyl-4-piperidinyl]-2-pyrrolidinone (Diastereoisomer 1 Chain Enantiomer 1)

A solution of formaldehyde in water (37% w/w; 72 µL) was added to a stirred solution of Example **24** (78 mg) in CH₃CN (6 mL) under Nitrogen atmosphere at r.t.. After 15 min NaBH(OAc)₃ (57.2 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (80 mg) as a white foam.
HPLC(LC/MS -ES /+):t_{R} = 4.6 min
MS (ES/+): m/z=449 [M+H]⁺
NMR (CDCl₃): δ (ppm) 7.39 (dd, 2H); 7.26 (t, 1H); 7.09 (t, 2H); 7.07 (d, 2H); 5.26 (q, 1H); 2.92 (b, 2H); 2.67 (b, 1H); 2.63 (m, 1H); 2.1 - 2.6 (bm, 5H), 2.38 (s, 3H), 2.17 (bt, 1H), 1.99 (m, 1H); 1.79 (m, 1H); 1.5 -1.8 (b, 1H); 1.15 (d, 3H).

### Example 37

### 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 1)

A solution of formaldehyde in water (37% w/w; 80 µL) was added to a stirred solution of Example **25** (86.5 mg) in CH₃CN (8 mL) under Nitrogen atmosphere at r.t.. After 15 min NaBH(OAc)₃ (63.6 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (82 mg) as a white foam.
HPLC(LC/MS -ES /+):t_{R} = 4.6 min
MS (ES/+): m/z=449 [M+H]⁺
NMR (CDCl₃): δ (ppm) 7.36 (dd, 2H); 7.24 (t, 1H); 7.01 (t, 2H); 6.78 (d, 2H); 5.25 (q, 1H); 3.07 (dd, 1H); 2.98 (b, 1H); 2.76 (bt, 1H); 2.43 (dd, 1H), 2.1 - 2.6 (bm,.5H), 2.41 (s, 3H), 2.22 (bd, 1H), 2.05 (m, 1H); 1.68 (m, 1H); 1.62 (m, 1H); 1.4 (d, 3H).

### Example 38

### 1-[1-(3,5-Dichlorophenyl)ethyl]-3-[4-(4-fluorophenyl)-1-methyl-4-piperidinyl]-2-pyrrolidinone (Diastereoisomer 1 Chain Enantiomer 2)

A solution of formaldehyde in water (37% w/w; 72 µL) was added to a stirred solution of Example **26** (80 mg) in CH₃CN (6 mL) under Nitrogen atmosphere at r.t.. After 15 min NaBH(OAc)₃ (57 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (81 mg) as a white foam.
HPLC(LC/MS -ES /+):t_{R} = 4.6 min
MS (ES/+): m/z=449 [M+H]⁺
NMR (CDCl₃): δ (ppm) 7.39 (dd, 2H); 7.26 (t, 1H); 7.08 (t, 2H); 7.07 (d, 2H); 5.27 (q, 1H); 2.88 (b, 2H); 2.71 (b, 1H); 2.62 (m, 1H); 2.2 - 2.6 (bm,.6H); 2.36 (s, 3H); 2.14 (bt, 1H); 1.97 (m, 1H); 1.8 (m, 1H); 1.14 (d, 3H).

### Example 39

### 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 2)

A solution of formaldehyde in water (37% w/w; 90 µL) was added to a stirred solution of Example **27** (80 mg) in CH₃CN (8 mL) under Nitrogen atmosphere at r.t.. After 15 min NaBH(OAc)₃ (73.1 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (91 mg) as a white foam.
HPLC(LC/MS -ES /+):t_{R} = 4.6 min
MS (ES/+): m/z=449 [M+H]⁺
NMR (CDCl₃): δ (ppm) 7.36 (dd, 2H); 7.24 (t, 1H); 6.99 (t, 2H); 6.79 (d, 2H); 5.26 (q, 1H); 3.05 (dd, 1H); 2.83 (b, 1H); 2.75 (bt, 1 H); 2.39 (td, 1H); 2.25 - 2.5 (bm,.5H); 2.32 (s, 3H); 2.18 (b, 1H); 2.06 (m, 1H); 1.7 (m, 1H); 1.67 (m, 1 H); 1.4 (d, 3H).

### Example 40 e 41

### 1-[(3,5-Dichlorophenyl)methyl]-3-[4-(4-fluorophenyl)-1-methyl-4-piperidinyl]-2-pyrrolidinone (Enantiomer 1) 1-[(3,5-Dichlorophenyl)methyl]-3-[4-(4-fluorophenyl)-1-methyl-4-piperidinyl]-2-pyrrolidinoneEnantiomer 2):

A solution of formaldehyde in water (37% w/w; 170 µL) was added to a stirred solution of Example **21** (175 mg) in CH₃CN (10 mL) under Nitrogen atmosphere at r.t.. After 15 min NaBH(OAc)₃ (134 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave a white foam (167 mg) which was purified by semipreparative SFC (Gilson) chromatography [semipreparative conditions: chiral column: CHIRALCEL OD, 25 x 2.1 cm; modifier: (n-Hexane / Ethanol + 0.1% isopropylamine) 90/10% v/v; flow rate= 7.0 mL/min; UV wavelenfght: 220 nm; injection: 20 mg each injection] to obtain title compound **40** [analytical conditions:Chiral column: CHIRACEL OD,26 x 0.46 cm; modifier: (n-Hexane / Ethanol + 0.1 % isopropylamine) 90/10% v/v; flow rate= 1.0 mL/min; UV wavelenfght: 220 nm; retention time = 9.5 min] (81 mg) and title compound **41** [analytical conditions:Chiral column: CHIRACEL OD, 26 x 0.46 cm; modifier: (n-Hexane / Ethanol + 0.1% isopropylamine) 90/10% v/v; flow rate= 1.0 mL/min; UV wavelenfght 220 nm; retention time = 11.4 min] (81.5 mg).

### Example 40

NMR (CDCl₃): δ (ppm) 7.38 (dd, 2H); 7.26 (t, 1 H); 7.03 (t, 2H); 6.88 (dd, 2H); 4.29 (d, 1 H); 4.1 (d, 1 H); 2.92 (m, 1 H); 2.7 (bm, 2H); 2.43 (m, 1 H); 2.3 (bd,.1H); 2.23 (s, 3H); 2.06 (m, 1 H); 1.96 (bt, 1 H); 1.77 (m, 1H).

### Example 41

NMR (CDCl₃): δ (ppm) 7.38 (dd, 2H); 7.26 (t, 1 H); 7.03 (t, 2H); 6.88 (dd, 2H); 4.29 (d, 1 H); 4.1 (d, 1H); 2.92 (m, 1 H); 2.7 (bm, 2H); 2.43 (m, 1 H); 2.3 (bd,.1H); 2.23 (s, 3H); 2.06 (m, 1 H); 1.96 (bt, 1H); 1.77 (m, 1H).

### Example 42

### 4-({3-[4-(4-Fluorophenyl)-1-methyl-4-piperidinyl]-2-oxo-1-pyrrolidinyl}methyl)-2-naphthalenecarbonitrile (Enantiomer 1)

A solution of formaldehyde in water (37% w/w; 20 µL) was added to a stirred solution of Example **28** (20 mg) in CH₃CN (3 mL) under Nitrogen atmosphere at r.t.. After 15 min NaBH(OAc)₃ (15.9 mg) was added portionwise. The mixture was stirred for further 1 h and then it was concentrated *in vacuo.* The residue was purified on a SCX-cartridge (loaded with DCM, washed with MeOH, eluted with NH₃ 2 M in MeOH, followed by MeOH).
Solvent evaporation gave the title compound 19 mg) as a white foam.
NMR (CDCl₃); δ (ppm) 8.15 (d, 1H); 8.09 (d, 1H); 7.89 (dd, 1H); 7.65 (td, 1H); 7.61 (td, 1H); 7.29 (dd, 2H); 7.25 (s, 1H); 6.85 (td, 2H); 4.67 (d, 1H); 4.61 (d, 1H); 2.96 (b, 1H); 2.61 - 2.83 (bm, 3H); 2.4 (b, 1H); 1.92 - 2.34 (bm, 6H); 2.25 (s, 3H); 1.91 (m, 1H); 1.67 (m, 1H).

### Example 43

### 4-({3-[4-(4-Fluorophenyl)-1-methyl-4-piperidinyl]-2-oxo-1-pyrrolidinyl}methyl)-2-naphthalenecarbonitrile (Enantiomer 2)

A solution of formaldehyde in water (37% w/w; 16 µL) was added to a stirred solution of Example **29** (15 mg) in CH₃CN (3 mL) under Nitrogen atmosphere at r.t.. After 15 min NaBH(OAc)₃ (13 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH). Solvent evaporation gave the title compound (15 mg) as a white foam.
NMR (CDCl₃): δ (ppm) 8.15 (d, 1H); 8.05 (d, 1H); 7.89 (dd, 1H); 7.65 (td, 1H); 7.61 (td, 1 H); 7.29 (dd, 2H); 7.22 (s, 1H); 6.87 (td, 2H); 4.65 (d, 1H); 2.92(bt, 2H); 2.81 (bd, 1H); 2.7 (m, 1H); 2.20 - 2.60 (bm, 7H); 2.36 (s, 3H); 1.92 (m, 1H); 1.50 -1.80 (m, 1H).

### Example 44

### 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 2)

A solution of formaldehyde in water (37% w/w; 11 µL) was added to a stirred solution of Example **30** (12 mg) in CH₃CN (2 mL) under Nitrogen atmosphere at r.t. After 15 min NaBH(OAc)₃ (8.5 mg) was added portionwise. The mixture was stirred for further 1 h and then it was concentrated *in vacuo*. The residue was purified on a SCX-cartridge (loaded with DCM, washed with MeOH, eluted with NH₃ 2 M in MeOH, followed by MeOH).
Solvent evaporation gave the title compound (11 mg) as a white foam.
HPLC(LC/MS -ES /+):t_{R} = 3.99 min
MS (ES/+): m/z=460 [M+H]⁺
NMR (CDCl₃): δ (ppm) 8.22 (d, 1H); 8.13 (s, 1H); 7.55 (dd, 1H); 7.47 (ddd, 1H); 7.33 (dd, 2H); 7.26 (d, 1H); 6.9 (t, 2H); 4.75 (d, 1H); 4.57 (d, 1H); 2.81 (m, 1H); 2.69 (b, 3H); 2.45-2.15 (bm, 5H); 2.22 (s, 3H); 2.15-1.9 (bm, 3H); 1.71 (m, 1H).

### Example 45

### 6-Fluoro-4-({3-[4-(4-fluorophenyl)-1-methyl-4-piperidinyl]-2-oxo-1-pyrrolidinyl}methyl)-2-naphthalenecarbonitrile (Enantiomer 2)

A solution of formaldehyde in water (37% w/w; 20 µL) was added to a stirred solution of Example **31** (8 mg) in CH₃CN (2 mL) under Nitrogen atmosphere at r.t.. After 15 min NaBH(OAc)₃ (6 mg) was added portionwise. The mixture was stirred for further 1 h and then it was concentrated *in vacuo.* The residue was purified on a SCX-cartridge (loaded with DCM, washed with MeOH, eluted with NH₃ 2 M in MeOH, followed by MeOH). Solvent evaporation gave the title compound (7 mg) as a white foam.
HPLC(LC/MS -ES /+):t_{R} = 3.99 min
MS (ES/+): m/z=460 [M+H]⁺
NMR (CDCl₃): δ (ppm) 8.18 (s, 1H); 7.95 (dd, 1H); 7.88 (dd, 1H); 7.45 (ddd, 1H); 7.34 (dd, 2H); 7.31 (s, 1H); 6.89 (t, 2H); 4.65 (d, 1H); 4.58 (d, 1H); 2.86 (m, 1 H); 2.8-2.6 (b, 3H); 2.45 - 2.25 (m, 5H); 2.23 (s, 3H); 1.97 (m, 3H); 1.67 (m, 1H).

### Example 46

### 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 1)

A solution of formaldehyde in water (37% w/w; 22 µL) was added to a stirred solution of Example **32** (24 mg) in CH₃CN (2.5 mL) under Nitrogen atmosphere at r.t.. After 15 min NaBH(OAc)₃ (17 mg) was added portionwise. The mixture was stirred for further 1 h and then it was concentrated *in vacuo.* The residue was purified on a SCX-cartridge (loaded with DCM, washed with MeOH, eluted with NH₃ 2 M in MeOH, followed by MeOH). Solvent evaporation gave the title compound (21 mg) as a white foam.
HPLC(LC/MS -ES /+):t_{R} = 3.99 min
MS (ES/+): m/z=460 [M+H]⁺
NMR (CDCl₃): δ (ppm) 8.21 (dd, 1H); 8.13 (s, 1H); 7.55 (dd, 1H); 7.47 (m, 1H); 733 (dd, 2H); 7.26 (d, 1H); 6.9 (t, 2H); 4.74 (d, 1H); 4.57 (d, 1H); 2.9 (b, 1H); 2.81 (m, 1H); 2.7 (bt, 4H); 2.45-2.2 (m, 3H); 2.23 (s, 3H); 2.1-1.9 (m, 3H); 1.72 (m, 1H).

### Example 47

### 6-Fluoro-4-({3-[4-(4-fluorophenyl)-1-methyl-4-piperidinyl]-2-oxo-1-pyrrolidinyl}methyl)-2-naphthalenecarbonitrile (Enantiomer 1)

A solution of formaldehyde in water (37% w/w; 15 µL) was added to a stirred solution of Example **33** (10 mg) in CH₃CN (2.5 mL) under Nitrogen atmosphere at r.t.. After 15 min NaBH(OAc)₃ (8 mg) was added portionwise. The mixture was stirred for further 1 h and then it was concentrated *in vacuo.* The residue was purified on a SCX-cartridge (loaded with DCM, washed with MeOH, eluted with NH₃ 2 M in MeOH, followed by MeOH). Solvent evaporation gave the title compound (8 mg) as a white foam.
HPLC(LC/MS -ES/+):t_{R} = 3.96 min
MS (ES/+): m/z=460 [M+H]⁺
NMR (CDCl₃): δ (ppm) 8.19 (s, 1H); 7.95 (dd, 1H); 7.87 (dd, 1H); 7.45 (m, 1H); 7.35 (dd, 2H); 7.32 (s, 1H); 6.9 (t, 2H); 4.65 (d, 1H); 4.58 (d, 1H); 3.00-2.60 (bt, 3H); 2.5-2.15 (bm, 5H); 2.24 (s, 3H); 2.09 (b, 1H); 1.98 (m, 2H); 1.69 (m, 2H).

### Example 48

### 1-[(3,5-Dichlorophenyl)methyl]-3-[4-(4-fluorophenyl)-1-methyl-4-piperidinyl]-1H-pyrrole-2,5-dione

Intermediate **63** (92g ) was taken up with MeOH (100 mL). After complete dissolution aqueous formaldehyde 37%(21mL) was added and the mixture was stirred for 10 min at 25°C. Sodium triacetoxyborohydride (60g) was added in 10 portions over 30 min. After 40 min the reaction mixture was concentrated *in vacuo* (P=250 mBar, internal temperature 37-40°C). Aqueous std NaHCO₃ (500mL) was added to reach pH 8. AcOEt (500mL) was added and the phases were separated. The aqueous phase was extracted with AcOEt (500 mL). The combined organic phases were washed with saturated brine/ water (1/1). The solution is concentrated up to 300 mL, then 450 ml of MeOH were added and the solution is concentrated to obtain an oil. A chromatographic column (DCM/MeOH 9/1)was needed to isolate the 5g of the title compound.
NMR (DMSO): δ (ppm) 7.46 (t, 1H), 7.41 (dd, 2H), 7.11 (m, 4H), 6.93 (s, 1H), 4.49 (s, 2H), 2.5-2.1 (bm, 8H), 2.10 (s, 3H)

### Example 49

### 1-[(3,5-Dichlorophenyl)methyl]-3-[4-(4-fluorophenyl)-1-methyl-4-piperidinyl]-5-methylidene-1,5-dihydro-2H-pyrrol-2-one

Example **3** (5g) was taken up with CH3CN (50mL) and 1.25 ml of aqueous formaldehyde 37% was added. After 10 min at 25°C aqueous NaOH 3M solution (10 mL) was added and the mixture was stirred at 40°C for 4hrs. The reaction was followed by HPLC. The reaction mixture was cooled and filtered to give 0.6g of the title compound.
NMR (CDCl₃): δ (ppm) 7.45 (t, 1H), 7.37 (dd, 2H), 7.18 (bs, 1H), 7.09 (t, 2H), 7.02 (d, 2H), 5.03 (d, 1H), 4.93 (d, 1H), 4.72 (s, 2H), 2.59 (bm, 2H), 2.49 (bm, 2H), 2.19 (bm, 2H), 2.10 (bm+s, 5H)

### Example 50

### 1-[(3,5-Dichlorophenyl)methyl]-3-[4-(4-fluorophenyl)-1-methyl-4-piperidinyl]-1,5-dihydro-2H-pyrrol-2-one fumarate salt

Example 3 (250mg) was dissolved in MeOH (2.5 mL) at r.t. under Nitrogen atmosphere. The clear solution was seeded and fumaric acid (67 mg) was added. Solid crystallization was observed. The slurry was stirred 16 h at r.t. The solid was filtered and dried at r.t. in vacuo to give the title compound (227 mg).
NMR :(d₆-DMSO) δ(ppm) 7.51 (t, 1H); 7.41 (dd, 2H); 7.13 (t, 2H); 7.13 (m, 2H); 7.13 (m, 1 H); 6.55 (s, 2H); 4.50 (s, 2H); 3.96 (s, 2H); 2.78 (bm, 2H); 2.70 (bm, 2H); 2.62 (bm, 2H); 2.24 (bm, 2H); 2.38 (s, 3H).
m.p. (by DSC): 205.8°C

**Table 1**

| **The X-ray powder diffraction pattern of the product of Example 50 in terms of 'd' spacings is as follows** | |
|---|---|
| **Two theta (deg)** | **d-spacing (Angstroms)** |
| 4,2 | 21,1 |
| 9,2 | 9,6 |
| 11,4 | 7,8 |
| 12,1 | 7,3 |
| 12,5 | 7,1 |
| 14,0 | 6,3 |
| 16,1 | 5,5 |
| 16,3 | 5,4 |
| 16,7 | 5,3 |
| 17,0 | 5,2 |
| 17,3 | 5,1 |
| 17,9 | 5,0 |
| 18,2 | 4,9 |
| 18,6 | 4,8 |
| 19,0 | 4,7 |
| 20,1 | 4,4 |
| 21,0 | 4,2 |
| 21,2 | 4,2 |
| 23,2 | 3,8 |
| 23,6 | 3,8 |
| 23,8 | 3,7 |
| 24,4 | 3,6 |
| 25,2 | 3,5 |
| 26,7 | 3,3 |
| 27,8 | 3,2 |
| 28,5 | 3,1 |
| 28,7 | 3,1 |
| 29,5 | 3,0 |
| 30,4 | 2,9 |
| 33,8 | 2,7 |
| 34,7 | 2,6 |
| 35,9 | 2,5 |
| 38,1 | 2,4 |

### Example 51

### 1-[(3,5-Dichlorophenyl)methyl]-3-[4-(4-fluorophenyl)-1-methyl-4-piperidinyl]-1,5-dihydro-2H-pyrrol-2-one citrate salt (Crystalline form 1)

### Method A

Example **3** (200mg) was dissolved in tetrahydrofuran (2 mL) at r.t. under Nitrogen atmosphere. The clear solution was seeded and a solution of citric acid (88 mg) in MeOH (0.5 mL) was dosed. Solid crystallization was observed. The slurry was stirred 16 h at r.t.. The solid was filtered and dried at r.t. in vacuo to give the title compound (200 mg).NMR (d₆-DMSO) δ(ppm) 11.6-10.4 (b, 3H); 7.52 (t, 1H); 7.42 (dd, 2H); 7.17 (t, 2H); 7.16 (m, 2H); 7.16 (m, 1H); 4.51 (s, 2H); 3.96 (s, 2H); 3.06-2.97 (bm, 4H); 2.82 (bm, 2H); 2.29 (bm, 2H); 2.65 (s, 3H); 2.60 (d, 2H); 2.52 (d, 2H) m.p. (by DSC): 156.6 °C

### Method B

Example 1 (5Kg) was taken up with 100 mL of MeOH (25L). After complete dissolution aqueous formaldehyde (1.15L,) was added and the mixture was stirred for 10 min at 25°C. Sodium triacetoxyborohydride (3.3kg) was added in 10 portions over 30 min. After 40 min the reaction was concentrated under vacuum (P=250 mBar, internal temperature 37-40°C) to 15L Aq. saturated NaHCO₃ (30L) was added to reach pH 8 (pH paper). AcOEt (25L) was added and the phases were separated. The aqueous phase was extracted with AcOEt (25L). The combined org. phases were washed with saturated brine/distilled water (1/1; 10L). The solution was concentrated up to 15L, then MeOH (25L) were added and the solution contain ning example 3 was concentrated to 25L. and Citric acid (2.285g) was added in one portion. The solution was stirred for 15 min (until complete dissolution of the solid) and Butanone (50L) was added in 1.5h. Some seeds were added and the solution was stirred for 18h. The suspension is concentrated up to 30L and stirred for 4h. The solid was filtered and washed with MeOH/2-Butanone 1/1 (10 I) to obtain the title compound (2.5Kg).
Melting point (by DSC): 161°C.
NMR (d₆-DMSO) δ(ppm) 11.6-11.4 ppm (broad, 3H), 7.49 ppm (t, 1 H), 7.40 ppm (m, 2H), 7.14 ppm (m, 5H), 4.49 ppm (s, 2H), 3.95 ppm (s, 2H), 3.1-2.9 ppm (bm, 4H), 2.8 ppm (bm, 2H), 2.65 ppm (s, 3H), 2.58 ppm (d, 2H), 2.52 ppm (d, 2H), 2.30 ppm (bm, 2H)

**Table 2**

| **The X-ray powder diffraction pattern of the product of Example 51 in terms of 'd' spacings is as follows** | | | |
|---|---|---|---|
| **Two theta (deg)** | **d-spacing (Angstroms)** | **Two theta (deg)** | **d-spacing (Angstrom s)** |
| 7,1 | 12,5 | 24,0 | 3,7 |
| 10,6 | 8,4 | 24,9 | 3,6 |
| 11,6 | 7,7 | 25,5 | 3,5 |
| 11,9 | 7,4 | 26,4 | 3,4 |
| 14,0 | 6,3 | 28,1 | 3,2 |
| 14,5 | 6,1 | 29,1 | 3,1 |
| 16,0 | 5,5 | 29,7 | 3,0 |
| 16,8 | 5,3 | 32,9 | 2,7 |
| 17,6 | 5,0 | | |
| 18,5 | 4,8 | | |
| 19,5 | 4,6 | | |
| 19,9 | 4,5 | | |
| 20,6 | 4,3 | | |
| 21,2 | 4,2 | | |
| 21,8 | 4,1 | | |
| 22,4 | 4,0 | | |
| 23,1 | 3,9 | | |
| 23,6 | 3,8 | | |

### Example 52

### 1-[(3,5-Dichlorophenyl)methyl]-3-[4-(4-fluorophenyl)-1-methyl-4-piperidinyl]-1,5-dihydro-2H-pyrrol-2-one citrate salt (Crystalline form 2)

Example 3 (25g) was dissolved in tetrahydrofuran (200ml) at room temperature under Nitrogen. The clear solution was seeded and a solution of citric acid (11g) in methanol (50ml) was dosed. Isooctane was added dropwise 125ml and left stirring overnight. Then additional 50ml of isooctane were added and stirred for 2 hours. The solid was filtered very slowly and dried into oven at 40deg to give 30.5g of **the title compound**.

Melting point (by DSC): 137°C.
NMR (d₆-DMSO) δ(ppm) 11.6-11.4 ppm (broad, 3H), 7.49 ppm (t, 1H), 7.40 ppm (m, 2H), 7.14 ppm (m, 5H), 4.49 ppm (s, 2H), 3.95 ppm (s, 2H), 3.1-2.9 ppm (bm, 4H), 2.8 ppm (bm, 2H), 2.65 ppm (s, 3H), 2.58 ppm (d, 2H), 2.52 ppm (d, 2H), 2.30 ppm (bm, 2H)

**Table 3**

| **The X-ray powder diffraction pattern of the product of Example 52 in terms of 'd' spacings is as follows** | |
|---|---|
| d spacing | Two Theta |
| Angstroms | (deg) |
| 7,7 | 11,5 |
| 7,2 | 12,2 |
| 5,5 | 16,1 |
| 5,3 | 16,7 |
| 5,0 | 17,6 |
| 4,8 | 18,6 |
| 4,6 | 19,4 |
| 4,2 | 21,1 |
| 3,9 | 23,1 |
| 3,8 | 23,6 |
| 3,6 | 24,5 |

### Example 53

### 1-[(3,5-Dichlorophenyl)methyl]-3-[4-(4-fluorophenyl)-1-methyl-4-piperidinyl]-1,5-dihydro-2H-pyrrol-2-onecitrate salt (Crystalline hydrate form)

Example 52 (0.200g) was suspended and stirred in water (2ml) at room temperature. The slurry was heated to 50°C. The solution was cooled down to 20°C. Solid precipitation occurred. The slurry was stirred 16 hrs at 20°C. The solid was filtered and dried at room temperature under vacuum to give the title compound (0.172g).

Melting point (by DSC): 100.29°C
NMR (s2364): (DMSO) δ (ppm) 11.8-10.2 (b, 3H), 7.48 (m, 1H), 7.39 (m, 2H), 7.14 (m, 2H), 7.13 (m, 3H), 4.48 (s, 2H), 3.93 (s, 2H), 3.6-2.1 (bm, 8H), 2.62 (s, 3H), 2.53(d, 2H), 2.49 (d, 2H)

**Table 4**

| **The X-ray powder diffraction pattern of the product of Example 53 in terms of 'd' spacings is as follows** | |
|---|---|
| d spacing | Two Theta |
| Angstroms | (deg) |
| 9,3 | 9,5 |
| 8,0 | 11,0 |
| 6,5 | 13,5 |
| 6,3 | 14,0 |
| 6,1 | 14,5 |
| 6,0 | 14,7 |
| 5,7 | 15,5 |
| 5,6 | 15,9 |
| 5,4 | 16,4 |
| 5,3 | 16,7 |
| 5,1 | 17,4 |
| 4,9 | 18,2 |
| 4,8 | 18,4 |
| 4,6 | 19,1 |
| 4,5 | 19,6 |
| 4,4 | 20,2 |
| 4,3 | 20,4 |
| 4,0 | 22,1 |
| 4,0 | 22,3 |
| 3,8 | 23,2 |
| 3,7 | 23,9 |
| 3,6 | 24,5 |
| 3,5 | 25,1 |
| 3,5 | 25,8 |
| 3,3 | 26,6 |
| 3,2 | 27,5 |
| 3,2 | 27,9 |
| 3,1 | 28,3 |
| 3,0 | 29,3 |
| 3,0 | 29,7 |
| 2,9 | 31,0 |
| 2,8 | 31,5 |
| 2,8 | 32,5 |
| 2,7 | 33,4 |
| 2,6 | 34,9 |
| 2,5 | 35,2 |
| 2,5 | 35,4 |
| 2,5 | 36,0 |
| 2,5 | 36,6 |
| 2,3 | 39,7 |
| 2,2 | 41,2 |
| 2,1 | 43,4 |

## Claims

1. A compound of formula (I) 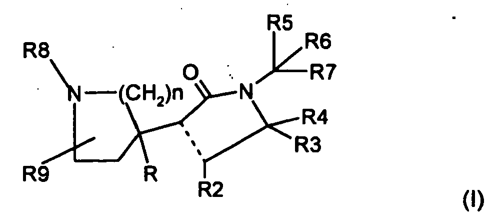 wherein
• ---- represents a single or a double bond;
• R represents a radical selected from: 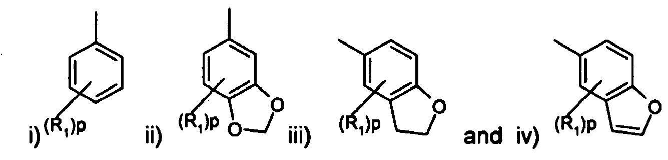 in which R₁ is halogen, cyano, C₁₋₄ alkyl, C₁₋₄ alkoxy, trifluoromethyl or trifluoromethoxy and p is zero or an integer from 1 to 3;
• R₂ represents hydrogen or C₁₋₄ alkyl;
• R₃ represents hydrogen, hydroxy or C₁₋₄ alkyl;
• R₄ represents hydrogen or R₄ together with R₃ represents =O or =CH₂;
• R₅ represents phenyl, naphthyl, a 9 to 10 membered fused bicyclic heterocyclic group or a 5 or 6 membered heteroaryl group, wherein said groups are optionally substituted by 1 to 3 groups independently selected from trifluoromethyl, C₁₋₄ alkyl, hydroxy, cyano, C₁₋₄ alkoxy, trifluoromethoxy, halogen or S(O)_{q}C₁₋₄ alkyl;
• R₆ and R₇ independently represent hydrogen, cyano, C₁₋₄ alkyl;
• R₈ is (CH₂)ᵣR₁₀;
• Rg represents hydrogen, halogen, C₃₋₇ cycloalkyl, hydroxy, nitro, cyano or C₁₋₄ alkyl optionally substituted by one or two groups selected from halogen, cyano, hydroxy or C₁₋₄ alkoxy;
• R₁₀ represents hydrogen or C₃₋₇ cycloalkyl;
• n represents 1 or 2;
• q is 0, 1 or 2;
• r is 0 or an integer from 1 to 4;
or a pharmaceutically acceptable salt or a solvate thereof.

2. A compound as claimed in claim 1 wherein n is 2.

3. A compound as claimed in claim 1 or claim 2 wherein R represents: 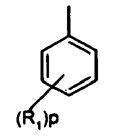 in which R₁ is halogen, C₁₋₄ alkyl, cyano, C₁₋₄ alkoxy, trifluoromethyl or trifluoromethoxy and p is zero or an integer from 1 to 3.

4. A compound as claimed in any of claims 1 to 3 wherein R₅ is phenyl or naphthyl optionally substituted by one or two groups selected from trifluoromethyl, cyano, C₁₋₄ alkyl or halogen.

5. A compound as claimed in any of claims 1 to 4 wherein R₈ is (CH₂)ᵣR₁₀ in which R₁₀ is hydrogen or C₃₋₇ cycloalkyl (e.g cyclopropyl) and r is 0 or 1.

6. A compound as claimed in any of claims 1 to 5, wherein Rg is hydrogen or C₁₋₄ alkyl optionally substituted by one or two groups selected from halogen.

7. A compound as claimed in any of claims 1 to 6 wherein R is phenyl substituted by a fluorine, R₂, R₉ and R₄ are hydrogen, R₃ is hydrogen, hydroxy or methyl, or together with R₄ forms =O or =CH₂, R₆ and R₇ are independently hydrogen or methyl, R₅ is phenyl or naphthyl optionally substituted by one or two groups independently selected from cyano, methyl, chlorine, bromine or fluorine atom, R₈ is hydrogen, methyl or cyclopropylmethyl, and n is 2.

8. A compound as claimed in claim 1 which is
• 1-[(3,5-Dichlorophenyl)methyl]-3-[4-(4-fluorophenyl)-4-piperidinyl]-1,5-dihydro-2*H-*pyrrol-2-one;
• 1-[(3,5-Dichlorophenyl)methyl]-3-[4-(4-fluorophenyl)-1-methyl-4-piperidinyl]-1,5-dihydro-2*H*-pyrrol-2-one ;
• 1-[1-(3,5-Dichlorophenyl)ethyl]-3-[4-(4-fluorophenyl)-4-piperidinyl]-1,5-dihydro-2*H-*pyrrol-2-one (Chain Enantiomer 1);
• 1-[(1*S*)-1-(3-Chloro-1-naphthalenyl)ethyl]-3-[4-(4-fluorophenyl)-4-piperidinyl]-1,5-dihydro-2H-pyrrol-2-one ;
• 1-[(3-Chloro-1-naphthalenyl)methyl]-3-[4-(4-fluorophenyl)-4-piperidinyl]-1,5-dihydro-2*H*-pyrrol-2-one;
• 4-({3-[4-(4-Fluorophenyl)-4-piperidinyl]-2-oxo-2,5-dihydro-1*H*-pyrrol-1-yl}methyl)-2-naphthalenecarbonitrile;
• 1-[1-(3,5-Dichlorophenyl)ethyl]-3-[4-(4-fluorophenyl)-4-piperidinyl]-1,5-dihydro-2*H-*pyrrol-2-one (Chain Enantiomer 2);
• 1-[(1*R*)-1-(3-Chloro-1-naphthalenyl)ethyl]-3-[4-(4-fluorophenyl)-4-piperidinyl]-1,5-dihydro-2*H*-pyrrol-2-one;
• 1-[1-(3,5-Dichlorophenyl)ethyl]-3-[4-(4-fluorophenyl)-1-methyl-4-piperidinyl]-1,5-dihydro-2*H*-pyrrol-2-one (Chain Enantiomer 1);
• 1-[(1*S*)-1-(3-Chloro-1-naphthalenyl)ethyl]-3-[4-(4-fluorophenyl)-1-methyl-4-piperidinyl]-1,5-dihydro-2*H*-pyrrol-2-one ;
• 1-[(3-Chloro-1-naphthalenyl)methyl]-3-[4-(4-fluorophenyl)-1-methyl-4-piperidinyl]-1,5-dihydro-2*H*-pyrrol-2-one;
• 1-[1-(3,5-Dichlorophenyl)ethyl]-3-[4-(4-fluorophenyl)-1-methyl-4-piperidinyl]-1,5-dihydro-2*H*-pyrrol-2-one (Chain Enantiomer 2);
• 1-[(1*R*)-1-(3-Chloro-1-naphthalenyl)ethyl]-3-[4-(4-fluorophenyl)-1-methyl-4-pipehdinyl]-1,5-dihydro-2H-pyrrol-2-one;
• 4-({3-[4-(4-Fluorophenyl)-1-methyl-4-piperidinyl]-2-oxo-2,5-dihydro-1H-pyrrol-1-yl}methyl)-2-naphthalenecarbonitrile;
• 1-[1-(3,5-Dichlorophenyl)ethyl]-3-[4-(4-fluorophenyl)-1-methyl-4-piperidinyl]-1,5-dihydro-2*H*-pyrrol-2-one hydrochloride (Chain Enantiomer 1);
• 1-[(3-Chloro-1-naphthalenyl)methyl]-3-[1-(cyclopropylmethyl)-4-(4-fluorophenyl)-4-piperidinyl]-1,5-dihydro-2H-pyrrol-2-one;
• 1-[(3,5-Dichlorophenyl)methyl]-3-[4-(4-fluorophenyl)-4-piperidinyl]-2-pyrrolidinone 1-[(1*S*)-1-(3-Chloro-1-naphthalenyl)ethyl]-3-[4-(4-fluorophenyl)-4-piperidinyl]-2-pyrrolidinone (Diastereoisomer 1);
• 1-[(1*S*)-1-(3-Chloro-1-naphthalenyl)ethyl]-3-[4-(4-fluorophenyl)-4-piperidinyl]-2-pyrrolidinone;
• 1-[1-(3,5-Dichlorophenyl)ethyl]-3-[4-(4-fluorophenyl)-4-piperidinyl]-2-pyrrolidinone (Diastereoisomer 1 Chain Enantiomer 1);
• 1-[1-(3,5-Dichlorophenyl)ethyl]-3-[4-(4-fluorophenyl)-4-piperidinyl]-2-pyrrolidinone (Diastereoisomer 2 Chain Enantiomer 1);
• 1-[1-(3,5-Dichlorophenyl)ethyl]-3-[4-(4-fluorophenyl)-4-piperidinyl]-2-pyrrolidinone (Diastereoisomer 1 Chain Enantiomer 2);
• 1-[1-(3,5-Dichlorophenyl)ethyl]-3-[4-(4-fluorophenyl)-4-piperidinyl]-2-pyrrolidinone (Diastereoisomer 2 Chain Enantiomer 2);
• 4-({3-[4-(4-Fluorophenyl)-4-piperidinyl]-2-oxo-1-pyrrolidinyl}methyl)-2-naphthalenecarbonitrile (Enantiomer 1);
• 4-({3-[4-(4-Fluorophenyl)-4-piperidinyl]-2-oxo-1-pyrrolidinyl}methyl)-2-naphthalenecarbonitrile (Enantiomer 2);
• 7-Fluoro-4-({3-[4-(4-fluorophenyl)-4-piperidinyl]-2-oxo-1-pyrrolidinyl}methyl)-2-naphthalenecarbonitrile (Enantiomer 2);
• 6-Fluoro-4-({3-[4-(4-fluorophenyl)-4-piperidinyl]-2-oxo-1-pyrrolidinyl}methyl)-2-naphthalenecarbonitrile (Enantiomer 2);
• 7-Fluoro-4-({3-[4-(4-fluorophenyl)-4-piperidinyl]-2-oxo-1-pyrrolidinyl}methyl)-2-naphthalenecarbonitrile (Enantiomer 1);
• 6-Fluoro-4-({3-[4-(4-fluorophenyl)-4-piperidinyl]-2-oxo-1-pyrrolidinyl}methyl)-2-naphthalenecarbonitrile (Enantiomer 1);
• 1-[(3,5-Dichlorophenyl)methyl]-3-[4-(4-fluorophenyl)-1-methyl-4-piperidinyl]-2-pyrrolidinone;
• 1-[1-(3-Chloro-1-naphthalenyl)ethyl]-3-[4-(4-fluorophenyl)-1-methyl-4-piperidinyl]-2-pyrrolidinone (Diastereoisomer 2 Chain Enantiomer 1);
• 1-[1-(3,5-Dichlorophenyl)ethyl]-3-[4-(4-fluorophenyl)-1-methyl-4-piperidinyl]-2-pyrrolidinone (Diastereoisomer 1 Chain Enantiomer 1);
• 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 1);
• 1-[1-(3,5-Dichlorophenyl)ethyl]-3-[4-(4-fluorophenyl)-1-methyl-4-piperidinyl]-2-pyrrolidinone (Diastereoisomer 1 Chain Enantiomer 2);
• 1-[1-(3,5-Dichlorophenyl)ethyl]-3-[4-(4-fluorophenyl)-1-methyl-4-piperidinyl]-2-pyrrolidinone (Diastereoisomer 2 Chain Enantiomer 2);
• 1-[(3,5-Dichlorophenyl)methyl]-3-[4-(4-fluorophenyl)-1-methyl-4-piperidinyl]-2-pyrrolidinone (Enantiomer 1);
• 1-[(3,5-Dichlorophenyl)methyl]-3-[4-(4-fluorophenyl)-1-methyl-4-piperidinyl]-2-pyrrolidinone (Enantiomer 2);
• 4-({3-[4-(4-Fluorophenyl)-1-methyl-4-piperidinyl]-2-oxo-1-pyrrolidinyl}methyl)-2-naphthalenecarbonitrile (Enantiomer 1);
• 4-({3-[4-(4-Fluorophenyl)-1-methyl-4-piperidinyl]-2-oxo-1-pyrrolidinyl}methyl)-2-naphthalenecarbonitrile (Enantiomer 2);
• 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 2);
• 6-Fluoro-4-({3-[4-(4-fluorophenyl)-1-methyl-4-piperidinyl]-2-oxo-1-pyrrolidinyl}methyl)-2-naphthalenecarbonitrile;
• 7-Fluoro-4-({3-[4-(4-fluorophenyl)-1-methyl-4-piperidinyl]-2-oxo-1-pyrrolidinyl}methyl)-2-naphthalenecarbonitrile (Enantiomer 1);
• 6-Fluoro-4-({3-[4-(4-fluorophenyl)-1-methyl-4-piperidinyl]-2-oxo-1-pyrrolidinyl}methyl)-2-naphthalenecarbonitrile (Enantiomer 1);
• 1-[(3,5-Dichlorophenyl)methyl]-3-[4-(4-fluorophenyl)-1-methyl-4-piperidinyl]-1*H-*pyrrole-2,5-dione;
• 1-[(3,5-Dichlorophenyl)methyl]-3-[4-(4-fluorophenyl)-1-methyl-4-piperidinyl]-5-methylidene-1,5-dihydro-2*H*-pyrrol-2-one;
or a phamaceutically acceptable salt (e.g hydrochloride, fumarate or citrate) or a solvate or amorphous or crystalline forms thereof.

9. A compound as claimed in claim 1 which is
1-[(3,5-Dichlorophenyl)methyl]-3-[4-(4-fluorophenyl)-1-methyl-4-piperidinyl]-1,5-dihydro-2*H*-pyrrol-2-one or a phamaceutically acceptable salt;

10. A compound as claimed in claim 1 which is
1-[(3,5-Dichlorophenyl)methyl]-3-[4-(4-fluorophenyl)-1-methyl-4-piperidinyl]-1,5-dihydro-2H-pyrrol-2-one hydrochloride or crystalline forms thereof.

11. A compound as claimed in claim 1 which is
1-[(3,5-Dichlorophenyl)methyl]-3-[4-(4-fluorophenyl)-1-methyl-4-piperidinyl]-1,5-dihydro-2H-pyrrol-2-one fumarate or crystalline forms thereof.

12. A compound as claimed in claim 1 which is
1-[(3,5-Dichlorophenyl)methyl]-3-[4-(4-fluorophenyl)-1-methyl-4-piperidinyl]-1,5-dihydro-2*H*-pyrrol-2-one citrate or crystalline forms thereof.

13. A compound as claimed in claim 1 which is
1-[(3,5-Dichlorophenyl)methyl]-3-[4-(4-fluorophenyl)-1-methyl-4-piperidinyl]-1,5-dihydro-2*H*-pyrrol-2-one citrate (crystalline form 1)

14. A process for the preparation of a compound as claimed in claim 1 which process comprises:
a) cyclisation of a compound of formula (IV), wherein R₁₁ is C₁₋₄ alkyl (e.g methyl 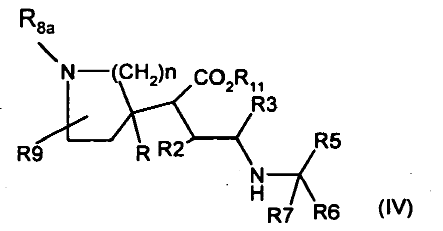 or ethyl), R₃ is hydrogen or C₁₋₄ alkyl, R₈ₐ has the meaning defined in formula (I) or is a nitrogen protecting group and R, R₂, R₅, R₆, R₇,R₉ and n are as defined in claim 1, to yield a compound of formula(I), wherein ---- is a single bond R₃ represents hydrogen or C₁₋₄ alkyl and R₄ represents hydrogen, or
b) cyclisation of a compound of formula (VI), wherein R₈ₐ has the 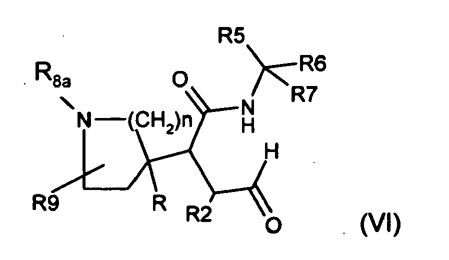 meaning defined in formula (I) or is a nitrogen protecting group, R, R₂, R₅, R₆, R₇,R₉ and n are as defined in claim 1, to yield a compound of formula(I) wherein ----is a single bond, R₃ is hydroxy and R₄ is hydrogen, or
c) cyclisation of a compound of formula (VII), wherein R₈ₐ has the meaning defined in formula (I) or is a nitrogen protecting group, L is a leaving group and R, R₂, R₅, R₆,R₇,R₉ and n are as defined in claim 1, 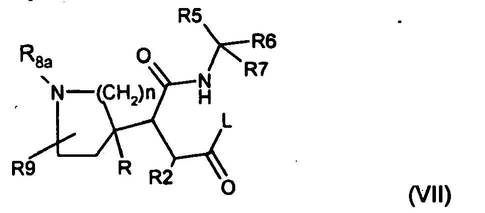 to yield a compound of formula(I) wherein ---- is a single bond and R₃ together with R₄ represents =O, or
d) reaction of a compound of formula(VIIA), wherein R, R₂, R₅, R₆,R₇,R₉ and n are as defined in claim 1, 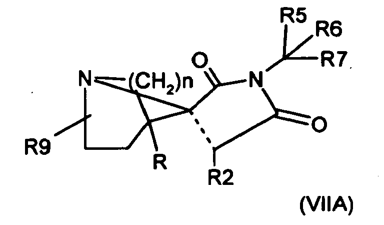 with an aldehyde, CH(O)(CH₂)ₘR₁₀ (VIIIa), wherein m is an integer from 0 to 3 and R₁₀ is as defined in claim 1, to yield a compound of formula (I), wherein ---- is a single bond, R₈ represents (CH₂)rR₁₀, wherein r is an integer from 1 to 4 and R₃ together with R₄ represents =O, or
e) cyclisation in the presence of an acid of a compound of formula (VIa), wherein 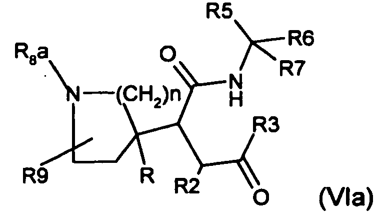 R₃ is hydrogen, R₈ₐ has the meaning defined in formula (I) or is a nitrogen protecting group, R, R₂, R₅, R₆, R₇,R₉ and n are as defined in claim 1, to yield compounds of formula (I), wherein ----is a double bond, R₃ represents hydrogen or C₁₋₄ alkyl and R₄ is hydrogen; or
f) N-alkylation of a compound of formula (VIII), wherein R₈ₐ has the meaning defined in formula (I) or is a nitrogen protecting group and R, R₂, R₃, R₄,R₉ and n are as defined in claim 1, with a compound of formula (IX) 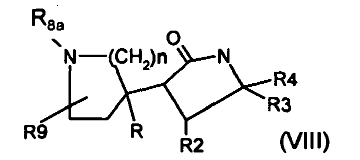
L-C(R₅)(R₆)(R₇) (IX)
in which L is a leaving group, R₅,R₆ and R₇ are as defined in claim 1, to yield a compound of formula(I) wherein ----is a single bond,
and thereafter optionally for any of steps (a) to (f):
• removing any protecting groups and/or
• converting a compound of formula (I) into another compound of formula (I) and/or
• separation of a compound of formula(I) or a derivative thereof into the enantiomers thereof
• forming a pharmaceutically acceptable salt.

15. A compound as claimed in any of claims 1 to 13 for use in therapy.

16. The use of a compound as claimed in any claims of 1 to 13 in the preparation of a medicament for use in the treatment of conditions mediated by tachykinins (including substance P and other neurokinins) and/or by selective inhibition of the serotonin reuptake transporter protein.

17. The use of a compound as claimed in any of claims 1 to 13 in the preparation of a medicament for use in the treatment of depression and/or anxiety.

18. A pharmaceutical composition comprising a compound as claimed in any of claims 1 to 13 in admixture with one or more pharmaceutically acceptable carriers or excipients.

## Patentansprüche

1. Verbindung der Formel (I) 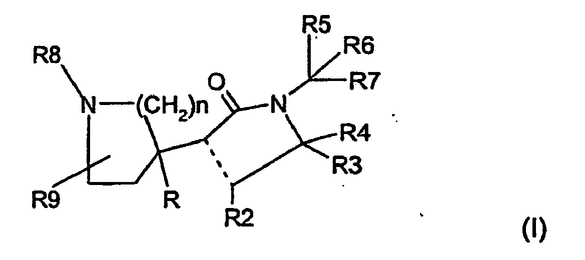 wobei
• --- eine Einfach- oder eine Doppelbindung darstellt;
• R ein Rest, ausgewählt aus 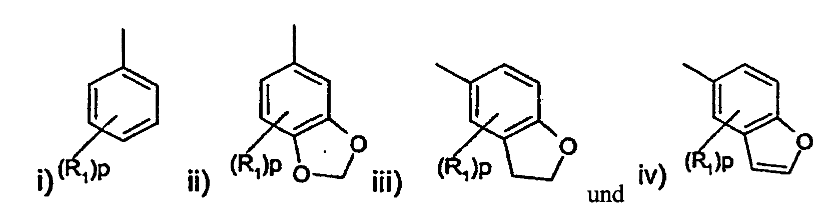 darstellt, wobei R₁ Halogen, Cyano, C₁₋₄-Alkyl, C₁₋₄-Alkoxy, Trifluormethyl oder Trifluormethoxy ist und p.0 oder eine ganze Zahl von 1 bis 3 ist;
• R₂ Wasserstoff oder C₁₋₄-Alkyl darstellt,
• R₃ Wasserstoff, Hydroxy oder C₁₋₄-Alkyl darstellt;
• R₄ Wasserstoff darstellt, oder R₄ zusammen mit R₃ =O oder =CH₂ darstellt;
• R₅ Phenyl, Naphthyl, einen 9- bis 10-gliedrigen kondensierten bicyclischen heterocyclischen Rest oder einen 5- oder 6-gliedrigen Heteroarylrest darstellt, wobei die Reste gegebenenfalls mit 1 bis 3 Resten substituiert sind, unabhängig ausgewählt aus Trifluormethyl, C₁₋₄-Alkyl, Hydroxy, Cyano, C₁₋₄-Alkoxy, Trifluormethoxy, Halogen oder S(O)_{q}C₁₋₄-Alkyl;
• R₆ und R₇ unabhängig Wasserstoff, Cyano oder C₁₋₄-Alkyl darstellen;
• R₈ (CH₂)ᵣR₁₀ ist;
• R₉ Wasserstoff, Halogen, C₃₋₇-Cycloalkyl, Hydroxy, Nitro, Cyano oder C₁₋₄-Alkyl darstellt, gegebenenfalls substituiert mit einem oder zwei Resten, ausgewählt aus Halogen, Cyano, Hydroxy oder C₁₋₄-Alkoxy;
• R₁₀ Wasserstoff oder C₃₋₇-Cycloalkyl darstellt;
• n 1 oder 2 darstellt;
• q 0, 1 oder 2 ist;
• r 0 oder eine ganze Zahl von 1 bis 4 ist;
oder ein pharmazeutisch verträgliches Salz oder ein Solvat davon.

2. Verbindung gemäß Anspruch 1, wobei n 2 ist.

3. Verbindung gemäß Anspruch 1 oder Anspruch 2, wobei R 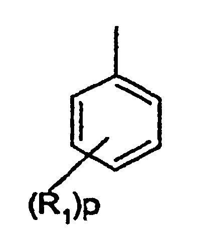 darstellt, wobei R₁ Halogen, C₁₋₄-Alkyl, Cyano, C₁₋₄-Alkoxy, Trifluormethyl oder Trifluormethoxy ist, p 0 oder eine ganze Zahl von 1 bis 3 ist.

4. Verbindung gemäß einem der Ansprüche 1 bis 3, wobei R₅ Phenyl oder Naphthyl ist, gegebenenfalls substituiert mit einem oder zwei Resten, ausgewählt aus Trifluormethyl, Cyano, C₁₋₄-Alkyl oder Halogen.

5. Verbindung gemäß einem der Ansprüche 1 bis 4, wobei R₈ (CH₂)ᵣR₁₀ ist, wobei R₁₀ Wasserstoff oder C₃₋₇-Cycloalkyl (z. B. Cyclopropyl) ist und r 0 oder 1 ist.

6. Verbindung gemäß einem der Ansprüche 1 bis 5, wobei R₉ Wasserstoff oder C₁₋₄-Alkyl ist, gegebenenfalls substituiert mit einem oder zwei Resten, ausgewählt aus Halogen.

7. Verbindung gemäß einem der Ansprüche 1 bis 6, wobei R Phenyl ist, substituiert mit einem Fluoratom, R₂, R₉ und R₄ Wasserstoff sind, R₃ Wasserstoff, Hydroxy oder Methyl ist oder zusammen mit R₄ =O oder =CH₂ bildet, R₆ und R₇ unabhängig Wasserstoff oder Methyl sind, R₅ Phenyl oder Naphthyl ist, gegebenenfalls substituiert mit einem oder zwei Resten, unabhängig ausgewählt aus Cyano, Methyl, Chlor-, Brom- oder Fluoratom, R₈ Wasserstoff, Methyl oder Cyclopropylmethyl ist und n 2 ist.

8. Verbindung gemäß Anspruch 1, nämlich
• 1-[(3,5-Dichlorphenyl)methyl]-3-[4-(4-fluorphenyl)-4-piperidinyl]-1,5-dihydro-2H-pyrrol-2-on;
• 1-[(3,5-Dichlorphenyl)methyl]-3-[4-(4-fluorphenyl)-1-methyl-4-piperidinyl]-1,5-dihydro-2H-pyrrol-2-on;
• 1-[1-(3,5-Dichlorphenyl)ethyl]-3-[4-(4-fluorphenyl)-4-piperidinyl]-1,5-dihydro-2H-pyrrol-2-on (Kettenenantiomer 1);
• 1-[(1S)-1-(3-Chlor-1-naphthalinyl)ethyl]-3-[4-(4-fluorphenyl)-4-piperidinyl]-1,5-dihydro-2H-pyrrol-2-on;
• 1-[(3-Chlor-1-naphthalinyl)methyl]-3-[4-(4-fluorphenyl)-4-piperidinyl]-1,5-dihydro-2H-pyrrol-2-on;
• 4-({3-[4-(4-Fluorphenyl)-4-piperidinyl]-2-oxo-2,5-dihydro-1H-pyrol-1-yl}methyl)-2-naphthalincarbonitril;
• 1-[1-(3,5-Dichlorphenyl)ethyl]-3-[4-(4-fluorphenyl)-4-piperidinyl]-1,5-dihydro-2H-pyrrol-2-on (Kettenenantiomer 2);
• 1-[(1R)-1-(3-Chlor-1-naphthalinyl)ethyl]-3-[4-(4-fluorphenyl)-4-piperidinyl]-1,5-dihydro-2H-pyrrol-2-on;
• 1-[1-(3,5-Dichlorphenyl)ethyl]-3-[4-(4-fluorphenyl)-1-methyl-4-piperidinyl]-1,5-dihydro-2H-pyrrol-2-on (Kettenenantiomer 1);
• 1-[(1S)-1-(3-Chlor-1-naphthalinyl)ethyl]-3-[4-(4-fluorphenyl)-1-methyl-4-piperidinyl]-1,5-dihydro-2H-pyrrol-2-on;
• 1-[(3-Chlor-1-naphthalinyl)methyl]-3-[4-(4-fluorphenyl)-1-methyl-4-piperidinyl]-1,5-dihydro-2H-pyrrol-2-on;
• 1-[1-(3,5-Dichlorphenyl)ethyl]-3-[4-(4-fluorphenyl)-1-methyl-4-piperidinyl]-1,5-dihydro-2H-pyrrol-2-on (Kettenenantiomer 2);
• 1-[(1R)-1-(3-Chlor-1-naphthalinyl)ethyl]-3-[4-(4-fluorphenyl)-1-methyl-4-piperidinyl]-1,5-dihydro-2H-pyrrol-2-on;
• 4-({3-[4-(4-Fluorphenyl)-1-methyl-4-piperidinyl]-2-oxo-2,5-dihydro-1H-pyrrol-1-yl}methyl)-2-naphthalincarbonitril;
• 1-[1-(3,5-Dichlorphenyl)ethyl]-3-[4-(4-fluorphenyl)-1-methyl-4-piperidinyl]-1,5-dihydro-2H-pyrrol-2-on Hydrochlorid (Kettenenantiomer 1);
• 1-[(3-Chlor-1-naphthalinyl)methyl]-3-[1-(cyclopropylmethyl)-4-(4-fluorphenyl)-4-piperidinyl]-1,5-dihydro-2H-pyrrol-2-on;
• 1-[(3,5-Dichlorphenyl)methyl]-3-[4-(4-fluorphenyl)-4-piperidinyl]-2-pyrrolidinon;
• 1-[(1S)-1-(3-Chlor-1-naphthalinyl)ethyl]-3-[4-(4-fluorphenyl)-4-piperidinyl]-2-pyrrolidinon (Diastereoisomer 1);
• 1-[(1S)-1-(3-Chlor-1-naphthalinyl)ethyl]-3-[4-(4-fluorphenyl)-4-piperidinyl]-2-pyrrolidinon;
• 1-[1-(3,5-Dichlorphenyl)ethyl]-3-[4-(4-fluorphenyl)-4-piperidinyl]-2-pyrrolidinon (Diastereoisomer 1, Kettenenantiomer 1);
• 1-[1-(3,5-Dichlorphenyl)ethyl]-3-[4-(4-fluorphenyl)-4-piperidinyl]-2-pyrrolidinon (Diastereoisomer 2, Kettenenantiomer 1);
• 1-[1-(3,5-Dichlorphenyl)ethyl]-3-[4-(4-fluorphenyl)-4-piperidinyl]-2-pyrrolidinon (Diastereoisomer 1, Kettenenantiomer 2);
• 1-[1-(3,5-Dichlorphenyl)ethyl]-3-[4-(4-fluorphenyl)-4-piperidinyl]-2-pyrrolidinon (Diastereoisomer 2, Kettenenantiomer 2);
• 4-({3-[4-(4-Fluorphenyl)-4-piperidinyl]-2-oxo-1-pyrrolidinyl}methyl)-2-naphthalincarbonitril (Enantiomer 1);
• 4-({3-[4-(4-Fluorphenyl)-4-piperidinyl]-2-oxo-1-pyrrolidinyl}methyl)-2-naphthalincarbonitril (Enantiomer 2);
• 7-Fluor-4-({3-[4-(4-fluorphenyl)-4-piperidinyl]-2-oxo-1-pyrrolidinyl}methyl)-2-naphthalincarbonitril (Enantiomer 2);
• 6-Fluor-4-({3-[4-(4-fluorphenyl)-4-piperidinyl]-2-oxo-1-pyrrolidinyl}methyl)-2-naphthalincarbonitril (Enantiomer 2);
• 7-Fluor-4-({3-[4-(4-fluorphenyl)-4-piperidinyl]-2-oxo-1-pyrrolidinyl}methyl)-2-naphthalincarbonitril (Enatiomer 1);
• 6-Fluor-4-({3-[4-(4-fluorphenyl)-4-piperidinyl]-2-oxo-1-pyrrolidinyl}methyl)-2-naphthalincarbonitril (Enantiomer 1);
• 1-[(3,5-Dichlorphenyl)methyl]-3-[4-(4-fluorphenyl)-1-methyl-4-piperidinyl]-2-pyrrolidinon;
• 1-[1-(3-Chlor-1-naphthalinyl)ethyl]-3-[4-(4-fluorphenyl)-1-methyl-4-piperidinyl]-2-pyrrolidinon (Diastereoisomer 2, Kettenenantiomer 1);
• 1-[1-(3,5-Dichlorphenyl)ethyl]-3-[4-(4-fluorphenyl)-1-methyl-4-piperidinyl]-2-pyrrolidinon (Diastereoisomer 1, Kettenenantiomer 1);
• 1-[1-(3,5-Dichlorphenyl)ethyl]-3-[4-(4-fluorphenyl)-1-methyl-4-piperidinyl]-2-pyrrolidinon (Diastereoisomer 2, Kettenenantiomer 1);
• 1-[1-(3,5-Dichlorphenyl)ethyl]-3-[4-(4-fluorphenyl)-1-methyl-4-piperidinyl]-2-pyrrolidinon (Diastereoisomer 1, Kettenenantiomer 2);
• 1-[1-(3,5-Dichlorphenyl)ethyl]-3-[4-(4-fluorphenyl)-1-methyl-4-piperidinyl]-2-pyrrolidinon (Diastereoisomer 2, Kettenenantiomer 2);
• 1-[(3,5-Dichlorphenyl)methyl]-3-[4-(4-fluorphenyl)-1-methyl-4-piperidinyl]-2-pyrrolidinon (Enantiomer 1);
• 1-[(3,5-Dichlorphenyl)methyl]-3-[4-(4-fluorphenyl)-1-methyl-4-piperidinyl]-2-pyrrolidinon (Enantiomer 2);
• 4-({3-[4-(4-Fluorphenyl)-1-methyl-4-piperidinyl]-2-oxo-1-pyrrolidinyl}methyl)-2-naphthalincarbonitril (Enantiomer 1);
• 4-({3-[4-(4-Fluorphenyl)-1-methyl-4-piperidinyl]-2-oxo-1-pyrrolidinyl}methyl)-2-naphthalincarbonitril (Enantiomer 2);
• 7-Fluor-4-({3-[4-(4-fluorphenyl)-1-methyl-4-piperidinyl]-2-oxo-1-pyrrolidinyl}-methyl)-2-naphthalincarbonitril (Enantiomer 2);
• 6-Fluor-4-({3-[4-(4-fluorphenyl)-1-methyl-4-piperidinyl]-2-oxo-1-pyrrolidinyl}-methyl)-2-naphthalincarbonitril;
• 7-Fluor-4-({3-[4-(4-fluorphenyl)-1-methyl-4-piperidinyl]-2-oxo-1-pyrrolidinyl}-methyl)-2-naphthalincarbonitril (Enantiomer 1);
• 6-Fluor-4-({3-[4-(4-fluorphenyl)-1-methyl-4-piperidinyl]-2-oxo-1-pyrrolidinyl}-methyl)-2-naphthalincarbonitril (Enantiomer 1);
• 1-[(3,5-Dichlorphenyl)methyl]-3-[4-(4-fluorphenyl)-1-methyl-4-piperidinyl]-1H-pyrrol-2,5-dion;
• 1-[(3,5-Dichlorphenyl)methyl-3-[4-(4-fluorphenyl)-1-methyl-4-piperidinyl]-5-methyliden-1,5-dihydro-2H-pyrrol-2-on;
oder ein pharmazeutisch verträgliches Salz (z.B. Hydrochlorid, Fumarat oder Citrat) oder ein Solvat oder amorphe oder kristalline Formen davon.

9. Verbindung gemäß Anspruch 1, nämlich
1-[(3,5-Dichlorphenyl)methyl]-3-[4-(4-fluorphenyl)-1-methyl-4-piperidinyl]-1,5-dihydro-2H-pyrrol-2-on oder ein pharmazeutisch verträgliches Salz.

10. Verbindung gemäß Anspruch 1, nämlich
1-[(3,5-Dichlorphenyl)methyl]-3-[4-(4-fluorphenyl)-1-methyl-4-piperidinyl]-1,5-dihydro-2H-pyrrol-2-on Hydrochlorid oder kristalline Formen davon.

11. Verbindung gemäß Anspruch 1, nämlich
1-[(3,5-Dichlorphenyl)methyl]-3-[4-(4-fluorphenyl)-1-methyl-4-piperidinyl]-1,5-dihydro-2H-pyrrol-2-on Fumarat oder kristalline Formen davon.

12. Verbindung gemäß Anspruch 1, nämlich
1-[(3,5-Dichlorphenyl)methyl]-3-[4-(4-fluorphenyl)-1-methyl-4-piperidinyl]-1,5-dihydro-2H-pyrrol-2-on Citrat oder kristalline Formen davon.

13. Verbindung gemäß Anspruch 1, nämlich
1-[(3,5-Dichlorphenyl)methyl]-3-[4-(4-fluorphenyl)-1-methyl-4-piperidinyl]-1,5-dihydro-2H-pyrrol-2-on Citrat (kristalline Form 1).

14. Verfahren zur Herstellung einer Verbindung gemäß Anspruch 1, wobei das Verfahren umfasst:
a) Cyclisierung einer Verbindung der Formel (IV), 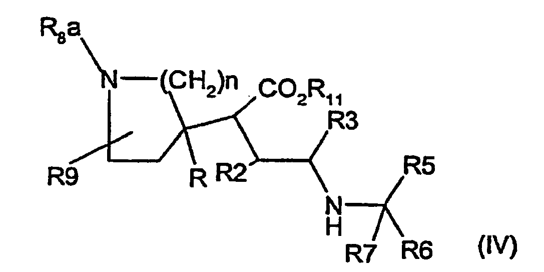 wobei R₁₁ C₁₋₄-Alkyl ist (z.B. Methyl oder Ethyl), R₃ Wasserstoff oder C₁₋₄-Alkyl ist, R₈ₐ die Bedeutung wie in Formel (I) definiert hat oder eine Stickstoffschutzgruppe ist und R, R₂, R₅, R₆, R₇, R₉ und n wie in Anspruch 1 definiert sind, um eine Verbindung der Formel (I) zu ergeben, wobei --- eine Einfachbindung ist, R₃ Wasserstoff oder C₁₋₄-Alkyl darstellt und R₄ Wasserstoff darstellt, oder
b) Cyclisierung einer Verbindung der Formel (VI), 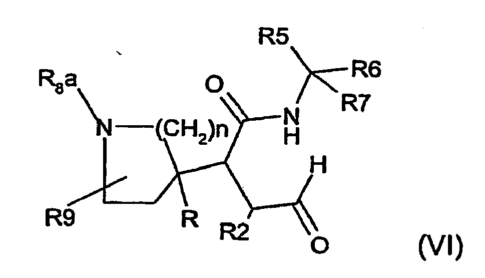 wobei R₈ₐ die Bedeutung wie in Formel (I) definiert hat oder eine Stickstoffschutzgruppe ist, R, R₂, R₅, R₆, R₇, R₉ und n wie in Anspruch 1 definiert sind, um eine Verbindung der Formel (I) zu ergeben, wobei --- eine Einfachbindung ist, R₃ Hydroxy ist und R₄ Wasserstoff ist, oder
c) Cyclisierung einer Verbindung der Formel (VII), 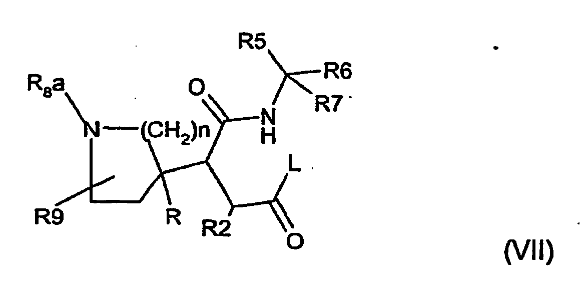 wobei R₈ₐ die Bedeutung wie in Formel (I) definiert hat oder eine Stickstoffschutzgruppe ist, L eine Abgangsgruppe ist und R, R₂, R₅, R₆, R₇, R₉ und n wie in Anspruch 1 definiert sind, um eine Verbindung der Formel (I) zu ergeben, wobei --- eine Einfachbindung ist und R₃ zusammen mit R₄ =O darstellt, oder
d) Umsetzung einer Verbindung der Formel (VIIA), 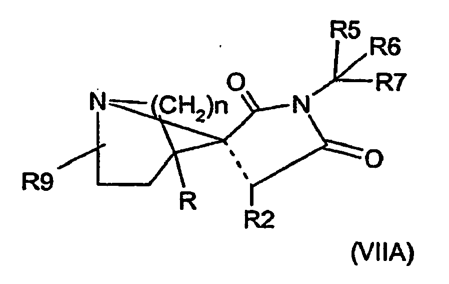 wobei R, R₂, R₅, R₆, R₇, R₉ und n wie in Anspruch 1 definiert sind, mit einem Aldehyd, CH(O)(CH₂)ₘR₁₀ (VIIIa), wobei m eine ganze Zahl von 0 bis 3 ist und R₁₀ wie in Anspruch 1 definiert ist, um eine Verbindung der Formel (I) zu ergeben, wobei --- eine Einfachbindung ist, R₈ (CH₂)ᵣR₁₀ darstellt, wobei r eine ganze Zahl von 1 bis 4 ist, und R₃ zusammen mit R₄ =O darstellt, oder
e) Cyclisierung einer Verbindung der Formel (VIa) in Gegenwart einer Säure, 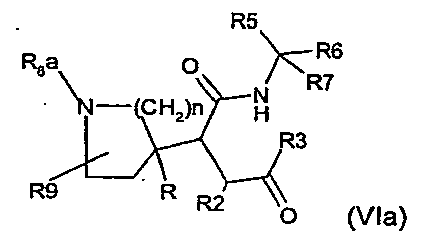 wobei R₃ Wasserstoff ist, R₈ₐ die Bedeutung wie in Formel (I) definiert hat oder eine Stickstoffschutzgruppe ist, R, R₂, R₅, R₆, R₇, R₉ und n wie in Anspruch 1 definiert sind, um Verbindungen der Formel (I) zu ergeben, wobei --- eine Doppelbindung ist, R₃ Wasserstoff oder C₁₋₄-Alkyl darstellt und R₄ Wasserstoff ist; oder
f) N-Alkylierung einer Verbindung der Formel (VIII), wobei R₈ₐ die Bedeutung wie in Formel (I) definiert hat oder eine Stickstoffschutzgruppe ist und R, R₂, R₃, R₄, R₉ und n wie in Anspruch 1 definiert sind, mit einer Verbindung der Formel (IX) 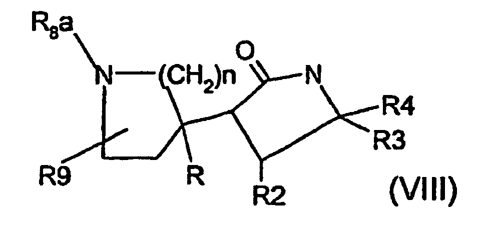
L-C(R₅)(R₆)(R₇) (IX)
wobei L eine Abgangsgruppe ist, R₅, R₆ und R₇ wie in Anspruch 1 definiert sind, um eine Verbindung der Formel (I) zu ergeben, wobei --- eine Einfachbindung ist,
und anschließend, gegebenenfalls für einen der Schritte (a) bis (f):
• Entfernen jeglicher Schutzgruppen und/oder
• Umwandeln einer Verbindung der Formel (I) in eine andere Verbindung der Formel (I) und/oder
• Trennen einer Verbindung der Formel (I), oder eines Derivats davon, in die Enantiomere davon,
• Bilden eines pharmazeutisch verträglichen Salzes.

15. Verbindung gemäß einem der Ansprüche 1 bis 13 zur Verwendung in der Therapie.

16. Verwendung einer Verbindung gemäß einem der Ansprüche 1 bis 13 zur Herstellung eines Medikaments zur Verwendung in der Behandlung von Erkrankungen, die durch Tachykinine (einschließlich Substanz P und andere Neurokinine) und/oder durch selektive Hemmung des Serotonin-Wiederaufnahme-Transportproteins vermittelt werden.

17. Verwendung einer Verbindung gemäß einem der Ansprüche 1 bis 13 zur Herstellung eines Medikaments zur Verwendung in der Behandlung von Depression und/oder Angst.

18. Arzneimittel, umfassend eine Verbindung gemäß einem der Ansprüche 1 bis 13 in Beimischung mit einem oder mehreren pharmazeutisch verträglichen Trägem oder Exzpienten.

## Revendications

1. Composé de formule (I) 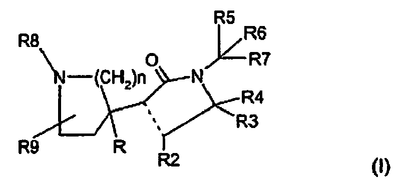 dans lequel :
• ---- représente une liaison simple ou une double liaison ;
• R représente un radical choisi entre des radicaux : 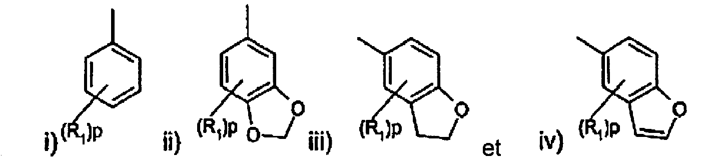
dans lesquels R₁ représente un groupe halogéno, cyano, alkyle en C₁ à C₄, alkoxy en C₁ à C₄, trifluorométhyle ou trifluorométhoxy et p est égal à zéro ou à un nombre entier de 1 à 3 ;
• R₂ représente un atome d'hydrogène ou un groupe alkyle en C₁ à C₄ ;
• R₃ représente un atome d'hydrogène, un groupe hydroxy ou alkyle en C₁ à C₄ ;
• R₄ représente un atome d'hydrogène, ou bien R₄, conjointement avec R₃, représente un groupe =O ou =CH₂ ;
• R₅ représente un groupe phényle, naphtyle, un groupe hétérocyclique bicyclique condensé nona- ou décagonal ou un groupe hétéroaryle penta- ou hexagonal, lesdits groupes étant facultativement substitués avec 1 à 3 groupes choisis indépendamment entre des groupes trifluorométhyle, alkyle en C₁ à C₄, hydroxy, cyano, alkoxy en C₁ à C₄, trifluorométhoxy, halogéno et S(O)q(alkyle en C₁ à C₄) ;
• R₆ et R₇ représentent indépendamment un atome d'hydrogène, un groupe cyano ou alkyle en C₁ à C₄ ;
• R₈ représente un groupe (CH₂)rR₁₀ ;
• R₉ représente un atome d'hydrogène, un atome d'halogène, un groupe cycloalkyle en C₃ à C₇, hydroxy, nitro, cyano ou alkyle en C₁ à C₄ facultativement substitué avec un ou deux groupes choisis entre des groupes halogéno, cyano, hydroxy et alkoxy en C₁ à C₄ ;
• R₁₀ représente un atome d'hydrogène ou un groupe cycloalkyle en C₃ à C₇ ;
• n est égal à 1 ou 2 ;
• q est égal à 0, 1 ou 2 ;
• r est égal à 0 ou à un nombre entier de 1 à 4 ;
ou un de ses sels pharmaceutiquement acceptables ou un de ses produits de solvatation.

2. Composé suivant la revendication 1, dans lequel n est égal à 2.

3. Composé suivant la revendication 1 ou la revendication 2, dans lequel R représente un groupe : 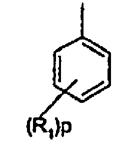 dans lequel R₁ représente un groupe halogéno, alkyle en C₁ à C₄, cyano, alkoxy en C₁ à C₄, trifluorométhyle ou trifluorométhoxy, et p est égal à zéro ou à un nombre entier de 1 à 3.

4. Composé suivant l'une quelconque des revendications 1 à 3, dans lequel R₅ représente un groupe phényle ou naphtyle facultativement substitué avec un ou deux groupes choisis entre des groupes trifluorométhyle, cyano, alkyle en C₁ à C₄ et halogéno.

5. Composé suivant l'une quelconque des revendications 1 à 4, dans lequel R₈ représente un groupe (CH₂)rR₁₀ dans lequel R₁₀ représente un atome d'hydrogène ou un groupe cycloalkyle en C₃ à C₇ (par exemple cyclopropyle) et r est égal à 0 ou 1.

6. Composé suivant l'une quelconque des revendications 1 à 5, dans lequel R₉ représente un atome d'hydrogène ou un groupe alkyle en C₁ à C₄ facultativement substitué avec un ou deux groupes choisis parmi des groupes halogéno.

7. Composé suivant l'une quelconque des revendications 1 à 6, dans lequel R représente un groupe phényle substitué avec un atome de fluor, R₂, R₉ et R₄ représentent des atomes d'hydrogène, R₃ représente un atome d'hydrogène, un groupe hydroxy ou méthyle ou bien, conjointement avec R₄, forme un groupe =O ou =CH₂, R₆ et R₇ représentent indépendamment un atome d'hydrogène ou un groupe méthyle, R₅ représente un groupe phényle ou naphtyle facultativement substitué avec un ou deux groupes choisis indépendamment entre des groupes cyano, méthyle, chloro, bromo et fluoro, R₈ représente un atome d'hydrogène, un groupe méthyle ou cyclopropylméthyle et n est égal à 2.

8. Composé suivant la revendication 1, qui est
• la 1-[(3,5-dichlorophényl)méthyl]-3-[4-(4-fluorophényl)-4-pipéridinyl]-1,5-dihydro-2*H*-pyrrole-2-one ;
• la 1-[(3,5-dichlorophényl)méthyl]-3-[4-(4-fluorophényl)-1-méthyl-4-pipéridinyl]-1,5-dihydro-2*H*-pyrrole-2-one ;
• la 1-[1-(3,5-dichlorophényl)éthyl]-3-[4-(4-fluorophényl)-4-pipéridinyl]-1,5-dihydro-2*H*-pyrrole-2-one (énantiomère de chaîne 1) ;
• la 1-[(1S)-1-(3-chloro-1-naphtalényl)éthyl]-3-[4-(4-fluorophényl)-4-pipéridinyl]-1,5-dihydro-2H-pyrrole-2-one ;
• la 1-[(3-chloro-1-naphtalényl)méthyl]-3-[4-(4-fluorophényl)-4-pipéridinyl]-1,5-dihydro-2*H*-pyrrole-2-one ;
• le 4-({3-[4-(4-fluorophényl)-4-pipéridinyl]-2-oxo-2,5-dihydro-1*H*-pyrrole-1-yl}méthyl)-2-naphtalènecarbonitrile ;
• la 1-[1-(3,5-dichlorophényl)éthyl]-3-[4-(4-fluorophényl)-4-pipéridinyl]-1,5-dihydro-2*H*-pyrrole-2-one (énantiomère de chaîne 2) ;
• la 1-[(1*R*)-1-(3-chloro-1-naphtalényl)éthyl]-3-[4-(4-fluorophényl)-4-pipéridinyl]-1,5-dihydro-2*H*-pyrrole-2-one ;
• la 1-[1-(3,5-dichlorophényl)éthyl]-3-[4-(4-fluorophényl)-1-méthyl-4-pipéridinyl]-1,5-dihydro-2*H*-pyrrole-2-one (énantiomère de chaîne 1) ;
• la 1-[(1*S*)-1-(3-chloro-1-naphtalényl)éthyl]-3-[4-(4-fluorophényl)-1-méthyl-4-pipéridinyl]-1,5-dihydro-2*H-*pyrrole-2-one ;
• la 1-[(3-chloro-1-naphtalényl)méthyl]-3-[4-(4-fluorophényl)-1-méthyl-4-pipéridinyl]-1,5-dihydro-2H-pyrrole-2-one ;
• la 1-[1-(3,5-dichlorophényl)éthyl]-3-[4-(4-fluorophényl)-1-méthyl-4-pipéridinyl]-1,5-dihydro-2*H*-pyrrole-2-one (énantiomère de chaîne 2) ;
• la 1-[(1*R*)-1-(3-chloro-1-naphtalényl)éthyl]-3-[4-(4-fluorophényl)-1-méthyl-4-pipéridinyl]-1,5-dihydro-2*H-*pyrrole-2-one ;
• le 4-({3-[4-(4-fluorophényl)-1-méthyl-4-pipéridinyl]-2-oxo-2,5-dihydro-1*H*-pyrrole-1-yl}méthyl)-2-naphtalène-carbonitrile ;
• le chlorhydrate de 1-[1-(3,5-dichlorophényl)éthyl]-3-[4-(4-fluorophényl)-1-méthyl-4-pipéridinyl]-1,5-dihydro-2*H*-pyrrole-2-one (énantiomère de chaîne 1) ;
• la 1-[(3-chloro-1-naphtalényl)méthyl]-3-[1-(cyclopropylméthyl)-4-(4-fluorophényl)-4-pipéridinyl]-1,5-dihydro-2H-pyrrole-2-one ;
• la 1- [(3,5-dichlorophényl)méthyl]-3-[4-(4-fluorophényl)-4-pipéridinyl]-2-pyrrolidinone ;
• la 1-[(1S)-1-(3-chloro-1-naphtalényl)éthyl]-3-[4-(4-fluorophényl)-4-pipéridinyl]-2-pyrrolidinone (diastéréoisomère 1) ;
• la 1-[(1S)-1-(3-chloro-1-naphtalényl)éthyl]-3-[4-(4-fluorophényl)-4-pipéridinyl]-2-pyrrolidinone ;
• la 1-[1-(3,5-dichlorophényl)éthyl]-3-[4-(4-fluorophényl)-4-pipéridinyl]-2-pyrrolidinone (diastéréoisomère 1, énantiomère de chaîne 1) ;
• la 1-[1-(3,5-dichlorophényl)éthyl]-3-[4-(4-fluorophényl)-4-pipéridinyl]-2-pyrrolidinone (diastéréoisomère 2, énantiomère de chaîne 1) ;
• la 1-[1-(3,5-dichlorophényl)éthyl]-3-[4-(4-fluorophényl)-4-pipéridinyl]-2-pyrrolidinone (diastéréoisomère 1, énantiomère de chaîne 2) ;
• la 1-[1-(3,5-dichlorophényl)éthyl]-3-[4-(4-fluorophényl)-4-pipéridinyl]-2-pyrrolidinone (diastéréoisomère 2, énantiomère de chaîne 2) ;
• le 4-({3-[4-(4-fluorophényl)-4-pipéridinyl]-2-oxo-1-pyrrolidinyl}méthyl)-2-naphtalènecarbonitrile (énantiomère 1) ;
• le 4-({3-[4-(4-fluorophényl)-4-pipéridinyl]-2-oxo-1-pyrrolidinyl}méthyl)-2-naphtalènecarbonitrile (énantiomère 2) ;
• le 7-fluoro-4-({3-[4-(4-fluorophényl)-4-pipéridinyl]-2-oxo-1-pyrrolidinyl}méthyl)-2-naphtalènecarbonitrile (énantiomère 2) ;
• le 6-fluoro-4-({3-[4-(4-fluorophényl)-4-pipéridinyl]-2-oxo-1-pyrrolidinyl}méthyl)-2-naphtalènecarbonitrile (énantiomère 2) ;
• le 7-fluoro-4-({3-[4-(4-fluorophényl)-4-pipéridinyl]-2-oxo-1-pyrrolidinyl}méthyl)-2-naphtalènecarbonitrile (énantiomère 1) ;
• le 6-fluoro-4-({3-[4-(4-fluorophényl)-4-pipéridinyl]-2-oxo-1-pyrrolidinyl}méthyl)-2-naphtalènecarbonitrile (énantiomère 1) ;
• la 1-[(3,5-dichlorophényl)méthyl]-3-[4-(4-fluorophényl)-1-méthyl-4-pipéridinyl]-2-pyrrolidinone ;
• la 1-[1-(3-chloro-1-naphtalényl)éthyl]-3-[4-(4-fluorophényl)-1-méthyl-4-pipéridinyl]-2-pyrrolidinone (diastéréoisomère 2, énantiomère de chaîne 1) ;
• la 1-[1-(3,5-dichlorophényl)éthyl]-3-[4-(4-fluorophényl)-1-méthyl-4-pipéridinyl]-2-pyrrolidinone (diastéréoisomère 1, énantiomère de chaîne 1) ;
• la 1-[1-(3,5-dichlorophényl)éthyl]-3-[4-(4-fluorophényl)-1-méthyl-4-pipéridinyl]-2-pyrrolidinone (diastéréoisomère 2, énantiomère de chaîne 1) ;
• la 1-[1-(3,5-dichlorophényl)éthyl]-3-[4-(4-fluorophényl)-1-méthyl-4-pipéridinyl]-2-pyrrolidinone (diastéréoisomère 1, énantiomère de chaîne 2) ;
• la 1-[1-(3,5-dichlorophényl)éthyl]-3-[4-(4-fluorophényl)-1-méthyl-4-pipéridinyl]-2-pyrrolidinone (diastéréoisomère 2, énantiomère de chaîne 2) ;
• la 1-[(3,5-dichlorophényl)méthyl]-3-[4-(4-fluorophényl)-1-méthyl-4-pipéridinyl]-2-pyrrolidinone (énantiomère 1) ;
• la 1-[(3,5-dichlorophényl)méthyl]-3-[4-(4-fluorophényl)-1-méthyl-4-pipéridinyl]-2-pyrrolidinone (énantiomère 2) ;
• le 4-({3-[4-(4-fluorophényl)-1-méthyl-4-pipéridinyl]-2-oxo-1-pyrrolidinyl}méthyl)-2-naphtalènecarbonitrile (énantiomère 1) ;
• le 4-({3-[4-(4-fluorophényl)-1-méthyl-4-pipéridinyl]-2-oxo-1-pyrrolidinyl}méthyl)-2-naphtalènecarbonitrile (énantiomère 2) ;
• le 7-fluoro-4-({3-[4-(4-fluorophényl)-1-méthyl-4-pipéridinyl]-2-oxo-1-pyrrolidinyl}méthyl)-2-naphtalènecarbonitrile (énantiomère 2) ;
• le 6-fluoro-4-({3-[4-(4-fluorophényl)-1-méthyl-4-pipéridinyl]-2-oxo-1-pyrrolidinyl}méthyl)-2-naphtalènecarbonitrile ;
• le 7-fluoro-4-({3-[4-(4-fluorophényl)-1-méthyl-4-pipéridinyl]-2-oxo-1-pyrrolidinyl}méthyl)-2-naphtalènecarbonitrile (énantiomère 1) ;
• le 6-fluoro-4-({3-[4-(4-fluorophényl)-1-méthyl-4-pipéridinyl]-2-oxo-1-pyrrolidinyl}méthyl)-2-naphtalènecarbonitrile (énantiomère 1) ;
• la 1-[(3,5-dichlorophényl)méthyl]-3-[4-(4-fluorophényl)-1-méthyl-4-pipéridinyl]-1*H*-pyrrole-2,5-dione ;
• la 1- [(3,5-dichlorophényl)méthyl]-3-[4- (4-fluorophényl) -1-méthyl-4-pipéridinyl]-5-méthylidène-1, 5-dihydro-2H-pyrrole-2-one ;
ou un de ses sels pharmaceutiquement acceptables (par exemple un chlorhydrate, fumarate ou citrate) ou un de ses produits de solvatation ou bien une de ses formes amorphes ou cristallines.

9. Composé suivant la revendication 1, qui est :
la 1-[(3,5-dichlorophényl)méthyl]-3-[4-(4-fluorophényl)-1-méthyl-4-pipéridinyl]-1,5-dihydro-2*H*-pyrrole-2-one ou un de ses sels pharmaceutiquement acceptables.

10. Composé suivant la revendication 1, qui est :
le chlorhydrate de 1-[(3,5-dichlorophényl)méthyl]-3-[4-(4-fluorophényl)-1-méthyl-4-pipéridinyl]-1,5-dihydro-2*H*-pyrrole-2-one ou ses formes cristallines.

11. Composé suivant la revendication 1, qui est :
le fumarate de 1-[(3,5-dichlorophényl)méthyl]-3-[4-(4-fluorophényl)-1-méthyl-4-pipéridinyl]-1,5-dihydro-2*H-*pyrrole-2-one ou ses formes cristallines.

12. Composé suivant la revendication 1, qui est :
le citrate de 1- [(3,5-dichlorophényl)méthyl]-3-[4-(4-fluorophényl)-1-méthyl-4-pipéridinyl]-1,5-dihydro-2*H-*pyrrole-2-one ou ses formes cristallines.

13. Composé suivant la revendication 1, qui est le citrate de 1-[(3,5-dichlorophényl)méthyl]-3-[4-(4-fluorophényl)-1-(méthyl-4-pipéridinyl]-1,5-dihydro-2*H*-pyrrole-2-one (forme cristalline 1).

14. Procédé pour la préparation d'un composé suivant la revendication 1, procédé qui comprend :
**a)** la cyclisation d'un composé de formule (IV), dans laquelle R₁₁ représente un groupe alkyle en C₁ à C₄ (par exemple 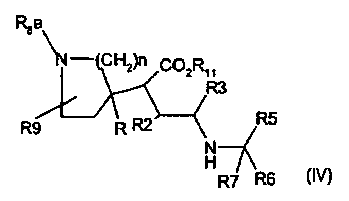 méthyle ou éthyle), R₃ représente un atome d'hydrogène ou un groupe alkyle en C₁ à C₄, R₈ₐ répond à la définition mentionnée pour la formule (I) ou représente un groupe protecteur de l'atome d'azote et R, R₂, R₅, R₆, R₇, R₉ et n sont tels que définis dans la revendication 1, ce qui donne un composé de formule (I), dans laquelle ---- représente une liaison simple, R₃ représente un atome d'hydrogène ou un groupe alkyle en C₁ à C₄ et R₄ représente un atome d'hydrogène, ou
**b)** la cyclisation d'un composé de formule (VI), dans laquelle R₈ₐ répond à la définition mentionnée pour la 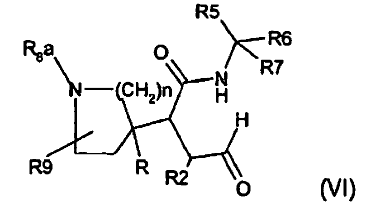 formule (I) ou représente un groupe protecteur de l'atome d'azote, R, R₂, R₅, R₆, R₇, R₉ et n sont tels que définis dans la revendication 1, ce qui donne un composé de formule (I) dans laquelle ---- représente une liaison simple, R₃ représente un groupe hydroxy et R₄ représente un atome d'hydrogène, ou
**c)** la cyclisation d'un composé de formule (VII), dans laquelle R₈ₐ a la définition mentionnée pour la formule (I) ou représente un groupe protecteur de l'atome d'azote, L représente un groupe partant et R, R₂, R₅, R₆, R₇, R₉ et n sont tels que définis dans la revendication 1, 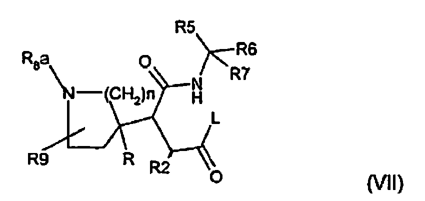 ce qui donne un composé de formule (I) dans laquellereprésente une liaison simple et R₃, conjointement avec R₄, représente un groupe =O, ou
**d)** la réaction d'un composé de formule (VIIA) dans laquelle R, R₂, R₅, R₆, R₇, R₉ et n sont tels que définis dans la revendication 1, 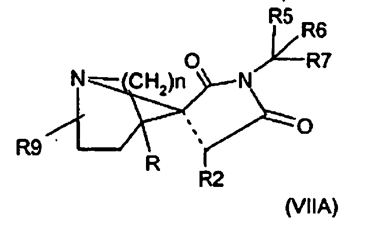 avec un aldéhyde, CH(O)(CH₂)ₘR₁₀ (VIIIa), dans lequel m représente un nombre entier de 0 à 3 et R₁₀ est tel que défini dans la revendication 1, ce qui donne un composé de formule (I) dans laquelle ---- représente une liaison simple, R₈ représente un groupe (CH₂)rR₁₀, dans lequel r représente un nombre entier de 1 à 4 et R₃, conjointement avec R₄, représente un groupe =O, ou
**e)** une cyclisation, en présence d'un acide, d'un composé de formule (VIa), dans laquelle 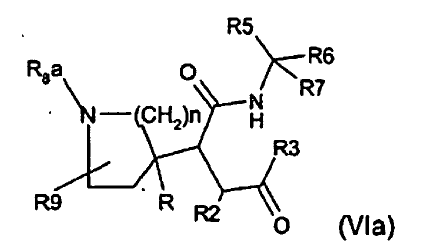 R₃ représente un atome d'hydrogène, R₈ₐ répond à la définition mentionnée pour la formule (I) ou représente un groupe protecteur de l'atome d'azote, R, R₂, R₅, R₆, R₇, R₉ et n sont tels que définis dans la revendication 1, ce qui donne des composés de formule (I), dans laquellereprésente une double liaison, R₃ représente un atome d'hydrogène ou un groupe alkyle en C₁ à C₄ et R₄ représente un atome d'hydrogène ; ou
**f)** la N-alkylation d'un composé de formule (VIII), dans laquelle R₈ₐ répond à la définition mentionnée pour la formule (I) ou représente un groupe protecteur de l'atome d'azote et R, R₂, R₃, R₄, R₉ et n sont tels que définis dans la revendication 1, avec un composé de formule (IX) 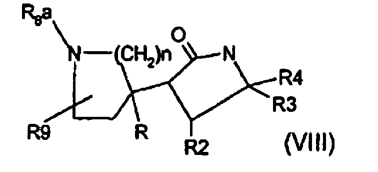
L-C(R₅)(R₆)(R₇) (IX)
dans laquelle L représente un groupe partant, R₅, R₆ et R₇ sont tels que définis dans la revendication 1, ce qui donne un composé de formule (I) dans laquelle ---- représente une liaison simple, et ensuite, facultativement, pour n'importe laquelle des étapes (a) à (f) :
• l'élimination de n'importe quels groupes protecteurs et/ou
• la conversion d'un composé de formule (I) en un autre composé de formule (I) et/ou
• la séparation d'un composé de formule (I) ou d'un de ses dérivés en ses énantiomères
• la formation d'un sel pharmaceutiquement acceptable.

15. Composé suivant l'une quelconque des revendications 1 à 13, destiné à être utilisé en thérapie.

16. Utilisation d'un composé suivant l'une quelconque des revendications 1 à 13, dans la préparation d'un médicament destiné à être utilisé dans le traitement d'affections à médiation par des tachykinines (comprenant la substance P et d'autres neurokinines) et/ou par inhibition sélective de la protéine servant de transporteur de réabsorption de sérotonine.

17. Utilisation d'un composé suivant l'une quelconque des revendications 1 à 13, dans la préparation d'un médicament destiné à être utilisé dans le traitement de la dépression et/ou de l'anxiété.

18. Composition pharmaceutique comprenant un composé suivant l'une quelconque des revendications 1 à 13, en mélange avec un ou plusieurs supports ou excipients pharmaceutiquement acceptables.
